# EUROPÄISCHE PATENTSCHRIFT

(11) **EP 0 885 194 B1**
(45) Veröffentlichungstag und Bekanntmachung des Hinweises auf die Patenterteilung: **16.10.2002**
(21) Anmeldenummer: 97905033.3
(22) Anmeldetag: 17.02.1997
(51) Int. Cl.: C07D 213/64, A01N 43/40

(54) **PYRIDYL-PHENYL- UND -BENZYLETHER, VERFAHREN UND ZWISCHENPRODUKTE ZU IHRER HERSTELLUNG UND IHRE VERWENDUNG ALS FUNGIZIDE UND ZUR BEKÄMPFUNG VON TIERISCHEN SCHÄDLINGEN**
PYRIDYL-PHENYL- AND BENZYLETHERS, PROCESS AND INTERMEDIATE PRODUCTS FOR THEIR PREPARATION AND THEIR USE AS FUNGICIDES AND FOR CONTROLLING ANIMAL PESTS
ETHERS DE PYRIDYLE, DE PHENYLE ET DE BENZYLE, PROCEDES ET PRODUITS INTERMEDIAIRES PERMETTANT DE LES PREPARER, ET LEUR UTILISATION COMME FONGICIDES ET POUR LUTTER CONTRE DES PARASITES ANIMAUX

(30) Priorität: 17.02.1996 DE 19605903
(43) Veröffentlichungstag der Anmeldung: 23.12.1998
(73) Patentinhaber: BASF AKTIENGESELLSCHAFT, 67056 Ludwigshafen (DE)
(72) Erfinder: OBERDORF, Klaus, D-69117 Heidelberg (DE); GRAMMENOS, Wassilios, D-67063 Ludwigshafen (DE); SAUTER, Hubert, D-68167 Mannheim (DE); GROTE, Thomas, D-67105 Schifferstadt (DE); MÜLLER, Bernd, D-67227 Frankenthal (DE); KIRSTGEN, Reinhard, D-67434 Neustadt (DE); MÜLLER, Ruth, D-67159 Friedelsheim (DE); BAYER, Herbert, D-68159 Mannheim (DE); PTOCK, Arne, D-67067 Ludwigshafen (DE); RACK, Michael, D-69123 Heidelberg (DE); HARREUS, Albrecht, D-67063 Ludwigshafen (DE); RÖHL, Franz, D-67105 Schifferstadt (DE); LORENZ, Gisela, D-67434 Hambach (DE); AMMERMANN, Eberhard, D-64646 Heppenheim (DE); STRATHMANN, Siegfried, D-67117 Limburgerhof (DE); HARRIES, Volker, D-67227 Frankenthal (DE)
(86) Internationale Anmeldenummer: EP9700736
(87) Internationale Veröffentlichungsnummer: WO97030032

(56) Entgegenhaltungen:
- EP-A- 0 278 595
- EP-A- 0 363 818
- EP-A- 0 398 692
- EP-A- 0 669 315

## Beschreibung

Die vorliegende Erfindung betrifft Pyridyl-phenyl- und -benzylether der Formel I, 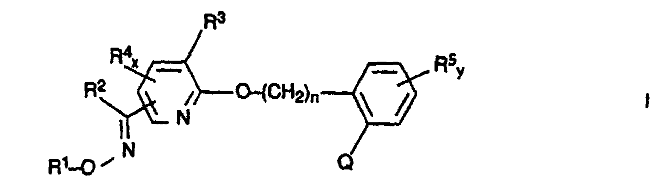 sowie deren Salze und N-Oxide, in denen die Substituenten und Indices die folgende Bedeutung haben:
- Q: C (CO₂CH₃)=CHCH₃, C (CO₂CH₃)=CHOCH₃, C (CONH₂)=NOCH₃, C (CO₂CH₃)=NOCH₃, C (CONHCH₃)=NOCH₃ oder N(OCH₃)-CO₂CH₃;
- n: 0 oder 1;
- R¹: Wasserstoff oder
C₁-C₆-Alkyl, C₃-C₆-Alkenyl, C₃-C₆-Alkinyl, C₁-C₆-Halogenalkyl, C₃-C₆-Halogenalkenyl, Aryl-C₁-C₂-alkyl, wobei der Arylrest partiell oder vollständig halogeniert sein kann und/oder eine bis drei der folgenden Gruppen tragen kann: C₁-C₄-Alkyl, C₁-C₄-Halogenalkyl und C₁-C₄-Alkoxy.
- R²: Wasserstoff oder
C₁-C₆-Alkyl, C₂-C₆-Alkenyl, C₂-C₆-Alkinyl, C₃-C₆-Cycloalkyl oder Phenyl.
- R³: Wasserstoff, Halogen, C₁-C₄-Alkyl oder C₁-C₂-Halogenalkyl;
- x: 0, 1 oder 2, wobei die Reste R⁴ verschieden sein können, wenn x für 2 steht;
- R⁴: Cyano, Nitro, Halogen, C₁-C₂-Alkyl oder C₁-C₂-Alkoxy;
- y: 0, 1, 2 oder 3, wobei die Reste R⁵ verschieden sein können, wenn y für 2 oder 3 steht;
- R⁵: Cyano, Halogen, C₁-C₄-Alkyl, C₁-C₄-Halogenalkyl oder C₁-C₄-Alkoxy.

Desweiteren betrifft die Erfindung Verfahren und Zwischenprodukte zur Herstellung dieser Verbindungen sowie ihre Verwendung zur Bekämpfung von tierischen Schädlingen und Schadpilzen.

Aus der Literatur (EP-A 254 426; EP-A 278 595; EP-A 299 694; EP-A 363 818; EP-A 350 691; EP-A 398 692; EP-A 407 873; EP-A 477 631; EP-A 513 580; JP-A 04/182,461; WO-A 93/15,046) sind Pyridyl-phenyl- und -benzylether mit fungiziden bzw. fungiziden und insektiziden Eigenschaften bekannt, die sich von den erfindungsgemäßen Verbindungen durch die Substituenten im Pyridylteil unterscheiden. Aus EP-A-0 669 315 sind Ortho-substituierte Phenylessigsäureamide mit Insektiziden, Nematiziden und Akarazichen Eingenschaften sowie fungiziden Eigenschaften bekannt.

Demgegenüber lagen der vorliegenden Erfindung Verbindungen mit verbesserter Wirkung und verbreitertem Wirkungsspektrum als Aufgabe zugrunde.

Demgemäß wurden die eingangs definierten Verbindungen I gefunden. Desweiteren wurden Verfahren und Zwischenprodukte zur Herstellung dieser Verbindungen sowie ihre Verwendung zur Bekämpfung von tierischen Schädlingen und Schadpilzen gefunden.

Die Verbindungen I sind auf verschiedenen Wegen nach an sich in der Literatur beschriebenen Verfahren erhältlich.

Der Aufbau der Gruppierung Q ist beispielsweise aus der eingangs zitierten Literatur bekannt und erfolgt im allgemeinen und im besonderen nach den dort beschriebenen Verfahren.

Üblicherweise geht man bei der Synthese der Verbindungen I so vor, daß man ein Pyridinderivat der Formel IIa in einem inerten Lösungsmittel mit einem Phenol bzw. Benzylalkohol der Formel IIIa in den entsprechenden Ether der Formel IVa überführt und IV anschließend mit einem O-substituierten Hydroxylamin (R¹-O-NH₂) oder dessen Salz zu I umsetzt.

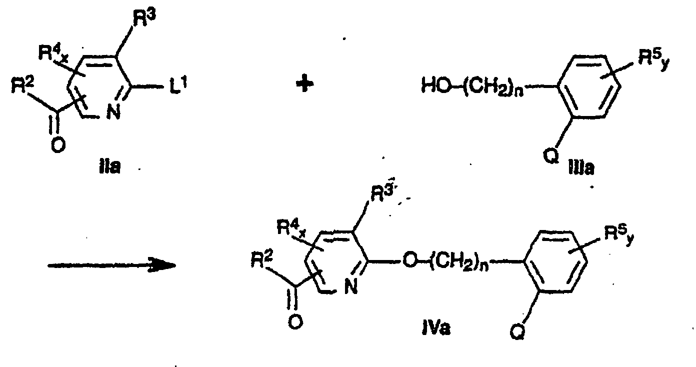

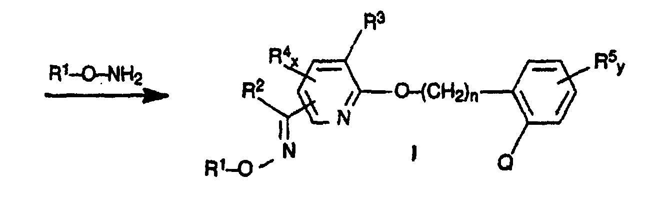

L¹ in der Formel IIa steht für eine nucleophil austauschbare Abgangsgruppe wie Halogen (z.B. Fluor, Chlor, Brom oder Iod) oder Alkyl- oder Arylsulfonat (z.B. Methylsulfonat, Trifluormethylsulfonat, Phenylsulfonat oder Methylphenylsulfonat).
1a) Die Umsetzung von IIa mit IIIa erfolgt üblicherweise in einem inerten Lösungsmittel bei Temperaturen von 0°C bis 130°C, vorzugsweise 20°C bis 80°C in Gegenwart einer Base.
   Geeignete Lösungsmittel sind aliphatische Kohlenwasserstoffe wie Pentan, Hexan, Cyclohexan und Petrolether, aromatische Kohlenwasserstoffe wie Toluol, o-, m- und p-Xylol, Ether wie Diethylether, Diisopropylether, tert.-Butylmethylether, Dioxan, Anisol und Tetrahydrofuran, Nitrile wie Acetonitril und Propionitril, Ketone wie Aceton, Methylethylketon, Diethylketon und tert.-Butylmethylketon, sowie Dimethylsulfoxid und Dimethylformamid, besonders bevorzugt Tetrahydrofuran, Acetonitril, Dimethylsulfoxid und Aceton. Es können auch Gemische der genannten Lösungsmittel verwendet werden.
   Als Basen kommen allgemein anorganische Verbindungen wie Alkalimetall- und Erdalkalimetallhydroxide wie Lithiumhydroxid, Natriumhydroxid, Kaliumhydroxid und Calziumhydroxid, Alkalimetall- und Erdalkalimetalloxide wie Lithiumoxid, Natriumoxid, Calziumoxid und Magnesiumoxid, Alkalimetall- und Erdalkalimetallhydride wie Lithiumhydrid, Natriumhydrid, Kaliumhydrid und Calziumhydrid, Alkalimetallamide wie Lithiumamid, Natriumamid und Kaliumamid, Alkalimetall- und Erdalkalimetallcarbonate wie Lithiumcarbonat und Calziumcarbonat sowie Alkalimetallhydrogencarbonate wie Natriumhydrogencarbonat, metallorganische Verbindungen, insbesondere Alkalimetallalkyle wie Methyllithium, Butyllithium und Phenyllithium, Alkylmagnesiumhalogenide wie Methylmagnesiumchlorid sowie Alkalimetall- und Erdalkalimetallalkoholate wie Matriummethanolat, Natriumethanolat, Kaliumethanolat, Kalium- tert.-Butanolat und Dimethoxymagnesium, außerdem organische Basen, z.B. tertiäre Amine wie Trimethylamin, Triethylamin, Tri-isopropylethylamin und N-Methylpiperidin, Pyridin, substituierte Pyridine wie Collidin, Lutidin und 4-Dimethylaminopyridin sowie bicyclische Amine in Betracht. Besonders bevorzugt werden Kaliumcarbonat, Natriumhydrid und Kalium-tert.-butylat. Die Basen werden im allgemeinen in katalytischen Mengen eingesetzt, sie können aber auch äquimolar, im Überschuß oder gegebenenfalls als Lösungsmittel verwendet werden.
   Die Edukte werden im allgemeinen in äquimolaren Mengen miteinander umgesetzt. Es kann für die Ausbeute vorteilhaft sein, IIa in einem Überschuß bezogen auf IIIa einzusetzen.
1b) Die Umsetzung von IVa mit dem O-substituierten Hydroxylamin oder dessen Salz erfolgt üblicherweise in einem inerten Lösungsmittel bei Temperaturen von 0°C bis 80°C, vorzugsweise 20°C bis 60°C, ggf. in Gegenwart einer Säure oder ggf. in Gegenwart einer Base, wenn das O-substituierte Hydroxylamin aus seinem Salz freigesetzt wird.
   Geeignete Lösungsmittel sind aliphatische Kohlenwasserstoffe wie Pentan, Hexan, Cyclohexan und Petrolether, aromatische Kohlenwasserstoffe wie Toluol, o-,'m- und p-Xylol, halogenierte Kohlenwasserstoffe wie Methylenchlorid, Chloroform und Chlorbenzol, Ether wie Diethylether, Diisopropylether, tert.-Butylmethylether, Dioxan, Anisol und Tetrahydrofuran, Nitrile wie Acetonitril und Propionitril, Alkohole wie Methanol, Ethanol, n-Propanol, Isopropanol, n-Butanol und tert.-Butanol sowie Dimethylsulfoxid, Dimethylformamid und Pyridin, besonders bevorzugt Methanol und Pyridin. Es können auch Gemische der genannten Lösungsmittel verwendet werden.
   Als Basen kommen allgemein anorganische Verbindungen wie Alkalimetall- und Erdalkalimetallhydroxide wie Lithiumhydroxid, Natriumhydroxid, Kaliumhydroxid und Calziumhydroxid, Alkalimetall- und Erdalkalimetalloxide wie Lithiumoxid, Natriumoxid, Calziumoxid und Magnesiumoxid, Alkalimetall- und Erdalkalimetallhydride wie Lithiumhydrid, Natriumhydrid, Kaliumhydrid und Calziumhydrid, Alkalimetallamide wie Lithiumamid, Natriumamid und Kaliumamid, Alkalimetall- und Erdalkalimetallcarbonate wie Lithiumcarbonat und Calziumcarbonat sowie Alkalimetallhydrogencarbonate wie Natriumhydrogencarbonat, metallorganische Verbindungen, insbesondere Alkalimetallalkyle wie Methyllithium, Butyllithium und Phenyllithium, A1-kylmagnesiumhalogenide wie Methylmagnesiumchlorid sowie Alkalimetall- und Erdalkalimetallalkoholate wie Natriummethanolat, Natriumethanolat, Kaliumethanolat, Kalium- tert.-Butanolat und Dimethoxymagnesium, außerdem organische Basen, z.B. tertiäre Amine wie Trimethylamin, Triethylamin, Tri-isopropylethylamin und N-Methylpiperidin, Pyridin, substituierte Pyridine wie Collidin, Lutidin und 4-Dimethylaminopyridin sowie bicyclische Amine in Betracht. Besonders bevorzugt werden Pyridin und Natriumhydroxid. Die Basen werden im allgemeinen in katalytischen Mengen eingesetzt, sie können aber auch äquimolar, im Überschuß oder gegebenenfalls als Lösungsmittel verwendet werden.
   Als Säuren und saure Katalysatoren finden anorganische Säuren wie Fluorwasserstoffsäure, Salzsäure, Bromwasserstoffsäure, Schwefelsäure und Perchlorsäure, Lewis-Säuren wie Bortrifluorid, Aluminiumtrichlorid, Eisen-III-chlorid, Zinn-IV-chlorid, Titan-IV-chlorid und Zink-II-chlorid, sowie organische Säuren wie Ameisensäure, Essigsäure, Propionsäure, Oxalsäure, Zitronensäure und Trifluoressigsäure Verwendung. Die Säuren werden im allgemeinen in katalytischen Mengen eingesetzt.
   Die Edukte werden im allgemeinen in äquimolaren Mengen miteinander umgesetzt. Es kann für die Ausbeute vorteilhaft sein, das Hydroxylamin bzw. dessen Salz in einem Überschuß bezogen auf IVa einzusetzen.

In entsprechender Weise erhält man die Verbindungen I dadurch, daß man ein Pyridinderivat der Formel IIa zunächst mit einem O-substituierten Hydroxylamin (R¹-O-NH₂) oder dessen Salz in die entsprechende Verbindung der Formel Va überführt und Va anschließend in einem inerten Lösungsmittel mit einem Phenol bzw. Benzylalkohol der Formel IIIa zu I umsetzt.

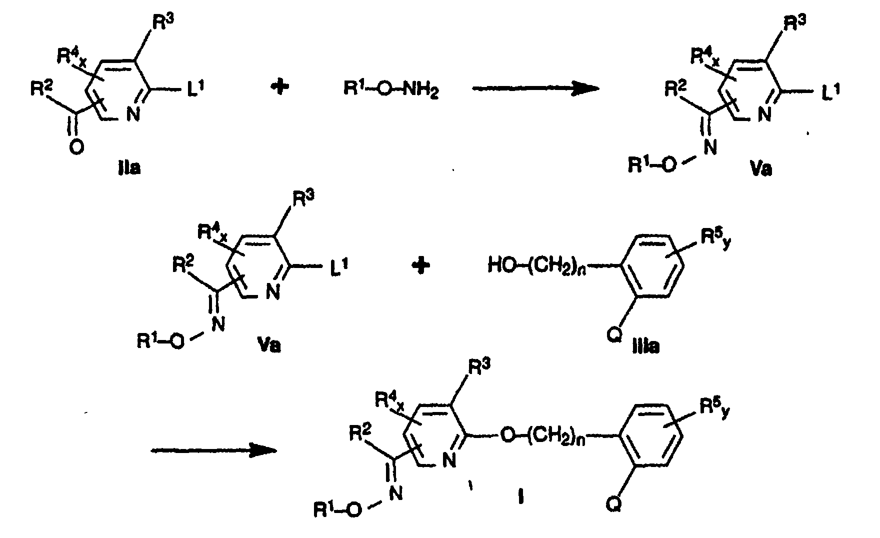

Die Umsetzungen erfolgen im allgemeinen und im besonderen nach den vorstehend beschriebenen Methoden.

Verbindungen I, in denen n für 1 steht, erhält man bevorzugt dadurch, daß man einen Pyridinalkohol der Formel IIb in einem inerten Lösungsmittel mit einer Benzylverbindung der Formel IIIb in den entsprechenden Benzylether der Formel IVb überführt und IVb anschließend mit einem O-substituierten Hydroxylamin (R¹-O-NH₂) oder dessen Salz zu I umsetzt.

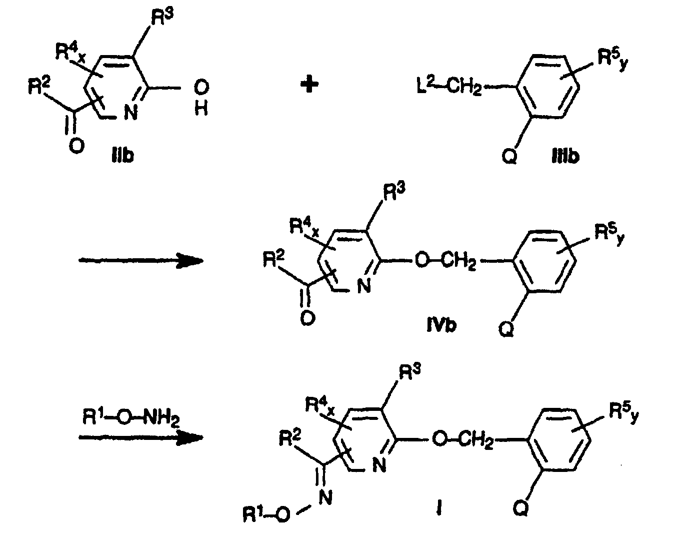

L² in der Formel IIIb steht für eine nucleophil austauschbare Abgangsgruppe wie Halogen (z.B. Chlor, Brom oder Iod) oder Alkyloder Arylsulfonat (z.B. Methylsulfonat, Trifluormethylsulfonat, Phenylsulfonat oder Methylphenylsulfonat).
2a) Die Umsetzung von IIb mit IIIb erfolgt üblicherweise in einem inerten Lösungsmittel bei Temperaturen von 0°C bis 130°C, vorzugsweise 20°C bis 60°C in Gegenwart einer Base.
   Geeignete Lösungsmittel sind aliphatische Kohlenwasserstoffe wie Pentan, Hexan, Cyclohexan und Petrolether, aromatische Kohlenwasserstoffe wie Toluol, o-, m- und p-Xylol, halogenierte Kohlenwasserstoffe wie Methylenchlorid, Chloroform und Chlorbenzol, Ether wie Diethylether, Diisopropylether, tert.-Butylmethylether, Dioxan, Anisol und Tetrahydrofuran, Nitrile wie Acetonitril und Propionitril, Ketone wie Aceton, Methylethylketon, Diethylketon und tert.-Butylmethylketon, sowie Dimethylsulfoxid und Dimethylformamid, besonders bevorzugt Tetrahydrofuran, Acetonitril, Dimethylsulfoxid und Aceton. Es können auch Gemische der genannten Lösungsmittel verwendet werden.
   Als Basen kommen allgemein anorganische Verbindungen wie Alkalimetall- und Erdalkalimetallhydroxide wie Lithiumhydroxid, Natriumhydroxid, Kaliumhydroxid und Calziumhydroxid, Alkalimetall- und Erdalkalimetalloxide wie Lithiumoxid, Natriumoxid, Calziumoxid und Magnesiumoxid, Alkalimetall- und Erdalkalimetallhydride wie Lithiumhydrid, Natriumhydrid, Kaliumhydrid und Calziumhydrid, Alkalimetallamide wie Lithiumamid, Natriumamid und Kaliumamid, Alkalimetall- und Erdalkalimetallcarbonate wie Lithiumcarbonat und Calziumcarbonat sowie Alkalimetallhydrogencarbonate wie Natriumhydrogencarbonat, metallorganische Verbindungen, insbesondere Alkalimetallalkyle wie Methyllithium, Butyllithium und Phenyllithium, Al-kylmagnesiumhalogenide wie Methylmagnesiumchlorid sowie Alkalimetall- und Erdalkalimetallalkoholate wie Natriummethanolat, Natriumethanolat, Kaliumethanolat, Kalium- tert.-Butanolat und Dimethoxymagnesium, außerdem organische Basen, z.B. tertiäre Amine wie Trimethylamin, Triethylamin, Tri-isopropylethylamin und N-Methylpiperidin, Pyridin, substituierte Pyridine wie Collidin, Lutidin und 4-Dimethylaminopyridin sowie bicyclische Amine in Betracht. Besonders bevorzugt werden Kaliumcarbonat, Natriumhydrid und Kalium-tert.-butylat. Die Basen werden im allgemeinen in katalytischen Mengen eingesetzt, sie können aber auch äquimolar, im Überschuß oder gegebenenfalls als Lösungsmittel verwendet werden.
   Die Edukte werden im allgemeinen in äquimolaren Mengen miteinander umgesetzt. Es kann für die Ausbeute vorteilhaft sein, IIb in einem Überschuß bezogen auf IIIb einzusetzen.
2b) Die Umsetzung von IVb mit dem O-substituierten Hydroxylamin oder dessen Salz erfolgt im allgemeinen und im besonderen nach den vorstehend unter Punkt 1b beschriebenen Bedingungen.
   Geeignete Lösungsmittel sind aliphatische Kohlenwasserstoffe wie Pentan, Hexan, Cyclohexan und Petrolether, aromatische Kohlenwasserstoffe wie Toluol, o-, m- und p-Xylol, halogenierte Kohlenwasserstoffe wie Methylenchlorid, Chloroform und Chlorbenzol, Ether wie Diethylether, Diisopropylether, tert.-Butylmethylether, Dioxan, Anisol und Tetrahydrofuran, Nitrile wie Acetonitril und Propionitril, Alkohole wie Methanol, Ethanol, n-Propanol, Isopropanol, n-Butanol und tert.-Butanol sowie Dimethylsulfoxid, Dimethylformamid und Pyridin, besonders bevorzugt Methanol und Pyridin. Es können auch Gemische der genannten Lösungsmittel verwendet werden.
   Als Basen kommen allgemein anorganische Verbindungen wie Alkalimetall- und Erdalkalimetallhydroxide wie Lithiumhydroxid, Natriumhydroxid, Kaliumhydroxid und Calziumhydroxid, Alkalimetall- und Erdalkalimetalloxide wie Lithiumoxid, Natriumoxid, Calziumoxid und Magnesiumoxid, Alkalimetall- und Erdalkalimetallhydride wie Lithiumhydrid, Natriumhydrid, Kaliumhydrid und Calziumhydrid, Alkalimetallamide wie Lithiumamid, Natriumamid und Kaliumamid, Alkalimetall- und Erdalkalimetallcarbonate wie Lithiumcarbonat und Calziumcarbonat sowie Alkalimetallhydrogencarbonate wie Natriumhydrogencarbonat, metallorganische Verbindungen, insbesondere Alkalimetallalkyle wie Methyllithium, Butyllithium und Phenyllithium, Alkylmagnesiumhalogenide wie Methylmagnesiumchlorid sowie Alkalimetall- und Erdalkalimetallalkoholate wie Natriummethanolat, Natriumethanolat, Kaliumethanolat, Kalium- tert.-Butanolat und Dimethoxymagnesium, außerdem organische Basen, z.B. tertiäre Amine wie Trimethylamin, Triethylamin, Tri-isopropylethylamin und N-Methylpiperidin, Pyridin, substituierte Pyridine wie Collidin, Lutidin und 4-Dimethylaminopyridin sowie bicyclische Amine in Betracht. Besonders bevorzugt werden Pyridin und Natriumhydroxid. Die Basen werden im allgemeinen in katalytischen Mengen eingesetzt, sie können aber auch äquimolar, im Überschuß oder gegebenenfalls als Lösungsmittel verwendet werden.
   Als Säuren und saure Katalysatoren finden anorganische Säuren wie Fluorwasserstoffsäure, Salzsäure, Bromwasserstoffsäure, Schwefelsäure und Perchlorsäure, Lewis-Säuren wie Bortrifluorid, Aluminiumtrichlorid, Eisen-III-chlorid, Zinn-IV-chlorid, Titan-IV-chlorid und Zink-II-chlorid, sowie organische Säuren wie Ameisensäure, Essigsäure, Propionsäure, Oxalsäure, Zitronensäure und Trifluoressigsäure Verwendung. Die Säuren werden im allgemeinen in katalytischen Mengen eingesetzt.
   Die Edukte werden im allgemeinen in äquimolaren Mengen miteinander umgesetzt. Es kann für die Ausbeute vorteilhaft sein, IIb in einem Überschuß bezogen auf IIIb einzusetzen.

In entsprechender Weise erhält man die Verbindungen I, in denen n für 1 steht, dadurch, daß man einen Pyridinalkohol der Formel IIb zunächst mit einem O-substituierten Hydroxylamin (R¹-O-NH₂) oder dessen Salz in die entsprechende Verbindung der Formel Vb überführt und Vb anschließend in einem inerten Lösungsmittel mit einer Benzylverbindung der Formel IIIb zu I umsetzt.

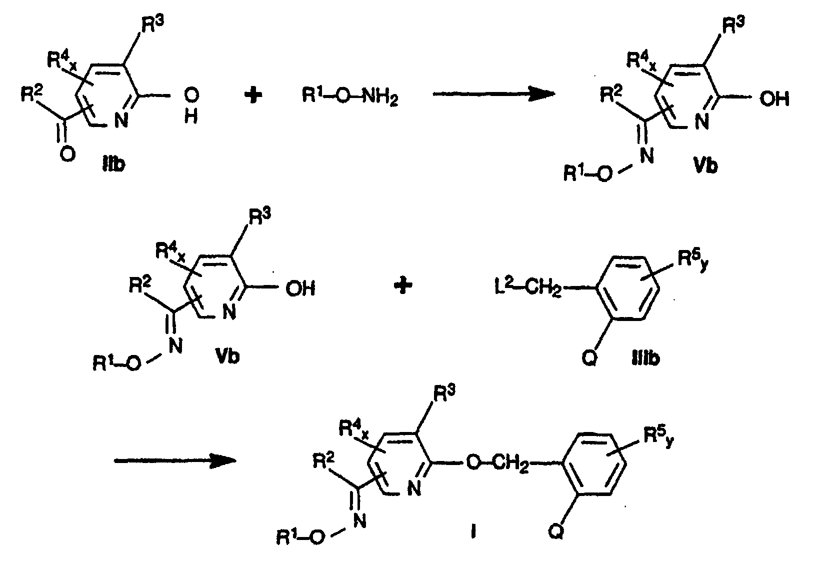

Die Umsetzungen erfolgen im allgemeinen und im besonderen nach den vorstehend beschriebenen Methoden.

Die für die Herstellung der Verbindungen I nach den vorstehend beschriebenen Verfahren benötigten Ausgangsstoffe der Formeln IIIa und IIIb sind aus der eingangs genannten Literatur bekannt oder können gemäß den dort beschriebenen Verfahren in analoger Weise hergestellt werden.

Die Ausgangsstoffe der Formel IIa können erhalten werden, indem man ein geeignet substituiertes Pyridin der Formel VIa in einem inerten Lösungsmittel in Gegenwart einer metallorganischen Base mit einer aktivierten Carbonsäure der Formel VIIa umsetzt [vgl. J. Organomet. Chem. 56, 53-66 (1973); Chem. Ber. 125, 1169-1190 (1992)].

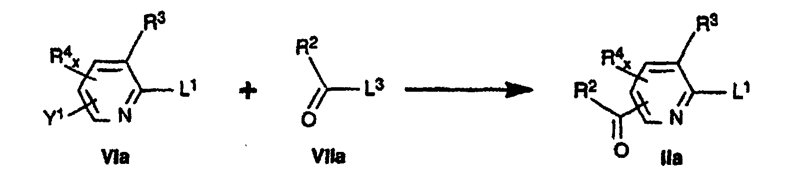

Y¹ in der Formel VIa steht für ein Halogenatom, z.B. Fluor, Chlor, Brom und Iod, besonders Brom und Iod.

L³ in der Formel VII steht für ein Halogenatom, z.B. Fluor, Chlor, Brom und Iod, besonders Chlor, oder einen Amid- oder einen Esterrest. Anstelle der Verbindung VIIa kann auch ein entsprechendes Cyanid R²-C≡N eingesetzt werden.

Diese Umsetzung erfolgt üblicherweise in einem inerten Lösungsmittel in Gegenwart einer metallorganischen Basebei Temperaturen von -75°C bis 40°C, vorzugsweise -75°C bis 0°C.

Geeignete Lösungsmittel sind aliphatische Kohlenwasserstoffe wie Pentan, Hexan, Cyclohexan und Petrolether, aromatische Kohlenwasserstoffe wie Toluol, o-, m- und p-Xylol, Ether wie Diethylether, Diisopropylether, tert.-Butylmethylether, Dioxan, Anisol und Tetrahydrofuran, sowie Dimethylsulfoxid und Dimethylformamid, besonders bevorzugt Diethylether und Tetrahydrofuran. Es können auch Gemische der genannten Lösungsmittel verwendet werden.

Als metallorganische Basen kommen allgemein metallorganische Verbindungen, insbesondere Alkalimetallalkyle wie Methyllithium, Butyllithium und Phenyllithium, Alkylmagnesiumhalogenide wie Methylmagnesiumchlorid sowie in Betracht. Besonders bevorzugt werden n-Butyllithium. Die Basen können im allgemeinen äquimolar oder im Überschuß verwendet werden.

Die Edukte werden im allgemeinen in äquimolaren Mengen miteinander umgesetzt. Es kann für die Ausbeute vorteilhaft sein, VIIa in einem Überschuß bezogen auf VIa einzusetzen.

Nach einer weiteren Methode erhält man die Verbindungen IIa auch dadurch, daß man ein Pyridincarbonsäure-halogenid der allgemeinen Formel VIIIa in einem inerten Lösungsmittel mit einer metallorganischen Verbindung (R²-M; M steht für das Äquivalent eines Metallions) umsetzt [vgl. DE-A 38 38 243; EP-A 446 872].

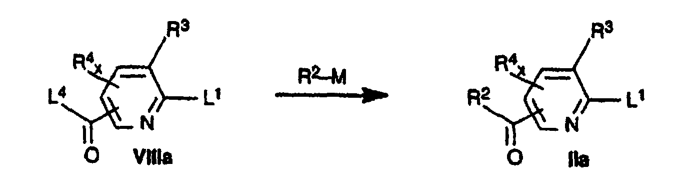

Als Metall eignen sich besonders Litium, Magnesium, Kupfer und Zink.

L⁴ in der Formel VIIIa steht für ein Halogenatom, z.B. Fluor, Chlor, Brom und Iod, besonders Chlor.

Diese Umsetzung erfolgt üblicherweise in einem inerten Lösungsmittel bei Temperaturen von -80°C bis 20°C, vorzugsweise -75°C bis 0°C.

Geeignete Lösungsmittel sind aliphatische Kohlenwasserstoffe wie Pentan, Hexan, Cyclohexan und Petrolether, aromatische Kohlenwasserstoffe wie Toluol, o-, m- und p-Xylol, Ether wie Diethylether, Diisopropylether, tert.-Butylmethylether, Dioxan, Anisol und Tetrahydrofuran, besonders bevorzugt Diethylether und Tetrahydrofuran. Es können auch Gemische der genannten Lösungsmittel verwendet werden.

Die Edukte werden im allgemeinen in äquimolaren Mengen miteinander umgesetzt. Es kann für die Ausbeute vorteilhaft sein, die metallorganische Verbindung in einem Überschuß bezogen auf VIIIa einzusetzen.

Nach einer weiteren Methode erhält man die Verbindungen IIa auch dadurch, daß man ein Pyridincarbonsäure-halogenid der allgemeinen Formel VIIIa in einem inerten Lösungsmittel mit einem Malonsäureester der Formel IX in das entsprechnede Triketon VIIIb überführt und VIIIb anschließend zu IIa umsetzt [vgl. Tetrahedron 48 (22), 9233 (1992)].

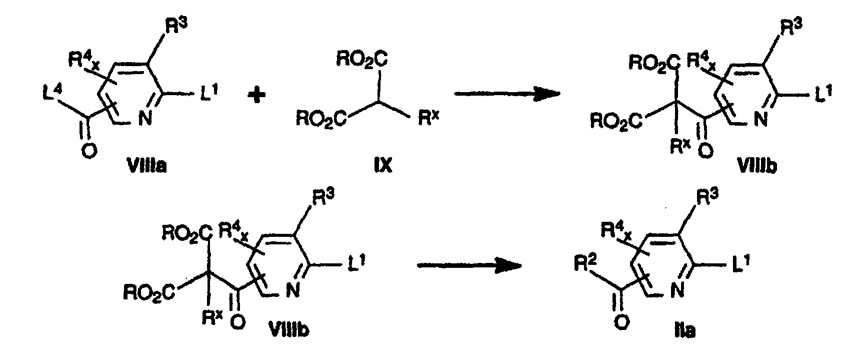

R^{x} in den Formeln VIIIb und IX steht für den Rest einer Gruppe R².

R in den Formeln VIIIb und IX steht für voneinander unabhängige C₁-C₄-Alkylgruppen, insbesondere Methyl und Ethyl.
3a) Die Umsetzung von VIIIa und IX erfolgt üblicherweise in einem inerten Lösungsmittel bei Temperaturen von 0°C bis 120°C, vorzugsweise 20°C bis 80°C in Gegenwart einer Base und ggf. in Gegenwart einer Lewis-Säure wie Magnesium-chlorid.
   Geeignete Lösungsmittel sind aliphatische Kohlenwasserstoffe wie Pentan, Hexan, Cyclohexan und Petrolether, aromatische Kohlenwasserstoffe wie Toluol, o-, m- und p-Xylol, halogenierte Kohlenwasserstoffe wie Methylenchlorid, Chloroform und Chlorbenzol, Ether wie Diethylether, Diisopropylether, tert.-Butylmethylether, Dioxan, Anisol und Tetrahydrofuran, Nitrile wie Acetonitril und Propionitril, Ketone wie Aceton, Methylethylketon, Diethylketon und tert.-Butylmethylketon, sowie Dimethylsulfoxid und Dimethylformamid, besonders bevorzugt Toluol. Es können auch Gemische der genannten Lösungsmittel verwendet werden.
   Als Basen kommen allgemein anorganische Verbindungen wie Alkalimetall- und Erdalkalimetallhydroxide wie Lithiumhydroxid, Natriumhydroxid, Kaliumhydroxid und Calziumhydroxid, Alkalimetall- und Erdalkalimetalloxide wie Lithiumoxid, Natriumoxid, Calziumoxid und Magnesiumoxid, Alkalimetall- und Erdalkalimetallhydride wie Lithiumhydrid, Natriumhydrid, Kaliumhydrid und Calziumhydrid, Alkalimetallamide wie Lithiumamid, Natriumamid und Kaliumamid, Alkalimetall- und Erdalkalimetallcarbonate wie Lithiumcarbonat und Calziumcarbonat sowie Alkalimetallhydrogencarbonate wie Natriumhydrogencarbonat, metallorganische Verbindungen, insbesondere Alkalimetallalkyle wie Methyllithium, Butyllithium und Phenyllithium, Alkylmagnesiumhalogenide wie Methylmagnesiumchlorid sowie Alkalimetall- und Erdalkalimetallalkoholate wie Natriummethanolat, Natriumethanolat, Kaliumethanolat, Kalium- tert.-Butanolat und Dimethoxymagnesium, außerdem organische Basen, z.B. tertiäre Amine wie Trimethylamin, Triethylamin, Tri-isopropylethylamin und N-Methylpiperidin, Pyridin, substituierte Pyridine wie Collidin, Lutidin und 4-Dimethylaminopyridin sowie bicyclische Amine in Betracht. Besonders bevorzugt werden Natriumhydroxid und Triethylamin. Die Basen werden im allgemeinen äquimolar eingesetzt, sie können aber auch im Überschuß oder gegebenenfalls als Lösungsmittel verwendet werden.
   Die Edukte werden im allgemeinen in äquimolaren Mengen miteinander umgesetzt. Es kann für die Ausbeute vorteilhaft sein, IX in einem Überschuß bezogen auf VIIIa einzusetzen.
3b) Die Decarboxylierung von VIIIb zu IIa erfolgt üblicherweise in einem inerten Lösungsmittel bei Temperaturen von 60°C bis 200°C, vorzugsweise 100°C bis 160°C ggf. in Gegenwart einer Base.
   Geeignete Lösungsmittel sind aromatische Kohlenwasserstoffe wie Toluol, o-, m- und p-Xylol, halogenierte Kohlenwasserstoffe wie Chlorbenzol, Nitrile wie Acetonitril und Propionitril, Alkohole wie Methanol, Ethanol, n-Propanol, Isopropanol, n-Butanol und tert.-Butanol sowie Dimethylsulfoxid und Dimethylformamid, besonders bevorzugt Wasser und Dimethylsulfoxid. Es können auch Gemische der genannten Lösungsmittel verwendet werden.
   Als Basen kommen allgemein anorganische Verbindungen wie Alkalimetall- und Erdalkalimetallhydroxide wie Lithiumhydroxid, Natriumhydroxid, Kaliumhydroxid und Calziumhydroxid, Alkalimetall- und Erdalkalimetalloxide wie Lithiumoxid, Natriumoxid, Calziumoxid und Magnesiumoxid, Alkalimetall- und Erdalkalimetallhydride wie Lithiumhydrid, Natriumhydrid, Kaliumhydrid und Calziumhydrid, Alkalimetallamide wie Lithiumamid, Natriumamid und Kaliumamid, Alkalimetall- und Erdalkalimetallcarbonate wie Lithiumcarbonat und Calziumcarbonat sowie Alkalimetallhydrogencarbonate wie Natriumhydrogencarbonat, metallorganische Verbindungen, insbesondere Alkalimetallalkyle wie Methyllithium, Butyllithium und Phenyllithium, Alkylmagnesiumhalogenide wie Methylmagnesiumchlorid sowie Alkalimetall- und Erdalkalimetallalkoholate wie Natriummethanolat, Natriumethanolat, Kaliumethanolat, Kalium- tert.-Butanolat und Dimethoxymagnesium, außerdem organische Basen, z.B. tertiäre Amine wie Trimethylamin, Triethylamin, Tri-isopropylethylamin und N-Methylpiperidin, Pyridin, substituierte Pyridine wie Collidin, Lutidin und 4-Dimethylaminopyridin sowie bicyclische Amine in Betracht. Besonders bevorzugt werden Natriumhydroxid und Natriummethanolat. Die Basen werden im allgemeinen in katalytischen Mengen eingesetzt, sie können aber auch äquimolar, im Überschuß oder gegebenenfalls als Lösungsmittel verwendet werden.

Die Ausgangsstoffe der Formel IIb erhält man dadurch, daß man ein geeignet substituiertes Pyridinderivat der Formel VIa in Gegenwart einer Base mit einem Alkoholat (R'-O- M⁺; R' bedeutet C₁-C₄-Alkyl, M⁺ steht für das Äquivalent eines Alkalimetall- oder Erdalkalimetall-kations, insbesondere Natrium oder Kalium) in den entsprechenden Alkyl-pyridylether der Formel VIb überfüht, VIb anschließend in Analogie zum vorstehend beschriebenen Verfahren (Umsetzung von VIa) durch Umsetzung mit einer aktivierten Carbonsäure der Formel VIIa in den entsprechenden Ether IIc überführt und IIc anschließend zu IIb spaltet. 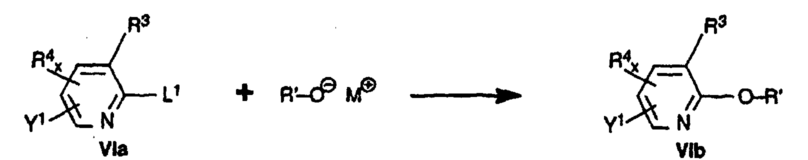 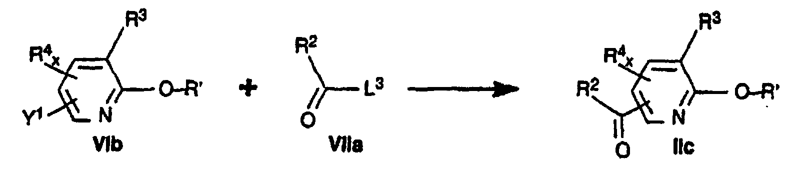 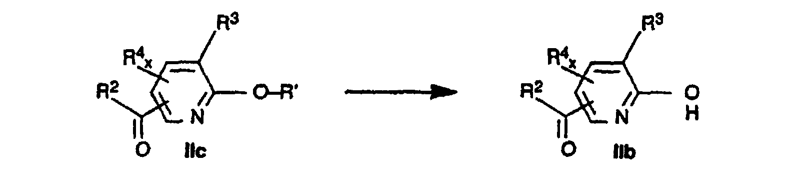
4a) Die Veretherung von VIa zu VIb erfolgt üblicherweise bei Temperaturen von 0°C bis 120°C, vorzugsweise 20°C bis 80°C in Gegenwart eines inerten Lösungsmittels.
   Geeignete Lösungsmittel sind aliphatische Kohlenwasserstoffe wie Pentan, Hexan, Cyclohexan und Petrolether, aromatische Kohlenwasserstoffe wie Toluol, o-, m- und p-Xylol, halogenierte Kohlenwasserstoffe wie Methylenchlorid, Chloroform und Chlorbenzol, Ether wie Diethylether, Diisopropylether, tert.-Butylmethylether, Dioxan, Anisol und Tetrahydrofuran, Nitrile wie Acetonitril und Propionitril, Ketone wie Aceton, Methylethylketon, Diethylketon und tert.-Butylmethylketon, sowie Dimethylsulfoxid und Dimethylformamid, besonders bevorzugt Dimethylformamid. Es können auch Gemische der genannten Lösungsmittel verwendet werden.
   Das Pyridinderivat VIa und das Alkoholat werden im allgemeinen in äquimolaren Mengen miteinander umgesetzt. Es kann für die Ausbeute vorteilhaft sein, das Alkoholat in einem Überschuß bezogen auf VIa oder als Lösungsmittel einzusetzen.
4b) Die Umsetzung des Ethers VIb mit der aktivierten Carbonsäure VIIa erfolgt im allgemeinen und im besonderen unter den bei der Herstellung der Verbindungen IIa aus den Verbindungen VIa beschriebenen Bedingungen.
4c) Die Etherspaltung von IIc nach IIb erfolgt üblicherweise in eienm inerten Lösungsmittel bei Temperaturen von 0°C bis 130°C, vorzugsweise 60°C bis 100°C in Gegenwart einer Säure.
   Geeignete Lösungsmittel sind aliphatische Kohlenwasserstoffe wie Pentan, Hexan, Cyclohexan und Petrolether, aromatische Kohlenwasserstoffe wie Toluol, o-, m- und p-Xylol, halogenierte Kohlenwasserstoffe wie Methylenchlorid, Chloroform und Chlorbenzol, Nitrile wie Acetonitril und Propionitril, Alkohole wie Methanol, Ethanol, n-Propanol, Isopropanol, n-Butanol und tert.-Butanol sowie Dimethylsulfoxid und Dimethylformamid, besonders bevorzugt Methylenchlorid. Es können auch Gemische der genannten Lösungsmittel verwendet werden.
   Als Säuren und saure Katalysatoren finden anorganische Säuren wie Fluorwasserstoffsäure, Salzsäure, Bromwasserstoffsäure, Schwefelsäure und Perchlorsäure, Lewis-Säuren wie Bortrifluorid, Aluminiumtrichlorid, Eisen-III-chlorid, Zinn-IV-chlorid, Titan-IV-chlorid und Zink-II-chlorid, sowie organische Säuren wie Ameisensäure, Essigsäure, Propionsäure, Oxalsäure, Zitronensäure und Trifluoressigsäure Verwendung. Die Säuren werden im allgemeinen in katalytischen Mengen eingesetzt, sie können aber auch äquimolar, im Überschuß oder gegebenenfalls als Lösungsmittel verwendet werden.

Die Zwischenprodukte der Formel IVa können außerdem dadurch erhalten werden, daß man einen Ether der Formel Xa entweder
a) in Gegenwart einer metallorganischen Base in einem inerten Lösungsmittel mit einer aktivierten Carbonsäure der Formel XI oder
b) in einem inerten Lösungsmittel mit einer Zinn-organischen Verbindung der Formel XII
umsetzt.

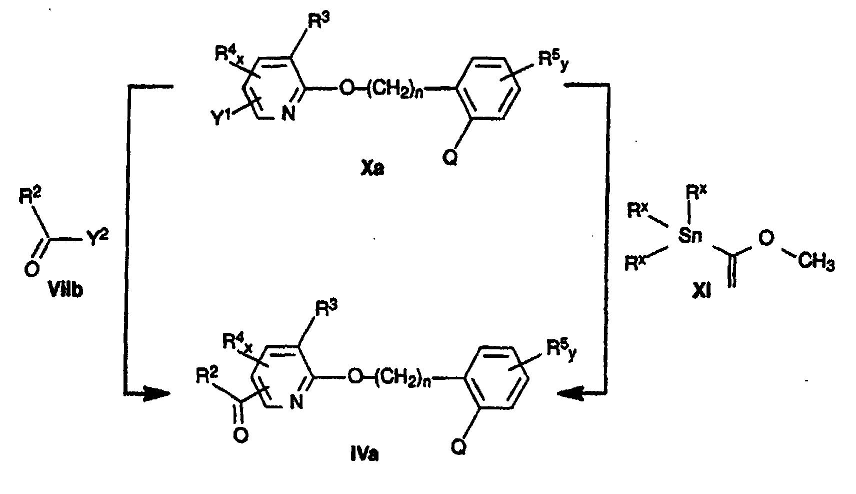

Y¹ in der Formel Xa steht für ein Halogenatom wie Fluor, Chlor, Brom und Iod, insbesondere Brom und Iod.

Y² in der Formel VIIb steht für ein Halogenatom wie Fluor, Chlor, Brom und Iod, insbesondere Chlor.

Die Reste R^{x} in der Formel XI sind voneinander unabhängig und stehen für Alkyl.
5a) Die Umsetzung des Ethers Xa mit der aktivierten Carbonsäure VIIb erfolgt im allgemeinen und im besonderen unter den bei der Herstellung der Verbindungen IIa aus den Verbindungen VIa beschriebenen Bedingungen.
5b) Die Umsetzung des Ethers Xa mit der Zinn-organischen Verbindung XI erfolgt üblicherweise in einem inerten Lösungsmittel bei Temperaturen von -70°C bis 40°C, vorzugsweise -70°C bis 0°C in Gegenwart eines Katalysators wie Pd[P(C₆H₅)₃]₃ und PdCl₂.
   Geeignete Lösungsmittel sind aliphatische Kohlenwasserstoffe wie Pentan, Hexan, Cyclohexan und Petrolether, aromatische Kohlenwasserstoffe wie Toluol, o-, m- und p-Xylol, Ether wie Diethylether, Diisopropylether, tert.-Butylmethylether, Dioxan, Anisol und Tetrahydrofuran, Nitrile wie Acetonitril und Propionitril, sowie Dimethylsulfoxid und Dimethylformamid, besonders bevorzugt Tetrahydrofuran und Diethylether. Es können auch Gemische der genannten Lösungsmittel verwendet werden.
   Die Edukte werden im allgemeinen in äquimolaren Mengen miteinander umgesetzt. Es kann für die Ausbeute vorteilhaft sein, die Zinn-organische Verbindung XI in einem Überschuß bezogen auf den Ether Xa einzusetzen.

Im allgemeinen ist es bei der Herstellung der erfindungsgemäßen Verbindungen I unerheblich, ob die den Phenyl- bzw. Benzylteil enthaltenden Edukte und Zwischenprodukte (Formeln III, IV und X) sowie bei den Ethern der Formel I bereits die Gruppe Q enthalten oder ob in dieser Position eine Gruppe steht, die nach den in der eingangs zitierten Literatur beschriebenen Verfahren in Q überführt werden kann. Die Gruppe Q kann grundsätzlich auf jeder der genannten Stufen (Formeln III, IV, X und I) aufgebaut werden.

In den neuen Zwischenprodukten allgemeinen Formel II 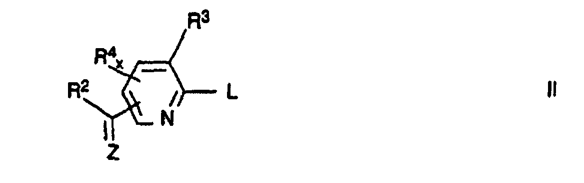 haben die Substituenten R², R³ und R⁴ und der Index x die eingangs gegebene Beutung haben und L und Z stehen für die folgenden Gruppen:
- L: Hydroxy oder eine nucleophil austauschbare Abgangsgruppe;
- Z: Sauerstoff oder eine Gruppe NOR¹, wobei R¹ die eingangs gegebene Bedeutung hat.

In den neuen Zwischenprodukten allgemeinen Formel X 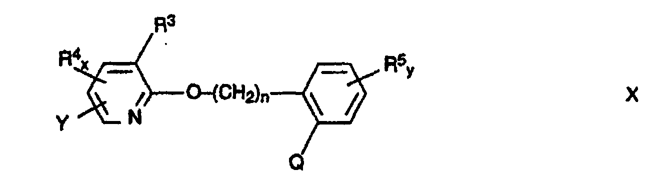 haben die Substituenten Q, R², R³, R⁴ und R⁵ und die Indices n, x und Y die eingangs gegebene Beutung haben und Y steht für die folgenden Gruppen: Halogen oder CO-R², wobei R² die eingangs gegebene Bedeutung hat.

Die Reaktionsgemische werden in üblicher Weise aufgearbeitet, z.B. durch Mischen mit Wasser, Trennung der Phasen und gegebenenfalls chromatographische Reinigung der Rohprodukte. Die Zwischenund Endprodukte fallen z.T. in Form farbloser oder schwach bräunlicher, zäher Öle an, die unter vermindertem Druck und bei mäßig erhöhter Temperatur von flüchtigen Anteilen befreit oder gereinigt werden. Sofern die Zwischen- und Endprodukte als Feststoffe erhalten werden, kann die Reinigung auch durch Umkristallisieren oder Digerieren erfolgen.

Die Verbindungen I können bei der Herstellung aufgrund ihrer C=Cund C=N-Doppelbindungen als E/Z-Isomerengemische anfallen, die z.B. durch Kristallisation oder Chromatographie in üblicher Weise in die Einzelverbindungen getrennt werden können.

Sofern bei der Synthese Isomerengemische anfallen, ist im allgemeinen jedoch eine Trennung nicht unbedingt erforderlich, da sich die einzelnen Isomere teilweise während der Aufbereitung für die Anwendung oder bei der Anwendung (z.B. unter Licht-, Säure- oder Baseneinwirkung) ineinander umwandeln können. Entsprechende Umwandlungen können auch nach der Anwendung, beispielsweise bei der Behandlung von Pflanzen in der behandelten Pflanze oder im zu bekämpfenden Schadpilz oder tierischen Schädling erfolgen.

In Bezug auf die C=NOR¹-Doppelbindung werden hinsichtlich ihrer Wirksamkeit die E-Isomere der Verbindungen I bevorzugt (Konfiguration bezogen auf die R²-Gruppe im Verhältnis zur OR¹-Gruppe).

Bei den in den vorstehenden Formeln angegebenen Definitionen der Symbole wurden Sammelbegriffe verwendet, die allgemein repräsentativ für die folgenden Substituenten stehen:
**Halogen:** Fluor, Chlor, Brom und Jod;
C₁-C₆ **Alkyl:** gesättigte, geradkettige oder verzweigte Kohlenwasserstoffreste mit 1 bis 6 Kohlenstoffatomen, z.B. C₁-C₆-Alkyl wie Methyl, Ethyl, Propyl, 1-Methylethyl, Butyl, 1-Methyl-propyl, 2-Methylpropyl, 1,1-Dimethylethyl, Pentyl, 1-Methylbutyl, 2-Methylbutyl, 3-Methylbutyl, 2,2-Di-methylpropyl, 1-Ethylpropyl, Hexyl, 1,1-Dimethylpropyl, 1,2-Dimethylpropyl, 1-Methylpentyl, 2-Methylpentyl, 3-Methylpentyl, 4-Methylpentyl, 1,1-Dimethylbutyl, 1,2-Dimethylbutyl, 1,3-Dimethylbutyl, 2,2-Dimethylbutyl, 2,3-Dimethylbutyl, 3,3-Dimethylbutyl, 1-Ethylbutyl, 2-Ethylbutyl, 1,1,2-Trimethylpropyl, 1,2,2-Trimethylpropyl, 1-Ethyl-1-methylpropyl und 1-Ethyl-2-methylpropyl;
C₁-C₆ **Halogenalkyl:** geradkettige oder verzweigte Alkylgruppen mit 1 bis 6 Kohlenstoffatomen (wie vorstehend genannt), wobei in diesen Gruppen teilweise oder vollständig die Wasserstoffatome durch Halogenatome wie vorstehend genannt ersetzt sein können, z.B. C₁-C₂-Halogenalkyl wie Chlormethyl, Brommethyl, Dichlormethyl, Trichlormethyl, Fluormethyl, Difluormethyl, Trifluormethyl, Chlorfluormethyl, Dichlorfluormethyl, Chlordifluormethyl, 1-Chlorethyl, 1-Bromethyl, 1-Fluorethyl, 2-Fluorethyl, 2,2-Difluorethyl, 2,2,2-Trifluorethyl, 2-Chlor-2-fluorethyl, 2-Chlor-2,2-difluorethyl, 2,2-Dichlor-2-fluorethyl, 2,2,2-Trichlorethyl und Pentafluorethyl;
C₁-C₄ **Alkoxy:** geradkettige oder verzweigte Alkylgruppen mit 1 bis 4 Kohlenstoffatomen (wie vorstehend genannt), welche über ein Sauerstoffatom (-O-) an das Gerüst gebunden sind;
C₂-C₆ **Alkenyl:** ungesättigte, geradkettige oder verzweigte Kohlenwasserstoffreste mit 2 bis 6, Kohlenstoffatomen und einer Doppelbindung in einer beliebigen Position, z.B. C₂-C₆-Alkenyl wie Ethenyl, 1-Propenyl, 2-Propenyl, 1-Methylethenyl, 1-Butenyl, 2-Butenyl, 3-Butenyl, 1-Methyl-1-propenyl, 2-Methyl-1-propenyl, 1-Methyl-2-propenyl, 2-Methyl-2-propenyl, 1-Pentenyl, 2-Pentenyl, 3-Pentenyl, 4-Pentenyl, 1-Methyl-1-butenyl, 2-Methyl-1-butenyl, 3-Methyl-1-butenyl, 1-Methyl-2-butenyl, 2-Methyl-2-butenyl, 3-Methyl-2-butenyl, 1-Methyl-3-butenyl, 2-Methyl-3-butenyl, 3-Methyl-3-butenyl, 1,1-Dimethyl-2-propenyl, 1,2-Dimethyl-1-propenyl, 1,2-Dimethyl-2-propenyl, 1-Ethyl-1-propenyl, 1-Ethyl-2-propenyl, 1-Hexenyl, 2-Hexenyl, 3-Hexenyl, 4-Hexenyl, 5-Hexenyl, 1-Methyl-1-pentenyl, 2-Methyl-1-pentenyl, 3-Methyl-1-pentenyl, 4-Methyl-1-pentenyl, 1-Methyl-2-pentenyl, 2-Methyl-2-pentenyl, 3-Methyl-2-pentenyl, 4-Methyl-2-pentenyl, 1-Methyl-3-pentenyl, 2-Methyl-3pentenyl, 3-Methyl-3-pentenyl, 4-Methyl-3-pentenyl, 1-Methyl-4-pentenyl, 2-Methyl-4-pentenyl, 3-Methyl-4-pentenyl, 4-Methyl-4-pentenyl, 1,1-Dimethyl-2-butenyl, 1,1-Dimethyl-3-butenyl, 1,2-Dimethyl-1-butenyl, 1,2-Dimethyl-2-butenyl, 1,2-Dimethyl-3-butenyl, 1,3-Dimethyl-1-butenyl, 1,3-Dimethyl-2-butenyl, 1,3-Dimethyl-3-butenyl, 2,2-Dimethyl-3-butenyl, 2,3-Dimethyl-1-butenyl, 2,3-Dimethyl-2-butenyl, 2,3-Dimethyl-3-butenyl, 3,3-Dimethyl-1-butenyl, 3,3-Dimethyl-2-butenyl, 1-Ethyl-1-butenyl, 1-Ethyl-2-butenyl, 1-Ethyl-3-butenyl, 2-Ethyl-1-butenyl, 2-Ethyl-2-butenyl, 2-Ethyl-3-butenyl, 1,1,2-Trimethyl-2-propenyl, 1-Ethyl-l-methyl-2-propenyl, 1-Ethyl-2-methyl-1propenyl und 1-Ethyl-2-methyl-2-propenyl;
C₃-C₆ **Halogenalkenyl:** ungesättigte, geradkettige oder verzweigte Kohlenwasserstoffreste mit 3 bis 10 Kohlenstoffatomen und einer Doppelbindung in einer beliebigen Position (wie vorstehend genannt), wobei in diesen Gruppen die Wasserstoffatome teilweise oder vollständig gegen Halogenatome wie vorstehend genannt, insbesondere Fluor, Chlor und Brom, ersetzt sein können;
C₃-C₆ **Alkinyl:** geradkettige oder verzweigte Kohlenwasserstoffgruppen mit 3 bis 6, Kohlenstoffatomen und einer Dreifachbindung in einer beliebigen Position, z.B. 1-Propinyl, 2-Propinyl, 1-Butinyl, 2-Butinyl, 3-Butinyl, 1-Methyl-2-propinyl, 1-Pentinyl, 2-Pentinyl, 3-Pentinyl, 4-Pentinyl, 1-Methyl-2-butinyl 1-Methyl-3-butinyl 2-Methyl-3-butinyl, 3-Methyl-1-butinyl, 1,1-Dimethyl-2-propinyl, 1-Ethyl-2-propinyl, 1-Hexinyl, 2-Hexinyl, 3-Hexinyl, 4-Hexinyl, 5-Hexinyl, 1-Methyl-2-pentinyl, 1-Methyl-3-pentinyl, 1-Methyl-4-pentinyl, 2-Methyl-3-pentinyl, 2-Methyl-4-pentinyl, 3-Methyl-1-pentinyl, 3-Methyl-4-pentinyl, 4-Methyl-1-pentinyl, 4-Methyl-2-pentinyl, 1,1-Dimethyl-2-butinyl, 1,1-Dimethyl-3-butinyl, 1,2-Dimethyl-3-butinyl, 2,2-Dimethyl-3-butinyl, 3,3-Dimethyl-1-butinyl, 1-Ethyl-2-butinyl, 1-Ethyl-3-butinyl, 2-Ethyl-3-butinyl und 1-Ethyl-1-methyl-2-propinyl;
**Aryl:** ein ein- bis dreikerniges aromatisches Ringsystem enthaltend 6 bis 14 Kohlenstoffringglieder, z.B. Phenyl, Naphthyl und Anthracenyl;

Besonders bevorzugt werden Verbindungen I, in denen Q für C (CO₂CH₃) =CHCH₃, C (CO₂CH₃) =CHOCH₃, C (CO₂CH₃) =NOCH₃, C (CONHCH₃) =NOCH₃ oder N(OCH₃)-CO₂CH₃ steht.

Außerdem werden Verbindungen I besonders bevorzugt, in denen R³ für Halogen, insbesondere Fluor, Chlor und Brom steht.

Gleichermaßen besonders bevorzugt sind Verbindungen I, in denen R³ für Methyl steht.

Daneben werden Verbindungen I besonders bevorzugt, in denen R³ für C₁-Halogenalkyl, insbesondere Trifluormethyl, steht.

Insbesondere werden Verbindungen I bevorzugt, in denen x für 0 oder 1 steht.

Insbesondere werden Verbindungen I bevorzugt, in denen R⁴ für Halogen, insbesondere Fluor, Chlor und Brom, steht.

Außerdem werden Verbindungen I besonders bevorzugt, in denen R⁴ für C₁-C₂-Alkyl steht.

Gleichermaßen besonders bevorzugt sind Verbindungen I, in denen R⁴ für C₁-C₂-Alkoxy steht.

Daneben werden Verbindungen I besonders bevorzugt, in denen R⁴ für Cyano steht.

Insbesondere werden auch Verbindungen I bevorzugt, in denen R⁴ für Nitro steht.

Insbesondere werden Verbindungen I bevorzugt, in denen y für 0 oder 1 steht.

Insbesondere werden Verbindungen I bevorzugt, in denen R⁵ für Methyl steht.

Außerdem werden Verbindungen I besonders bevorzugt, in denen R⁵ für Methoxy steht.

Gleichermaßen besonders bevorzugt sind Verbindungen I, in denen R⁵ für Fluor steht.

Daneben werden Verbindungen I besonders bevorzugt, in denen R⁵ für Chlor steht.

Insbesondere werden auch Verbindungen I bevorzugt, in denen R⁵ für Trifluormethyl steht.

Insbesondere sind im Hinblick auf ihre Verwendung die in den folgenden Tabellen zusammengestellten Verbindungen I bevorzugt. Die in den Tabellen für einen Substituenten genannten Gruppen stellen außerdem für sich betrachtet, unabhängig von der Kombination, in der sie genannt sind, eine besonders bevorzugte Ausgestaltung des betreffenden Substituenten dar.

### Tabelle 1

Verbindungen der allgemeinen Formel IA.1, in denen R² für Methyl, R³ für Wasserstoff, R⁴ für Wasserstoff und R⁵_{y} für Wasserstoff steht und R¹ für eine Verbindung jeweils einer Gruppe der Tabelle A entspricht.

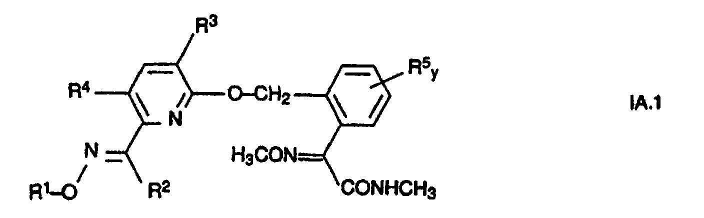

### Tabelle 2

Verbindungen der allgemeinen Formel IA.2, in denen R² für Methyl, R³ für Wasserstoff, R⁴ für Wasserstoff und R⁵_{y} für Wasserstoff steht und R¹ für eine Verbindung jeweils einer Gruppe der Tabelle A entspricht.

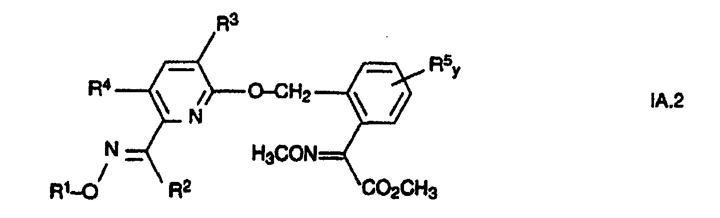

### Tabelle 3

Verbindungen der allgemeinen Formel IA.3, in denen R² für Methyl, R³ für Wasserstoff, R⁴ für Wasserstoff und R⁵_{y} für Wasserstoff steht und R¹ für eine Verbindung jeweils einer Gruppe der Tabelle A entspricht.

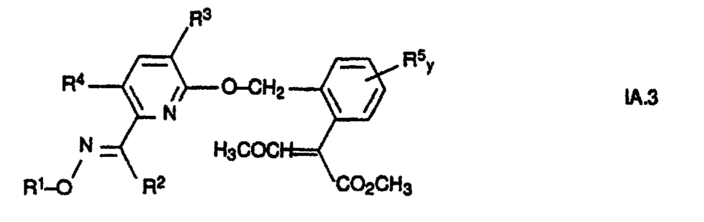

### Tabelle 4

Verbindungen der allgemeinen Formel IA.4, in denen R² für Methyl, R³ für Wasserstoff, R⁴ für Wasserstoff und R⁵_{y} für Wasserstoff steht und R¹ für eine Verbindung jeweils einer Gruppe der Tabelle A entspricht.

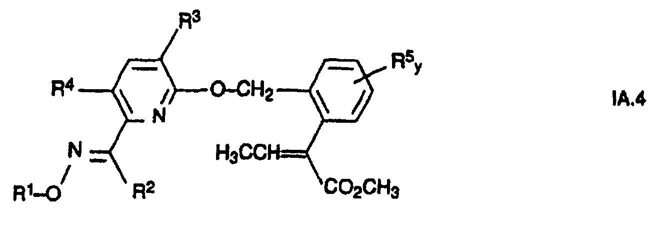

### Tabelle 5

Verbindungen der allgemeinen Formel IA.5, in denen R² für Methyl, R³ für Wasserstoff, R⁴ für Wasserstoff und R⁵_{y} für Wasserstoff steht und R¹ für eine Verbindung jeweils einer Gruppe der Tabelle A entspricht.

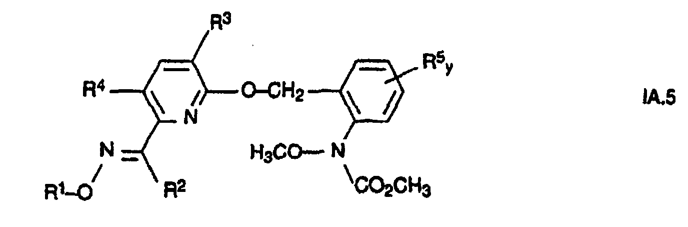

### Tabelle 6

Verbindungen der allgemeinen Formel IA.6, in denen R² für Methyl, R³ für Wasserstoff, R⁴ für Wasserstoff und R⁵_{y} für Wasserstoff steht und R¹ für eine Verbindung jeweils einer Gruppe der Tabelle A entspricht.

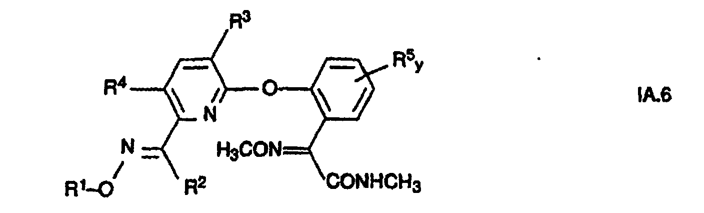

### Tabelle 7

Verbindungen der allgemeinen Formel IA.7, in denen R² für Methyl, R³ für Wasserstoff, R⁴ für Wasserstoff und R⁵_{y} für Wasserstoff steht und R¹ für eine Verbindung jeweils einer Gruppe der Tabelle A entspricht.

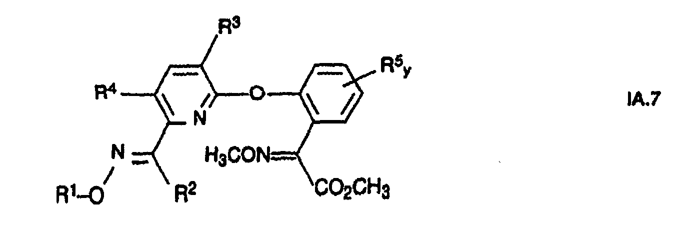

### Tabelle 8

Verbindungen der allgemeinen Formel IA.8, in denen R² für Methyl, R³ für Wasserstoff, R⁴ für Wasserstoff und R⁵_{y} für Wasserstoff steht und R¹ für eine Verbindung jeweils einer Gruppe der Tabelle A entspricht.

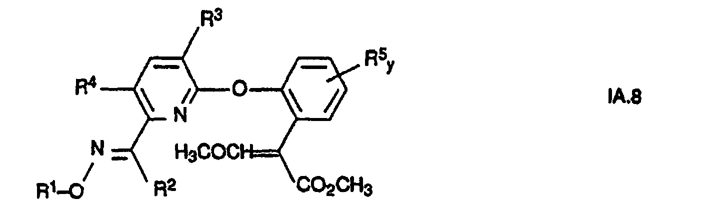

### Tabelle 9

Verbindungen der allgemeinen Formel IA.9, in denen R² für Methyl, R³ für Wasserstoff, R⁴ für Wasserstoff und R⁵_{y} für Wasserstoff steht und R¹ für eine Verbindung jeweils einer Gruppe der Tabelle A entspricht.

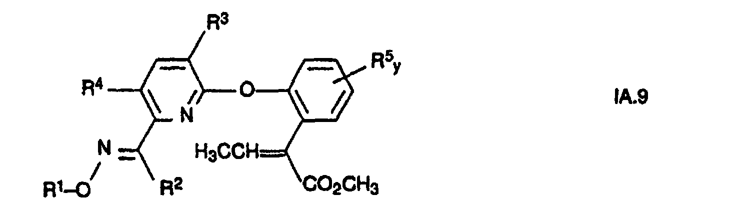

### Tabelle 10

Verbindungen der allgemeinen Formel IA.10, in denen R² für Methyl, R³ für Wasserstoff, R⁴ für Wasserstoff und R⁵_{y} für Wasserstoff steht und R¹ für eine Verbindung jeweils einer Gruppe der Tabelle A entspricht.

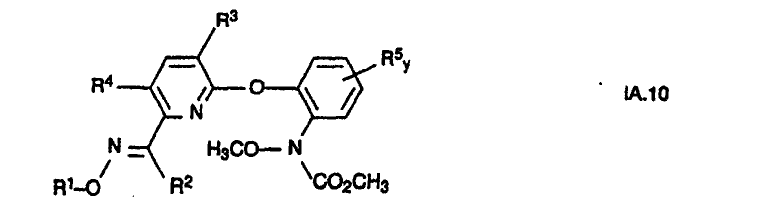

### Tabelle 11

Verbindungen der allgemeinen Formel IB.1, in denen R² für Methyl, R³ für Wasserstoff, R⁴ für Wasserstoff und R⁵_{y} für Wasserstoff steht und R¹ für eine Verbindung jeweils einer Gruppe der Tabelle A entspricht.

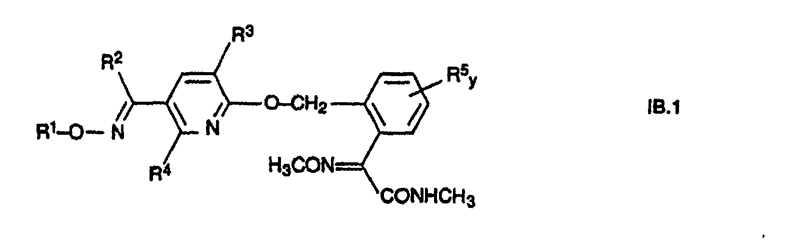

### Tabelle 12

Verbindungen der allgemeinen Formel IB.2, in denen R² für Methyl, R³ für Wasserstoff, R⁴ für Wasserstoff und R⁵_{y} für Wasserstoff steht und R¹ für eine Verbindung jeweils einer Gruppe der Tabelle A entspricht.

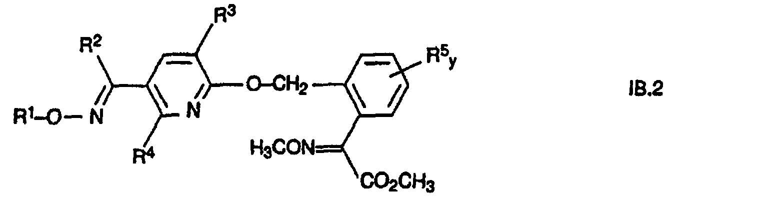

### Tabelle 13

Verbindungen der allgemeinen Formel IB.3, in denen R² für Methyl, R³ für Wasserstoff, R⁴ für Wasserstoff und R⁵_{y} für Wasserstoff steht und R¹ für eine Verbindung jeweils einer Gruppe der Tabelle A entspricht.

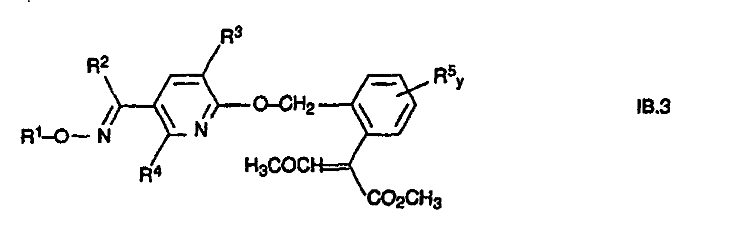

### Tabelle 14

Verbindungen der allgemeinen Formel IB.4, in denen R² für Methyl, R³ für Wasserstoff, R⁴ für Wasserstoff und R⁵_{y} für Wasserstoff steht und R¹ für eine Verbindung jeweils einer Gruppe der Tabelle A entspricht.

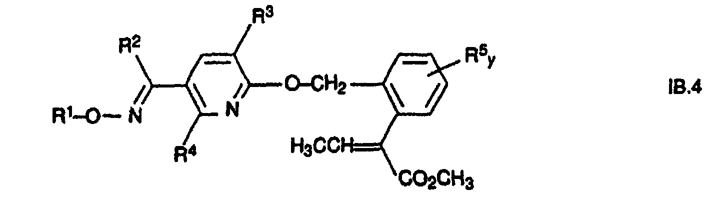

### Tabelle 15

Verbindungen der allgemeinen Formel IB.5, in denen R² für Methyl, R³ für Wasserstoff, R⁴ für Wasserstoff und R⁵_{y} für Wasserstoff steht und R¹ für eine Verbindung jeweils einer Gruppe der Tabelle A entspricht.

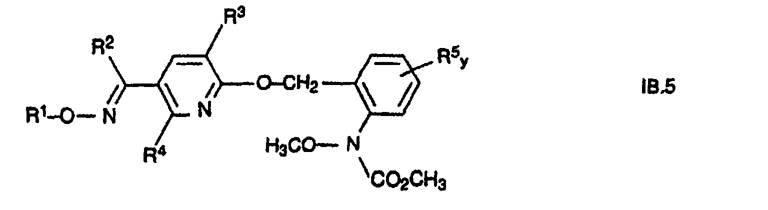

### Tabelle 16

Verbindungen der allgemeinen Formel IB.6, in denen R² für Methyl, R³ für Wasserstoff, R⁴ für Wasserstoff und R⁵_{y} für Wasserstoff steht und R¹ für eine Verbindung jeweils einer Gruppe der Tabelle A entspricht.

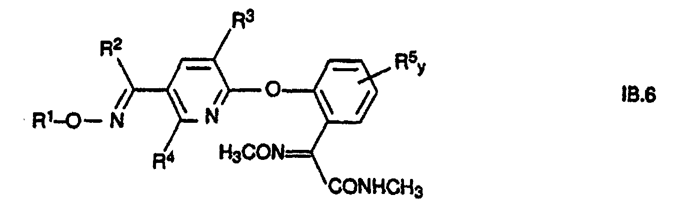

### Tabelle 17

Verbindungen der allgemeinen Formel IB.7, in denen R² für Methyl, R³ für Wasserstoff, R⁴ für Wasserstoff und R⁵_{y} für Wasserstoff steht und R¹ für eine Verbindung jeweils einer Gruppe der Tabelle A entspricht.

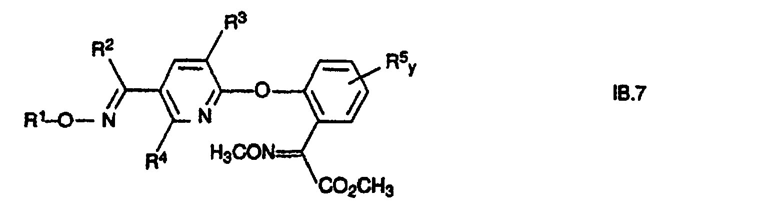

### Tabelle 18

Verbindungen der allgemeinen Formel IB.8, in denen R² für Methyl, R³ für Wasserstoff, R⁴ für Wasserstoff und R⁵_{y} für Wasserstoff steht und R¹ für eine Verbindung jeweils einer Gruppe der Tabelle A entspricht.

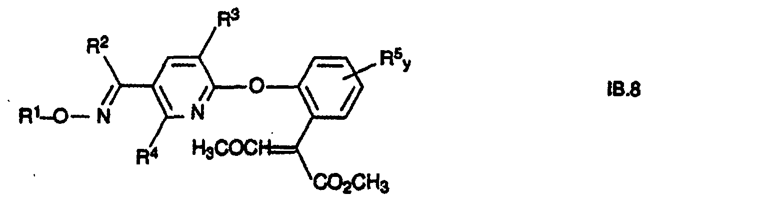

### Tabelle 19

Verbindungen der allgemeinen Formel IB.9, in denen R² für Methyl, R³ für Wasserstoff, R⁴ für Wasserstoff und R⁵_{y} für Wasserstoff steht und R¹ für eine Verbindung jeweils einer Gruppe der Tabelle A entspricht.

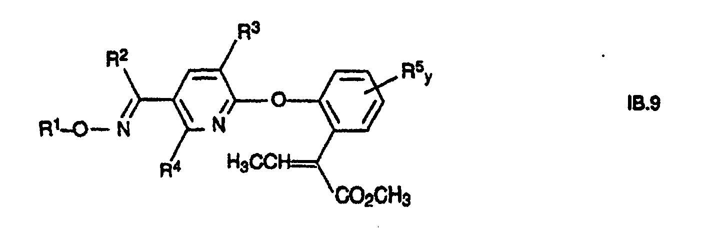

### Tabelle 20

Verbindungen der allgemeinen Formel IB.10, in denen R² für Methyl, R³ für Wasserstoff, R⁴ für Wasserstoff und R⁵_{y} für Wasserstoff steht und R¹ für eine Verbindung jeweils einer Gruppe der Tabelle A entspricht.

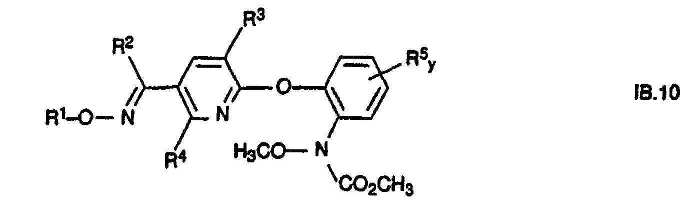

### Tabelle 21

Verbindungen der allgemeinen Formel IA.1, in denen R² für Methyl, R³ für Methyl, R⁴ für Wasserstoff und R⁵_{y} für Wasserstoff steht und R¹ für eine Verbindung jeweils einer Gruppe der Tabelle A entspricht.

### Tabelle 22

Verbindungen der allgemeinen Formel IA.2, in denen R² für Methyl, R³ für Methyl, R⁴ für Wasserstoff und R⁵_{y} für Wasserstoff steht und R¹ für eine Verbindung jeweils einer Gruppe der Tabelle A entspricht.

### Tabelle 23

Verbindungen der allgemeinen Formel IA.3, in denen R² für Methyl, R³ für Methyl, R⁴ für Wasserstoff und R⁵_{y} für Wasserstoff steht und R¹ für eine Verbindung jeweils einer Gruppe der Tabelle A entspricht.

### Tabelle 24

Verbindungen der allgemeinen Formel IA.4, in denen R² für Methyl, R³ für Methyl, R⁴ für Wasserstoff und R⁵_{y} für Wasserstoff steht und R¹ für eine Verbindung jeweils einer Gruppe der Tabelle A entspricht.

### Tabelle 25

Verbindungen der allgemeinen Formel IA.5, in denen R² für Methyl, R³ für Methyl, R⁴ für Wasserstoff und R⁵_{y} für Wasserstoff steht und R¹ für eine Verbindung jeweils einer Gruppe der Tabelle A entspricht.

### Tabelle 26

Verbindungen der allgemeinen Formel IA.6, in denen R² für Methyl, R³ für Methyl, R⁴ für Wasserstoff und R⁵_{y} für Wasserstoff steht und R¹ für eine Verbindung jeweils einer Gruppe der Tabelle A entspricht.

### Tabelle 27

Verbindungen der allgemeinen Formel IA.7, in denen R² für Methyl, R³ für Methyl, R⁴ für Wasserstoff und R⁵_{y} für Wasserstoff steht und R¹ für eine Verbindung jeweils einer Gruppe der Tabelle A entspricht.

### Tabelle 28

Verbindungen der allgemeinen Formel IA.8, in denen R² für Methyl, R³ für Methyl, R⁴ für Wasserstoff und R⁵y für Wasserstoff steht und R¹ für eine Verbindung jeweils einer Gruppe der Tabelle A entspricht.

### Tabelle 29

Verbindungen der allgemeinen Formel IA.9, in denen R² für Methyl, R³ für Methyl, R⁴ für Wasserstoff und R⁵_{y} für Wasserstoff steht und R¹ für eine Verbindung jeweils einer Gruppe der Tabelle A entspricht.

### Tabelle 30

Verbindungen der allgemeinen Formel IA.10, in denen R² für Methyl, R³ für Methyl, R⁴ für Wasserstoff und R⁵_{y} für Wasserstoff steht und R¹ für eine Verbindung jeweils einer Gruppe der Tabelle A entspricht.

### Tabelle 31

Verbindungen der allgemeinen Formel IB.1, in denen R² für Methyl, R³ für Methyl, R⁴ für Wasserstoff und R⁵_{y} für Wasserstoff steht und R¹ für eine Verbindung jeweils einer Gruppe der Tabelle A entspricht.

### Tabelle 32

Verbindungen der allgemeinen Formel IB.2, in denen R² für Methyl, R³ für Methyl, R⁴ für Wasserstoff und R⁵_{y} für Wasserstoff steht und R¹ für eine Verbindung jeweils einer Gruppe der Tabelle A entspricht.

### Tabelle 33

Verbindungen der allgemeinen Formel IB.3, in denen R² für Methyl, R³ für Methyl, R⁴ für Wasserstoff und R⁵_{y} für Wasserstoff steht und R¹ für eine Verbindung jeweils einer Gruppe der Tabelle A entspricht.

### Tabelle 34

Verbindungen der allgemeinen Formel IB.4, in denen R² für Methyl, R³ für Methyl, R⁴ für Wasserstoff und R⁵_{y} für Wasserstoff steht und R¹ für eine Verbindung jeweils einer Gruppe der Tabelle A entspricht.

### Tabelle 35

Verbindungen der allgemeinen Formel IB.5, in denen R² für Methyl, R³ für Methyl, R⁴ für Wasserstoff und R⁵_{y} für Wasserstoff steht und R¹ für eine Verbindung jeweils einer Gruppe der Tabelle A entspricht.

### Tabelle 36

Verbindungen der allgemeinen Formel IB.6, in denen R² für Methyl, R³ für Methyl, R⁴ für Wasserstoff und R⁵_{y} für Wasserstoff steht und R¹ für eine Verbindung jeweils einer Gruppe der Tabelle A entspricht.

### Tabelle 37

Verbindungen der allgemeinen Formel IB.7, in denen R² für Methyl, R³ für Methyl, R⁴ für Wasserstoff und R⁵_{y} für Wasserstoff steht und R¹ für eine Verbindung jeweils einer Gruppe der Tabelle A entspricht.

### Tabelle 38

Verbindungen der allgemeinen Formel IB.8, in denen R² für Methyl, R³ für Methyl, R⁴ für Wasserstoff und R⁵_{y} für Wasserstoff steht und R¹ für eine Verbindung jeweils einer Gruppe der Tabelle A entspricht.

### Tabelle 39

Verbindungen der allgemeinen Formel IB.9, in denen R² für Methyl, R³ für Methyl, R⁴ für Wasserstoff und R⁵_{y} für Wasserstoff steht und R¹ für eine Verbindung jeweils einer Gruppe der Tabelle A entspricht.

### Tabelle 40

Verbindungen der allgemeinen Formel IB.10, in denen R² für Methyl, R³ für Methyl, R⁴ für Wasserstoff und R⁵_{y} für Wasserstoff steht und R¹ für eine Verbindung jeweils einer Gruppe der Tabelle A entspricht.

### Tabelle 41

Verbindungen der allgemeinen Formel IA.1, in denen R² für Methyl, R³ für Chlor, R⁴ für Wasserstoff und R⁵_{y} für Wasserstoff steht und R¹ für eine Verbindung jeweils einer Gruppe der Tabelle A entspricht.

### Tabelle 42

Verbindungen der allgemeinen Formel IA.2, in denen R² für Methyl, R³ für Chlor, R⁴ für Wasserstoff und R⁵_{y} für Wasserstoff steht und R¹ für eine Verbindung jeweils einer Gruppe der Tabelle A entspricht.

### Tabelle 43

Verbindungen der allgemeinen Formel IA.3, in denen R² für Methyl, R³ für Chlor, R⁴ für Wasserstoff und R⁵_{y} für Wasserstoff steht und R¹ für eine Verbindung jeweils einer Gruppe der Tabelle A entspricht.

### Tabelle 44

Verbindungen der allgemeinen Formel IA.4, in denen R² für Methyl, R³ für Chlor, R⁴ für Wasserstoff und R⁵_{y} für Wasserstoff steht und R¹ für eine Verbindung jeweils einer Gruppe der Tabelle A entspricht.

### Tabelle 45

Verbindungen der allgemeinen Formel IA.5, in denen R² für Methyl, R³ für Chlor, R⁴ für Wasserstoff und R⁵_{y} für Wasserstoff steht und R¹ für eine Verbindung jeweils einer Gruppe der Tabelle A entspricht.

### Tabelle 46

Verbindungen der allgemeinen Formel IA.6, in denen R² für Methyl, R³ für Chlor, R⁴ für Wasserstoff und R⁵_{y} für Wasserstoff steht und R¹ für eine Verbindung jeweils einer Gruppe der Tabelle A entspricht.

### Tabelle 47

Verbindungen der allgemeinen Formel IA.7, in denen R² für Methyl, R³ für Chlor, R⁴ für Wasserstoff und R⁵_{y} für Wasserstoff steht und R¹ für eine Verbindung jeweils einer Gruppe der Tabelle A entspricht.

### Tabelle 48

Verbindungen der allgemeinen Formel IA.8, in denen R² für Methyl, R³ für Chlor, R⁴ für Wasserstoff und R⁵_{y} für Wasserstoff steht und R¹ für eine Verbindung jeweils einer Gruppe der Tabelle A entspricht.

### Tabelle 49

Verbindungen der allgemeinen Formel IA.9, in denen R² für Methyl, R³ für Chlor, R⁴ für Wasserstoff und R⁵_{y} für Wasserstoff steht und R¹ für eine Verbindung jeweils einer Gruppe der Tabelle A entspricht.

### Tabelle 50

Verbindungen der allgemeinen Formel IA.10, in denen R² für Methyl, R³ für Chlor, R⁴ für Wasserstoff und R⁵_{y} für Wasserstoff steht und R¹ für eine Verbindung jeweils einer Gruppe der Tabelle A entspricht.

### Tabelle 51

Verbindungen der allgemeinen Formel IB.1, in denen R² für Methyl, R³ für Chlor, R⁴ für Wasserstoff und R⁵_{y} für Wasserstoff steht und R¹ für eine Verbindung jeweils einer Gruppe der Tabelle A entspricht.

### Tabelle 52

Verbindungen der allgemeinen Formel IB.2, in denen R² für Methyl, R³ für Chlor, R⁴ für Wasserstoff und R⁵_{y} für Wasserstoff steht und R¹ für eine Verbindung jeweils einer Gruppe der Tabelle A entspricht.

### Tabelle 53

Verbindungen der allgemeinen Formel IB.3, in denen R² für Methyl, R³ für Chlor, R⁴ für Wasserstoff und R⁵_{y} für Wasserstoff steht und R¹ für eine Verbindung jeweils einer Gruppe der Tabelle A entspricht.

### Tabelle 54

Verbindungen der allgemeinen Formel IB.4, in denen R² für Methyl, R³ für Chlor, R⁴ für Wasserstoff und R⁵_{y} für Wasserstoff steht und R¹ für eine Verbindung jeweils einer Gruppe der Tabelle A entspricht.

### Tabelle 55

Verbindungen der allgemeinen Formel IB.5, in denen R² für Methyl, R³ für Chlor, R⁴ für Wasserstoff und R⁵_{y} für Wasserstoff steht und R¹ für eine Verbindung jeweils einer Gruppe der Tabelle A entspricht.

### Tabelle 56

Verbindungen der allgemeinen Formel IB.6, in denen R² für Methyl, R³ für Chlor, R⁴ für Wasserstoff und R⁵_{y} für Wasserstoff steht und R¹ für eine Verbindung jeweils einer Gruppe der Tabelle A entspricht.

### Tabelle 57

Verbindungen der allgemeinen Formel IB.7, in denen R² für Methyl, R³ für Chlor, R⁴ für Wasserstoff und R⁵_{y} für Wasserstoff steht und R¹ für eine Verbindung jeweils einer Gruppe der Tabelle A entspricht.

### Tabelle 58

Verbindungen der allgemeinen Formel IB.8, in denen R² für Methyl, R³ für Chlor, R⁴ für Wasserstoff und R⁵_{y} für Wasserstoff steht und R¹ für eine Verbindung jeweils einer Gruppe der Tabelle A entspricht.

### Tabelle 59

Verbindungen der allgemeinen Formel IB.9, in denen R² für Methyl, R³ für Chlor, R⁴ für Wasserstoff und R⁵_{y} für Wasserstoff steht und R¹ für eine Verbindung jeweils einer Gruppe der Tabelle A entspricht.

### Tabelle 60

Verbindungen der allgemeinen Formel IB.10, in denen R² für Methyl, R³ für Chlor, R⁴ für Wasserstoff und R⁵_{y} für Wasserstoff steht und R¹ für eine Verbindung jeweils einer Gruppe der Tabelle A entspricht.

### Tabelle 61

Verbindungen der allgemeinen Formel IA.1, in denen R² für Methyl, R³ für Fluor, R⁴ für Wasserstoff und R⁵_{y} für Wasserstoff steht und R¹ für eine Verbindung jeweils einer Gruppe der Tabelle A entspricht.

### Tabelle 62

Verbindungen der allgemeinen Formel IA.2, in denen R² für Methyl, R³ für Fluor, R⁴ für Wasserstoff und R⁵_{y} für Wasserstoff steht und R¹ für eine Verbindung jeweils einer Gruppe der Tabelle A entspricht.

### Tabelle 63

Verbindungen der allgemeinen Formel IA.3, in denen R² für Methyl, R³ für Fluor, R⁴ für Wasserstoff und R⁵_{y} für Wasserstoff steht und R¹ für eine Verbindung jeweils einer Gruppe der Tabelle A entspricht.

### Tabelle 64

Verbindungen der allgemeinen Formel IA.4, in denen R² für Methyl, R³ für Fluor, R⁴ für Wasserstoff und R⁵_{y} für Wasserstoff steht und R¹ für eine Verbindung jeweils einer Gruppe der Tabelle A entspricht.

### Tabelle 65

Verbindungen der allgemeinen Formel IA.5, in denen R² für Methyl, R³ für Fluor, R⁴ für Wasserstoff und R⁵_{y} für Wasserstoff steht und R¹ für eine Verbindung jeweils einer Gruppe der Tabelle A entspricht.

### Tabelle 66

Verbindungen der allgemeinen Formel IA.6, in denen R² für Methyl, R³ für Fluor, R⁴ für Wasserstoff und R⁵_{y} für Wasserstoff steht und R¹ für eine Verbindung jeweils einer Gruppe der Tabelle A entspricht.

### Tabelle 67

Verbindungen der allgemeinen Formel IA.7, in denen R² für Methyl, R³ für Fluor, R⁴ für Wasserstoff und R⁵_{y} für Wasserstoff steht und R¹ für eine Verbindung jeweils einer Gruppe der Tabelle A entspricht.

### Tabelle 68

Verbindungen der allgemeinen Formel IA.8, in denen R² für Methyl, R³ für Fluor, R⁴ für Wasserstoff und R⁵_{y} für Wasserstoff steht und R¹ für eine Verbindung jeweils einer Gruppe der Tabelle A entspricht.

### Tabelle 69

Verbindungen der allgemeinen Formel IA.9, in denen R² für Methyl, R³ für Fluor, R⁴ für Wasserstoff und R⁵_{y} für Wasserstoff steht und R¹ für eine Verbindung jeweils einer Gruppe der Tabelle A entspricht.

### Tabelle 70

Verbindungen der allgemeinen Formel IA.10, in denen R² für Methyl, R³ für Fluor, R⁴ für Wasserstoff und R⁵_{y} für Wasserstoff steht und R¹ für eine Verbindung jeweils einer Gruppe der Tabelle A entspricht.

### Tabelle 71

Verbindungen der allgemeinen Formel IB.1, in denen R² für Methyl, R³ für Fluor, R⁴ für Wasserstoff und R⁵_{y} für Wasserstoff steht und R¹ für eine Verbindung jeweils einer Gruppe der Tabelle A entspricht.

### Tabelle 72

Verbindungen der allgemeinen Formel IB.2, in denen R² für Methyl, R³ für Fluor, R⁴ für Wasserstoff und R⁵_{y} für Wasserstoff steht und R¹ für eine Verbindung jeweils einer Gruppe der Tabelle A entspricht.

### Tabelle 73

Verbindungen der allgemeinen Formel IB.3, in denen R² für Methyl, R³ für Fluor, R⁴ für Wasserstoff und R⁵_{y} für Wasserstoff steht und R¹ für eine Verbindung jeweils einer Gruppe der Tabelle A entspricht.

### Tabelle 74

Verbindungen der allgemeinen Formel IB.4, in denen R² für Methyl, R³ für Fluor, R⁴ für Wasserstoff und R⁵_{y} für Wasserstoff steht und R¹ für eine Verbindung jeweils einer Gruppe der Tabelle A entspricht.

### Tabelle 75

Verbindungen der allgemeinen Formel IB.5, in denen R² für Methyl, R³ für Fluor, R⁴ für Wasserstoff und R⁵_{y} für Wasserstoff steht und R¹ für eine Verbindung jeweils einer Gruppe der Tabelle A entspricht.

### Tabelle 76

Verbindungen der allgemeinen Formel IB.6, in denen R² für Methyl, R³ für Fluor, R⁴ für Wasserstoff und R⁵_{y} für Wasserstoff steht und R¹ für eine Verbindung jeweils einer Gruppe der Tabelle A entspricht.

### Tabelle 77

Verbindungen der allgemeinen Formel IB.7, in denen R² für Methyl, R³ für Fluor, R⁴ für Wasserstoff und R⁵_{y} für Wasserstoff steht und R¹ für eine Verbindung jeweils einer Gruppe der Tabelle A entspricht.

### Tabelle 78

Verbindungen der allgemeinen Formel IB.8, in denen R² für Methyl, R³ für Fluor, R⁴ für Wasserstoff und R⁵_{y} für Wasserstoff steht und R¹ für eine Verbindung jeweils einer Gruppe der Tabelle A entspricht.

### Tabelle 79

Verbindungen der allgemeinen Formel IB.9, in denen R² für Methyl, R³ für Fluor, R⁴ für Wasserstoff und R⁵_{y} für Wasserstoff steht und R¹ für eine Verbindung jeweils einer Gruppe der Tabelle A entspricht.

### Tabelle 80

Verbindungen der allgemeinen Formel IB.10, in denen R² für Methyl, R³ für Fluor, R⁴ für Wasserstoff und R⁵_{y} für Wasserstoff steht und R¹ für eine Verbindung jeweils einer Gruppe der Tabelle A entspricht.

### Tabelle 81

Verbindungen der allgemeinen Formel IA.1, in denen R² für Methyl, R³ für Brom, R⁴ für Wasserstoff und R⁵_{y} für Wasserstoff steht und R¹ für eine Verbindung jeweils einer Gruppe der Tabelle A entspricht.

### Tabelle 82

Verbindungen der allgemeinen Formel IA.2, in denen R² für Methyl, R³ für Brom, R⁴ für Wasserstoff und R⁵_{y} für Wasserstoff steht und R¹ für eine Verbindung jeweils einer Gruppe der Tabelle A entspricht.

### Tabelle 83

Verbindungen der allgemeinen Formel IA.3, in denen R² für Methyl, R³ für Brom, R⁴ für Wasserstoff und R⁵_{y} für Wasserstoff steht und R¹ für eine Verbindung jeweils einer Gruppe der Tabelle A entspricht.

### Tabelle 84

Verbindungen der allgemeinen Formel IA.4, in denen R² für Methyl, R³ für Brom, R⁴ für Wasserstoff und R⁵_{y} für Wasserstoff steht und R¹ für eine Verbindung jeweils einer Gruppe der Tabelle A entspricht.

### Tabelle 85

Verbindungen der allgemeinen Formel IA.5, in denen R² für Methyl, R³ für Brom, R⁴ für Wasserstoff und R⁵_{y} für Wasserstoff steht und R¹ für eine Verbindung jeweils einer Gruppe der Tabelle A entspricht.

### Tabelle 86

Verbindungen der allgemeinen Formel IA.6, in denen R² für Methyl, R³ für Brom, R⁴ für Wasserstoff und R⁵_{y} für Wasserstoff steht und R¹ für eine Verbindung jeweils einer Gruppe der Tabelle A entspricht.

### Tabelle 87

Verbindungen der allgemeinen Formel IA.7, in denen R² für Methyl, R³ für Brom, R⁴ für Wasserstoff und R⁵_{y} für Wasserstoff steht und R¹ für eine Verbindung jeweils einer Gruppe der Tabelle A entspricht.

### Tabelle 88

Verbindungen der allgemeinen Formel IA.8, in denen R² für Methyl, R³ für Brom, R⁴ für Wasserstoff und R⁵_{y} für Wasserstoff steht und R¹ für eine Verbindung jeweils einer Gruppe der Tabelle A entspricht.

### Tabelle 89

Verbindungen der allgemeinen Formel IA.9, in denen R² für Methyl, R³ für Brom, R⁴ für Wasserstoff und R⁵_{y} für Wasserstoff steht und R¹ für eine Verbindung jeweils einer Gruppe der Tabelle A entspricht.

### Tabelle 90

Verbindungen der allgemeinen Formel IA.10, in denen R² für Methyl, R³ für Brom, R⁴ für Wasserstoff und R⁵_{y} für Wasserstoff steht und R¹ für eine Verbindung jeweils einer Gruppe der Tabelle A entspricht.

### Tabelle 91

Verbindungen der allgemeinen Formel IB.1, in denen R² für Methyl, R³ für Brom, R⁴ für Wasserstoff und R⁵_{y} für Wasserstoff steht und R¹ für eine Verbindung jeweils einer Gruppe der Tabelle A entspricht.

### Tabelle 92

Verbindungen der allgemeinen Formel IB.2, in denen R² für Methyl, R³ für Brom, R⁴ für Wasserstoff und R⁵_{y} für Wasserstoff steht und R¹ für eine Verbindung jeweils einer Gruppe der Tabelle A entspricht.

### Tabelle 93

Verbindungen der allgemeinen Formel IB.3, in denen R² für Methyl, R³ für Brom, R⁴ für Wasserstoff und R⁵_{y} für Wasserstoff steht und R¹ für eine Verbindung jeweils einer Gruppe der Tabelle A entspricht.

### Tabelle 94

Verbindungen der allgemeinen Formel IB.4, in denen R² für Methyl, R³ für Brom, R⁴ für Wasserstoff und R⁵_{y} für Wasserstoff steht und R¹ für eine Verbindung jeweils einer Gruppe der Tabelle A entspricht.

### Tabelle 95

Verbindungen der allgemeinen Formel IB.5, in denen R² für Methyl, R³ für Brom, R⁴ für Wasserstoff und R⁵_{y} für Wasserstoff steht und R¹ für eine Verbindung jeweils einer Gruppe der Tabelle A entspricht.

### Tabelle 96

Verbindungen der allgemeinen Formel IB.6, in denen R² für Methyl, R³ für Brom, R⁴ für Wasserstoff und R⁵_{y} für Wasserstoff steht und R¹ für eine Verbindung jeweils einer Gruppe der Tabelle A entspricht.

### Tabelle 97

Verbindungen der allgemeinen Formel IB.7, in denen R² für Methyl, R³ für Brom, R⁴ für Wasserstoff und R⁵_{y} für Wasserstoff steht und R¹ für eine Verbindung jeweils einer Gruppe der Tabelle A entspricht.

### Tabelle 98

Verbindungen der allgemeinen Formel IB.8, in denen R² für Methyl, R³ für Brom, R⁴ für Wasserstoff und R⁵_{y} für Wasserstoff steht und R¹ für eine Verbindung jeweils einer Gruppe der Tabelle A entspricht.

### Tabelle 99

Verbindungen der allgemeinen Formel IB.9, in denen R² für Methyl, R³ für Brom, R⁴ für Wasserstoff und R⁵_{y} für Wasserstoff steht und R¹ für eine Verbindung jeweils einer Gruppe der Tabelle A entspricht.

### Tabelle 100

Verbindungen der allgemeinen Formel IB.10, in denen R² für Methyl, R³ für Brom, R⁴ für Wasserstoff und R⁵_{y} für Wasserstoff steht und R¹ für eine Verbindung jeweils einer Gruppe der Tabelle A entspricht.

### Tabelle 101

Verbindungen der allgemeinen Formel IA.1, in denen R² für Methyl, R³ für Trifluormethyl, R⁴ für Wasserstoff und R⁵_{y} für Wasserstoff steht und R¹ für eine Verbindung jeweils einer Gruppe der Tabelle A entspricht.

### Tabelle 102

Verbindungen der allgemeinen Formel IA.2, in denen R² für Methyl, R³ für Trifluormethyl, R⁴ für Wasserstoff und R⁵_{y} für Wasserstoff steht und R¹ für eine Verbindung jeweils einer Gruppe der Tabelle A entspricht.

### Tabelle 103

Verbindungen der allgemeinen Formel IA.3, in denen R² für Methyl, R³ für Trifluormethyl, R⁴ für Wasserstoff und R⁵_{y} für Wasserstoff steht und R¹ für eine Verbindung jeweils einer Gruppe der Tabelle A entspricht.

### Tabelle 104

Verbindungen der allgemeinen Formel IA.4, in denen R² für Methyl, R³ für Trifluormethyl, R⁴ für Wasserstoff und R⁵_{y} für Wasserstoff steht und R¹ für eine Verbindung jeweils einer Gruppe der Tabelle A entspricht.

### Tabelle 105

Verbindungen der allgemeinen Formel IA.5, in denen R² für Methyl, R³ für Trifluormethyl, R⁴ für Wasserstoff und R⁵_{y} für Wasserstoff steht und R¹ für eine Verbindung jeweils einer Gruppe der Tabelle A entspricht.

### Tabelle 106

Verbindungen der allgemeinen Formel IA.6, in denen R² für Methyl, R³ für Trifluormethyl, R⁴ für Wasserstoff und R⁵_{y} für Wasserstoff steht und R¹ für eine Verbindung jeweils einer Gruppe der Tabelle A entspricht.

### Tabelle 107

Verbindungen der allgemeinen Formel IA.7, in denen R² für Methyl, R³ für Trifluormethyl, R⁴ für Wasserstoff und R⁵_{y} für Wasserstoff steht und R¹ für eine Verbindung jeweils einer Gruppe der Tabelle A entspricht.

### Tabelle 108

Verbindungen der allgemeinen Formel IA.8, in denen R² für Methyl, R³ für Trifluormethyl, R⁴ für Wasserstoff und R⁵_{y} für Wasserstoff steht und R¹ für eine Verbindung jeweils einer Gruppe der Tabelle A entspricht.

### Tabelle 109

Verbindungen der allgemeinen Formel IA.9, in denen R² für Methyl, R³ für Trifluormethyl, R⁴ für Wasserstoff und R⁵_{y} für Wasserstoff steht und R¹ für eine Verbindung jeweils einer Gruppe der Tabelle A entspricht.

### Tabelle 110

Verbindungen der allgemeinen Formel IA.10, in denen R² für Methyl, R³ für Trifluormethyl, R⁴ für Wasserstoff und R⁵_{y} für Wasserstoff steht und R¹ für eine Verbindung jeweils einer Gruppe der Tabelle A entspricht.

### Tabelle 111

Verbindungen der allgemeinen Formel IB.1, in denen R² für Methyl, R³ für Trifluormethyl, R⁴ für Wasserstoff und R⁵_{y} für Wasserstoff steht und R¹ für eine Verbindung jeweils einer Gruppe der Tabelle A entspricht.

### Tabelle 112

Verbindungen der allgemeinen Formel IB.2, in denen R² für Methyl, R³ für Trifluormethyl, R⁴ für Wasserstoff und R⁵_{y} für Wasserstoff steht und R¹ für eine Verbindung jeweils einer Gruppe der Tabelle A entspricht.

### Tabelle 113

Verbindungen der allgemeinen Formel IB.3, in denen R² für Methyl, R³ für Trifluormethyl, R⁴ für Wasserstoff und R⁵_{y} für Wasserstoff steht und R¹ für eine Verbindung jeweils einer Gruppe der Tabelle A entspricht.

### Tabelle 114

Verbindungen der allgemeinen Formel IB.4, in denen R² für Methyl, R³ für Trifluormethyl, R⁴ für Wasserstoff und R⁵_{y} für Wasserstoff steht und R¹ für eine Verbindung jeweils einer Gruppe der Tabelle A entspricht.

### Tabelle 115

Verbindungen der allgemeinen Formel IB.5, in denen R² für Methyl, R³ für Trifluormethyl, R⁴ für Wasserstoff und R⁵_{y} für Wasserstoff steht und R¹ für eine Verbindung jeweils einer Gruppe der Tabelle A entspricht.

### Tabelle 116

Verbindungen der allgemeinen Formel IB.6, in denen R² für Methyl, R³ für Trifluormethyl, R⁴ für Wasserstoff und R⁵_{y} für Wasserstoff steht und R¹ für eine Verbindung jeweils einer Gruppe der Tabelle A entspricht.

### Tabelle 117

Verbindungen der allgemeinen Formel IB.7, in denen R² für Methyl, R³ für Trifluormethyl, R⁴ für Wasserstoff und R⁵_{y} für Wasserstoff steht und R¹ für eine Verbindung jeweils einer Gruppe der Tabelle A entspricht.

### Tabelle 118

Verbindungen der allgemeinen Formel IB.8, in denen R² für Methyl, R³ für Trifluormethyl, R⁴ für Wasserstoff und R⁵_{y} für Wasserstoff steht und R¹ für eine Verbindung jeweils einer Gruppe der Tabelle A entspricht.

### Tabelle 119

Verbindungen der allgemeinen Formel IB.9, in denen R² für Methyl, R³ für Trifluormethyl, R⁴ für Wasserstoff und R⁵_{y} für Wasserstoff steht und R¹ für eine Verbindung jeweils einer Gruppe der Tabelle A entspricht.

### Tabelle 120

Verbindungen der allgemeinen Formel IB.10, in denen R² für Methyl, R³ für Trifluormethyl, R⁴ für Wasserstoff und R⁵_{y} für Wasserstoff steht und R¹ für eine Verbindung jeweils einer Gruppe der Tabelle A entspricht.

### Tabelle 121

Verbindungen der allgemeinen Formel IA.1, in denen R² für Methyl, R³ für Methyl, R⁴ für Methyl und R⁵_{y} für Wasserstoff steht und R¹ für eine Verbindung jeweils einer Gruppe der Tabelle A entspricht.

### Tabelle 122

Verbindungen der allgemeinen Formel IA.2, in denen R² für Methyl, R³ für Methyl, R⁴ für Methyl und R⁵_{y} für Wasserstoff steht und R¹ für eine Verbindung jeweils einer Gruppe der Tabelle A entspricht.

### Tabelle 123

Verbindungen der allgemeinen Formel IA.3, in denen R² für Methyl, R³ für Methyl, R⁴ für Methyl und R⁵_{y} für Wasserstoff steht und R¹ für eine Verbindung jeweils einer Gruppe der Tabelle A entspricht.

### Tabelle 124

Verbindungen der allgemeinen Formel IA.4, in denen R² für Methyl, R³ für Methyl, R⁴ für Methyl und R⁵_{y} für Wasserstoff steht und R¹ für eine Verbindung jeweils einer Gruppe der Tabelle A entspricht.

### Tabelle 125

Verbindungen der allgemeinen Formel IA.5, in denen R² für Methyl, R³ für Methyl, R⁴ für Methyl und R⁵_{y} für Wasserstoff steht und R¹ für eine Verbindung jeweils einer Gruppe der Tabelle A entspricht.

### Tabelle 126

Verbindungen der allgemeinen Formel IA.6, in denen R² für Methyl, R³ für Methyl, R⁴ für Methyl und R⁵_{y} für Wasserstoff steht und R¹ für eine Verbindung jeweils einer Gruppe der Tabelle A entspricht.

### Tabelle 127

Verbindungen der allgemeinen Formel IA.7, in denen R² für Methyl, R³ für Methyl, R⁴ für Methyl und R⁵_{y} für Wasserstoff steht und R¹ für eine Verbindung jeweils einer Gruppe der Tabelle A entspricht.

### Tabelle 128

Verbindungen der allgemeinen Formel IA.8, in denen R² für Methyl, R³ für Methyl, R⁴ für Methyl und R⁵_{y} für Wasserstoff steht und R¹ für eine Verbindung jeweils einer Gruppe der Tabelle A entspricht.

### Tabelle 129

Verbindungen der allgemeinen Formel IA.9, in denen R² für Methyl, R³ für Methyl, R⁴ für Methyl und R⁵_{y} für Wasserstoff steht und R¹ für eine Verbindung jeweils einer Gruppe der Tabelle A entspricht.

### Tabelle 130

Verbindungen der allgemeinen Formel IA.10, in denen R² für Methyl, R³ für Methyl, R⁴ für Methyl und R⁵_{y} für Wasserstoff steht und R¹ für eine Verbindung jeweils einer Gruppe der Tabelle A entspricht.

### Tabelle 131

Verbindungen der allgemeinen Formel IB.1, in denen R² für Methyl, R³ für Methyl, R⁴ für Methyl und R⁵_{y} für Wasserstoff steht und R¹ für eine Verbindung jeweils einer Gruppe der Tabelle A entspricht.

### Tabelle 132

Verbindungen der allgemeinen Formel IB.2, in denen R² für Methyl, R³ für Methyl, R⁴ für Methyl und R⁵_{y} für Wasserstoff steht und R¹ für eine Verbindung jeweils einer Gruppe der Tabelle A entspricht.

### Tabelle 133

Verbindungen der allgemeinen Formel IB.3, in denen R² für Methyl, R³ für Methyl, R⁴ für Methyl und R⁵_{y} für Wasserstoff steht und R¹ für eine Verbindung jeweils einer Gruppe der Tabelle A entspricht.

### Tabelle 134

Verbindungen der allgemeinen Formel IB.4, in denen R² für Methyl, R³ für Methyl, R⁴ für Methyl und R⁵_{y} für Wasserstoff steht und R¹ für eine Verbindung jeweils einer Gruppe der Tabelle A entspricht.

### Tabelle 135

Verbindungen der allgemeinen Formel IB.5, in denen R² für Methyl, R³ für Methyl, R⁴ für Methyl und R⁵_{y} für Wasserstoff steht und R¹ für eine Verbindung jeweils einer Gruppe der Tabelle A entspricht.

### Tabelle 136

Verbindungen der allgemeinen Formel IB.6, in denen R² für Methyl, R³ für Methyl, R⁴ für Methyl und R⁵_{y} für Wasserstoff steht und R¹ für eine Verbindung jeweils einer Gruppe der Tabelle A entspricht.

### Tabelle 137

Verbindungen der allgemeinen Formel IB.7, in denen R² für Methyl, R³ für Methyl, R⁴ für Methyl und R⁵_{y} für Wasserstoff steht und R¹ für eine Verbindung jeweils einer Gruppe der Tabelle A entspricht.

### Tabelle 138

Verbindungen der allgemeinen Formel IB.8, in denen R² für Methyl, R³ für Methyl, R⁴ für Methyl und R⁵_{y} für Wasserstoff steht und R¹ für eine Verbindung jeweils einer Gruppe der Tabelle A entspricht.

### Tabelle 139

Verbindungen der allgemeinen Formel IB.9, in denen R² für Methyl, R³ für Methyl, R⁴ für Methyl und R⁵_{y} für Wasserstoff steht und R¹ für eine Verbindung jeweils einer Gruppe der Tabelle A entspricht.

### Tabelle 140

Verbindungen der allgemeinen Formel IB.10, in denen R² für Methyl, R³ für Methyl, R⁴ für Methyl und R⁵_{y} für Wasserstoff steht und R¹ für eine Verbindung jeweils einer Gruppe der Tabelle A entspricht.

**Tabelle A**

| Nr. | R¹ |
|---|---|
| A.1 | H |
| A.2 | CH₃ |
| A.3 | C₂H₅ |
| A.4 | n-C₃H₇ |
| A.5 | i-C₃H₇ |
| A.7 | n-C₄H₉ |
| A.8 | s-C₄H₉ |
| A.9 | i-C₄H₉ |
| A.10 | t-C₄H₉ |
| A.11 | n-C₅H₁₁ |
| A.12 | i-C₅H₁₁ |
| A.13 | neo-C₅H₁₁ |
| A.15 | n-C₆H₁₃ |
| A.18 | CH₂CH₂Cl |
| A.19 | (CH₂)₄Cl |
| A.37 | Propen-3-yl |
| A.38 | But-2-en-1-yl |
| A.39 | 3-Methylbut-2-en-1-yl |
| A.75 | Benyzl |
| A.76 | 2-F-C₆H₄-CH₂ |
| A.77 | 3-F-C₆H₄-CH₂ |
| A.78 | 4-F-C₆H₄-CH₂ |
| A.79 | 2,3-F₂-C₆H₃-CH₂ |
| A.80 | 2,4-F₂-C₆H₃-CH₂ |
| A.81 | 2,5-F₂-C₆H₃-CH₂ |
| A.82 | 2,6-F₂-C₆H₃-CH₂ |
| A.83 | 3,4-F₂-C₆H₃-CH₂ |
| A.84 | 3,5-F₂-C₆H₃-CH₂ |
| A.85 | 2-Cl-C₆H₄-CH₂ |
| A.86 | 3-Cl-C₆H₄-CH₂ |
| A.87 | 4-Cl-C₆H₄-CH₂ |
| A.88 | 2,3-Cl₂-C₆H₃-CH₂ |
| A.89 | 2,4-Cl₂-C₆H₃-CH₂ |
| A.90 | 2,5-Cl₂-C₆H₃-CH₂ |
| A.91 | 2,6-Cl₂-C₆H₃-CH₂ |
| A.92 | 3,4-Cl₂-C₆H₃-CH₂ |
| A.93 | 3,5-Cl₂-C₆H₃-CH₂ |
| A.94 | 2,3,4-Cl₃-C₆H₂-CH₂ |
| A.95 | 2,3,5-Cl₃-C₆H₂-CH₂ |
| A.96 | 2,3,6-Cl₃-C₆H₂-CH₂ |
| A.97 | 2,4,5-Cl₃-C₆H₂-CH₂ |
| A.98 | 2,4,6-Cl₃-C₆H₂-CH₂ |
| A.99 | 3,4,5-Cl₃-C₆H₂-CH₂ |
| A.100 | 2-Br-C₆H₄-CH₂ |
| A.101 | 3-Br-C₆H₄-CH₂ |
| A.102 | 4-Br-C₆H₄-CH₂ |
| A.103 | 2,3-Br₂-C₆H₃-CH₂ |
| A.104 | 2,4-Br₂-C₆H₃-CH₂ |
| A.105 | 2,5-Br₂-C₆H₃-CH₂ |
| A.106 | 2,6-Br₂-C₆H₃-CH₂ |
| A.107 | 3,4-Br₂-C₆H₃-CH₂ |
| A.108 | 3,5-Br₂-C₆H₃-CH₂ |
| A.109 | 2-F, 3-Cl-C₆H₃-CH₂ |
| A.110 | 2-F, 4-Cl-C₆H₃-CH₂ |
| A.111 | 2-F, 5-Cl-C₆H₃-CH₂ |
| A.112 | 2-F, 3-Br-C₆H₃-CH₂ |
| A.113 | 2-F, 4-Br-C₆H₃-CH₂ |
| A.114 | 2-F, 5-Br-C₆H₃-CH₂ |
| A.115 | 2-Cl, 3-Br-C₆H₃-CH₂ |
| A.116 | 2-Cl, 4-Br-C₆H₃-CH₂ |
| A.117 | 2-Cl, 5-Br-C₆H₃-CH₂ |
| A.118 | 3-F, 4-Cl-C₆H₃-CH₂ |
| A.119 | 3-F, 5-Cl-C₆H₃-CH₂ |
| A.120 | 3-F, 6-Cl-C₆H₃-CH₂ |
| A.121 | 3-F, 4-Br-C₆H₃-CH₂ |
| A.122 | 3-F, 5-Br-C₆H₃-CH₂ |
| A.123 | 3-F, 6-Br-C₆H₃-CH₂ |
| A.124 | 3-Cl, 4-Br-C₆H₃-CH₂ |
| A.125 | 3-Cl, 5-Br-C₆H₃-CH₂ |
| A.126 | 3-Cl, 6-Br-C₆H₃-CH₂ |
| A.127 | 4-F, 5-Cl-C₆H₃-CH₂ |
| A.128 | 4-F, 6-Cl-C₆H₃-CH₂ |
| A.129 | 4-F, 5-Br-C₆H₃-CH₂ |
| A.130 | 4-F, 6-Br-C₆H₃-CH₂ |
| A.131 | 4-Cl, 5-Br-C₆H₃-CH₂ |
| A.132 | 5-F, 6-Cl-C₆H₃-CH₂ |
| A.133 | 5-F, 6-Br-C₆H₃-CH₂ |
| A.134 | 5-Cl, 6-Br-C₆H₃-CH₂ |
| A.135 | 3-Br, 4-Cl, 5-Br-C₆H₂-CH₂ |
| A.142 | 2-CH₃-C₆H₄-CH₂ |
| A.143 | 3-CH₃-C₆H₄-CH₂ |
| A.144 | 4-CH₃-C₆H₄-CH₂ |
| A.145 | 2,3-(CH₃)₂-C₆H₃-CH₂ |
| A.146 | 2,4-(CH₃)₂-C₆H₃-CH₂ |
| A.147 | 2,5-(CH₃)₂-C₆H₃-CH₂ |
| A.148 | 2,6-(CH₃)₂-C₆H₃-CH₂ |
| A.149 | 3,4-(CH₃)₂-C₆H₃-CH₂ |
| A.150 | 3,5-(CH₃)₂-C₆H₃-CH₂ |
| A.151 | 2-C₂H₅-C₆H₄-CH₂ |
| A.152 | 3-C₂H₅-C₆H₄-CH₂ |
| A.153 | 4-C₂H₅-C₆H₄-CH₂ |
| A.154 | 2-i-C₃H₇-C₆H₄-CH₂ |
| A.155 | 3-i-C₃H₇-C₆H₄-CH₂ |
| A.156 | 4-i-C₃H₇-C₆H₄-CH₂ |
| A.173 | 2-OCH₃-C₆H₄-CH₂ |
| A.174 | 3-OCH₃-C₆H₄-CH₂ |
| A.175 | 4-OCH₃-C₆H₄-CH₂ |
| A.176 | 2,3-(OCH₃)₂-C₆H₃-CH₂ |
| A.177 | 2,4-(OCH₃)₂-C₆H₃-CH₂ |
| A.178 | 2,5-(OCH₃)₂-C₆H₃-CH₂ |
| A.179 | 3,4-(OCH₃)₂-C₆H₃-CH₂ |
| A.180 | 3,5-(OCH₃)₂-C₆H₃-CH₂ |
| A.181 | 3,4,5-(OCH₃)₃-C₆H₂-CH₂ |
| A.182 | 2-OC₂H₅-C₆H₄-CH₂ |
| A.183 | 3-OC₂H₅-C₆H₄-CH₂ |
| A.184 | 4-OC₂H₅-C₆H₄-CH₂ |
| A.185 | 2-O-(n-C₃H₇)-C₆H₄-CH₂ |
| A.186 | 3-O-(n-C₃H₇)-C₆H₄-CH₂ |
| A.187 | 4-O-(n-C₃H₇)-C₆H₄-CH₂ |
| A.188 | 2-O-(i-C₃H₇)-C₆H₄-CH₂ |
| A.189 | 3-O-(i-C₃H₇)-C₆H₄-CH₂ |
| A.190 | 4-O-(i-C₃H₇)-C₆H₄-CH₂ |
| A.191 | 4-O-(n-C₄H₉)-C₆H₄-CH₂ |
| A.192 | 3-O-(t-C₄H₉)-C₆H₄-CH₂ |
| A.193 | 4-O-(n-C₆H₁₃)-C₆H₄-CH₂ |
| A.194 | 2-O-Allyl-C₆H₄-CH₂ |
| A.195 | 3-O-Allyl-C₆H₄-CH₂ |
| A.196 | 4-O-Allyl-C₆H₄-CH₂ |
| A.197 | 2-CF₃-C₆H₄-CH₂ |
| A.198 | 3-CF₃-C₆H₄-CH₂ |
| A.199 | 4-CF₃-C₆H₄-CH₂ |
| A.243 | 1-Naphthyl-CH₂ |
| A.244 | 2-Naphthyl-CH₂ |
| A.300 | 2-Phenyleth-1-yl |
| A.301 | 2-(2'-Chlorphenyl)eth-1-yl |
| A.302 | 2-(3'-Chlorphenyl)eth-1-yl |
| A.303 | 2-(4'-Chlorphenyl)eth-1-yl |
| A.304 | 2-(3',5'-Dichlorphenyl)eth-1-yl |
| A.308 | 2- (2'-Methylphenyl)eth-1-yl |
| A.309 | 2-(3'-Methylphenyl)eth-1-yl |
| A.310 | 2-(4'-Methylphenyl)eth-1-yl |
| A.314 | 2- (2'-Trifluormethylphenyl)eth-1-yl |
| A.315 | 2- (3' -Trifluormethylphenyl)eth-1-yl |
| A.316 | 2-(4'-Trifluormethylphenyl)eth-1-yl |
| A.592 | 2-t-C₄H₉-C₆H₄-CH₂ |
| A.593 | 3-t-C₄H₉-C₆H₄-CH₂ |
| A.594 | 4-t-C₄H₉-C₆H₄-CH₂ |

Die Verbindungen I eignen sich als Fungizide.

Die Verbindungen I zeichnen sich durch eine hervorragende Wirksamkeit gegen ein breites Spektrum von pflanzenpathogenen Pilzen, insbesondere aus der Klasse der Ascomyceten, Phycomyceten und Basidiomyceten, aus. Sie sind zum Teil systemisch wirksam und können im Pflanzenschutz als Blatt- und Bodenfungizide eingesetzt werden.

Besondere Bedeutung haben sie für die Bekämpfung einer Vielzahl von Pilzen an verschiedenen Kulturpflanzen wie Weizen, Roggen, Gerste, Hafer, Reis, Mais, Gras, Baumwolle, Soja, Kaffee, Zuckerrohr, Wein, Obst- und Zierpflanzen und Gemüsepflanzen wie Gurken, Bohnen und Kürbisgewächsen, sowie an den Samen dieser Pflanzen.

Speziell eignen sie sich zur Bekämpfung folgender Pflanzenkrankheiten: Erysiphe graminis (echter Mehltau) in Getreide, Erysiphe cichoracearum und Sphaerotheca fuliginea an Kürbisgewächsen, Podosphaera leucotricha an Äpfeln, Uncinula necator an Reben, Puccinia-Arten an Getreide, Rhizoctonia-Arten an Baumwolle, Reis und Rasen, Ustilago-Arten an Getreide und Zuckerrohr, venturia inaequalis (Schorf) an Äpfeln, Helminthosporium-Arten an Getreide, Septoria nodorum an Weizen, Botrytis cinerea (Grauschimmel) an Erdbeeren, Gemüse und Zierpflanzen, Reben, Cercospora arachidicola an Erdnüssen, Pseudocercosporella herpotrichoides an Weizen, Gerste, Pyricularia oryzae an Reis, Phytophthora infestans an Kartoffeln und Tomaten, Fusarium- und Verticillium-Arten an verschiedenen Pflanzen, Plasmopara viticola an Reben, Pseudoperonospora-Arten in Äpfeln und Gurken, Alternaria-Arten an Gemüse und Obst.

Die Verbindungen I eignen sich außerdem zur Bekämpfung von Schadpilzen im Materialschutz (z.B. Holz, Papier, Fasern bzw. Gewebe) und im Vorratsschutz.

Die Verbindungen I werden angewendet, indem man die Pilze oder die vor Pilzbefall zu schützenden Pflanzen, Saatgüter, Materialien oder den Erdboden mit einer fungizid wirksamen Menge der Wirkstoffe behandelt. Die Anwendung erfolgt vor oder nach der Infektion der Materialien, Pflanzen oder Samen durch die Pilze.

Sie können in die üblichen Formulierungen überführt werden, wie Lösungen, Emulsionen, Suspensionen, Stäube, Pulver, Pasten und Granulate. Die Anwendungsform richtet sich nach dem jeweiligen Verwendungszweck; sie soll in jedem Fall eine feine und gleichmäßige Verteilung der erfindungsgemäßen Verbindung gewährleisten. Die Formulierungen werden in bekannter Weise hergestellt, z.B. durch Verstrecken des Wirkstoffs mit Lösungsmitteln und/oder Trägerstoffen, gewünschtenfalls unter Verwendung von Emulgiermitteln und Dispergiermitteln, wobei im Falle von Wasser als Verdünnungsmittel auch andere organische Lösungsmittel als Hilfslösungsmittel verwendet werden können. Als Hilfsstoffe kommen dafür im wesentlichen in Betracht: Lösungsmittel wie Aromaten (z.B. Xylol), chlorierte Aromaten (z.B. Chlorbenzole), Paraffine (z.B. Erdölfraktionen), Alkohole (z.B. Methanol, Butanol), Ketone (z.B. Cyclohexanon), Amine (z.B.Ethanolamin, Dimethylformamid) und Wasser; Trägerstoffe wie natürliche Gesteinsmehle (z.B. Kaoline, Tonerden, Talkum, Kreide) und synthetische Gesteinsmehle (z.B. hochdisperse Kieselsäure, Silikate); Emulgiermittel wie nichtionogene und anionische Emulgatoren (z.B. Polyoxyethylen-Fettalkohol-Ether, Alkylsulfonate und Arylsulfonate) und Dispergiermittel wie Lignin-Sulfitablaugen und Methylcellulose.

Die fungiziden Mittel enthalten im allgemeinen zwischen 0,1 und 95, vorzugsweise zwischen 0,5 und 90 Gew.-% Wirkstoff.

Die Aufwandmengen liegen bei der Anwendung im Pflanzenschutz je nach Art des gewünschten Effektes zwischen 0,01 und 2,0 kg Wirkstoff pro ha.

Bei der Saatgutbehandlung werden im allgemeinen Wirkstoffmengen von 0,001 bis 0,1 g, vorzugsweise 0,01 bis 0,05 g je Kilogramm Saatgut benötigt.

Bei der Anwendung im Material- bzw. Vorratsschutz richtet sich die Aufwandmenge an Wirkstoff nach der Art des Einsatzgebietes und des gewünschten Effekts. Übliche Aufwandmengen sind im Materialschutz beispielsweise 0,001 g bis 2 kg, vorzugsweise 0,005 g bis 1 kg Wirkstoff pro Qubikmeter behandelten Materials.

Die erfindungsgemäßen Mittel können in der Anwendungsform als Fungizide auch zusammen mit anderen Wirkstoffen vorliegen, der z.B. mit Herbiziden, Insektiziden, Wachstumsregulatoren, Fungiziden oder auch mit Düngemitteln.

Beim Vermischen mit Fungiziden erhält man dabei in vielen Fällen eine Vergrößerung des fungiziden Wirkungsspektrums.

Die folgende Liste von Fungiziden, mit denen die erfindungsgemäßen Verbindungen gemeinsam angewendet werden können, soll die Kombinationsmöglichkeiten erläutern, nicht aber einschränken:
Schwefel, Dithiocarbamate und deren Derivate, wie Ferridimethyldithiocarbamat, Zinkdimethyldithiocarbamat, Zinkethylenbisdithiocarbamat, Manganethylenbisdithiocarbamat, Mangan-Zink-ethylendiamin-bis-dithiocarbamat, Tetramethylthiuramdisulfide, Ammoniak-Komplex von Zink-(N,N-ethylen-bis-dithiocarbamat), Ammoniak-Komplex von Zink-(N,N'-propylen-bis-dithiocarbamat), Zink-(N,N'-propylenbis-dithiocarbamat), N,N'-Polypropylen-bis-(thiocarbamoyl)disulfid;
Nitroderivate, wie Dinitro-(1-methylheptyl)-phenylcrotonat, 2-sec-Butyl-4,6-dinitrophenyl-3,3-dimethylacrylat, 2-sec-Butyl-4,6-dinitrophenyl-isopropylcarbonat, 5-Nitro-isophthalsäure-diisopropylester;
heterocyclische Substanzen, wie 2-Heptadecyl-2-imidazolin-acetat, 2,4-Dichlor-6-(o-chloranilino)-s-triazin, O,O-Diethyl-phthalimidophosphonothioat, 5-Amino-1-[bis-(dimethylamino)-phosphinyl]-3-phenyl-1,2,4- triazol, 2,3-Dicyano-1,4-dithioanthrachinon, 2-Thio-1,3-dithiolo[4,5-b]chinoxalin, 1-(Butylcarbamoyl)-2-benzimidazol-carbaminsäuremethylester, 2-Methoxycarbonylamino-benzimidazol, 2-(Furyl-(2))-benzimidazol, 2-(Thiazolyl-(4))-benzimidazol, N-(1,1,2,2-Tetrachlorethylthio)-tetrahydrophthalimid, N-Trichlormethylthio-tetrahydrophthalimid, N-Trichlormethylthio-phthalimid,
N-Dichlorfluormethylthio-N',N'-dimethyl-N-phenyl-schwefelsäurediamid, 5-Ethoxy-3-trichlormethyl-1,2,3-thiadiazol, 2-Rhodanmethylthiobenzthiazol, 1,4-Dichlor-2,5-dimethoxybenzol, 4-(2-Chlorphenylhydrazono)-3-methyl-5-isoxazolon, Pyridin-2-thio-1-oxid, 8-Hydroxychinolin bzw. dessen Kupfersalz, 2,3-Dihydro-5-carboxanilido-6-methyl-1,4-oxathin, 2,3-Dihydro-5-carboxanilido-6-methyl-1,4-oxathiin-4,4-dioxid, 2-Methyl-5,6-dihydro-4H-pyran-3-carbonsäure-anilid, 2-Methyl-furan-3-carbonsäureanilid, 2,5-Dimethyl-furan-3-carbonsäureanilid, 2,4,5-Trimethyl-furan-3-carbonsäureanilid, 2,5-Dimethyl-furan-3-carbonsäurecyclohexylamid, N-Cyclohexyl-N-methoxy-2,5-dimethyl-furan-3-carbonsäureamid, 2-Methyl-benzoesäure-anilid, 2-Iod-benzoesäure-anilid, N-Formyl-N-morpholin-2,2,2-trichlorethylacetal, Piperazin-1,4-diylbis-(1-(2,2,2-trichlor-ethyl)-formamid, 1-(3,4-Dichloranilino)-1-formylamino-2,2,2-trichlorethan, 2,6-Dimethyl-N-tridecylmorpholin bzw. dessen Salze, 2,6-Dimethyl-N-cyclododecyl-morpholin bzw. dessen Salze, N-(3-(p-tert.-Butylphenyl)-2-methylpropyl]-cis-2,6-dimethyl-morpholin, N- [3-(p-tert.-Butylphenyl)-2-methylpropyl]-piperidin, 1-[2-(2,4-Dichlorphenyl)-4-ethyl-1,3-dioxolan-2-yl-ethyl]-1H-1,2,4-triazol, 1-[2-(2,4-Dichlorphenyl)-4-n-propyl-1,3-dioxolan-2-yl-ethyl]-1H-1,2,4-triazol, N-(n-Propyl)-N-(2,4,6-trichlorphenoxyethyl)-N'-imidazol-yl-harnstoff, 1-(4-Chlorphenoxy)-3,3-dimethyl-1-(1H-1,2,4-triazol-1-yl)-2-butanon, 1-(4-Chlorphenoxy)-3,3-dimethyl-1-(1H-1,2,4-triazol-1-yl)-2-butanol, α-(2-Chlorphenyl)-α-(4-chlorphenyl)-5-pyrimidinmethanol, 5-Butyl-2-dimethylamino-4-hydroxy-6-methyl-pyrimidin, Bis-(p-chlorphenyl)-3-pyridinmethanol, 1,2-Bis-(3-ethoxycarbonyl-2-thioureido)-benzol, 1,2-Bis-(3-methoxycarbonyl-2-thioureido)-benzol,
sowie verschiedene Fungizide, wie Dodecylguanidinacetat, 3-[3-(3,5-Dimethyl-2-oxycyclohexyl)-2-hydroxyethyl]-glutarimid, Hexachlorbenzol, DL-Methyl-N-(2,6-dimethyl-phenyl)-N-furoyl(2)-alaninat, DL-N-(2,6-Dimethyl-phenyl)-N-(2'-methoxyacetyl)-alanin-methyl- ester, N-(2,6-Dimethylphenyl)-N-chloracetyl-D,L-2-aminobutyrolacton, DL-N-(2,6-Dimethylphenyl)-N-(phenylacetyl)-alaninmethylester, 5-Methyl-5-vinyl-3-(3,5-dichlorphenyl)-2,4-dioxo-1,3-oxazolidin, 3-[3,5-Dichlorphenyl(-5-methyl-5-methoxymethyl]-1,3-oxazolidin- 2,4-dion, 3-(3,5-Dichlorphenyl)-1-isopropylcarbamoylhydantoin, N-(3,5-Dichlorphenyl)-1,2-dimethylcyclopropan-1,2-dicarbonsäureimid, 2-Cyano-[N-(ethylaminocarbonyl)-2-methoximino]-acetamid, 1-[2-(2,4-Dichlorphenyl)-pentyl]-1,2,4-triazol, 2,4-Difluor-α-(1H-1,2,4-triazolyl-1-methyl) -benzhydrylalkohol, N-(3-Chlor-2,6-dinitro-4-trifluormethyl-phenyl)-5-trifluormethyl-3-chlor-2-aminopyridin, 1-((bis-(4-Fluorphenyl)-methylsilyl)-methyl)-1H-1,2,4-triazol.

Strobilurine wie Methyl-E-methoxyimino-[α-(o-tolyloxy)-o-tolyl]-acetat, Methyl-E-2-{2-[6-(2-cyanophenoxy)-pyrimidin-4-yloxy]-phenyl}-3-methoxyacrylat, Methyl-E-methoxyimino-[α-(2-phenoxyphenyl)]-acetamid, Methyl-E-methoxyimino-[α-(2,5-dimethylphenoxy)-o-tolyl]-acetamid.

Anilinopyrimidine wie N-(4,6-Dimethylpyrimidin-2-yl)-anilin, N- [4-Methyl-6-(1-propinyl)-pyrimidin-2-yl]-anilin, N- (4-Methyl-6-cyclopropyl-pyrimidin-2-yl)-anilin.

Phenylpyrrole wie 4-(2,2-Difluor-1,3-benzodioxol-4-yl)-pyrrol-3-carbonitril.

Zimtsäureamide wie 3-(4-Chlorphenyl)-3-(3,4-dimethoxyphenyl)-acrylsäuremorpholid.

Die Verbindungen der Formel I sind außerdem geeignet, Schädlinge aus der Klasse der Insekten, Spinnentiere und Nematoden wirksam zu bekämpfen. Sie können im Pflanzenschutz sowie auf dem Hygiene-, Vorratsschutz- und Veterinärsektor als Schädlingsbekämpfungsmittel eingesetzt werden.

Zu den schädlichen Insekten gehören aus der Ordnung der Schmetterlinge (Lepidoptera) beispielsweise Agrotis ypsilon, Agrotis segetum, Alabama argillacea, Anticarsia gemmatalis, Argyresthia conjugella, Autographa gamma, Bupalus piniarius, Cacoecia murinana, Capua reticulana, Cheimatobia brumata, Choristoneura fumiferana, Choristoneura occidentalis, Cirphis unipuncta, Cydia pomonella, Dendrolimus pini, Diaphania nitidalis, Diatraea grandiosella, Earias insulana, Elasmopalpus lignosellus, Eupoecilia ambiguella, Evetria bouliana, Feltia subterranea, Galleria mellonella, Grapholitha funebrana, Grapholitha molesta, Heliothis armigera, Heliothis virescens, Heliothis zea, Hellula undalis, Hibernia defoliaria, Hyphantria cunea, Hyponomeuta malinellus, Keiferia lycopersicella, Lambdina fiscellaria, Laphygma exigua, Leucoptera coffeella, Leucoptera scitella, Lithocolletis blancardella, Lobesia botrana, Loxostege sticticalis, Lymantria dispar, Lymantria monacha, Lyonetia clerkella, Malacosoma neustria, Mamestra brassicae, Orgyia pseudotsugata, Ostrinia nubilalis, Panolis flammea, Pectinophora gossypiella, Peridroma saucia, Phalera bucephala, Phthorimaea operculella, Phyllocnistis citrella, Pieris brassicae, Plathypena scabra, Plutella xylostella, Pseudoplusia includens, Rhyacionia frustrana, Scrobipalpula absoluta, Sitotroga cerealella, Sparganothis pilleriana, Spodoptera frugiperda, Spodoptera littoralis, Spodoptera litura, Thaumatopoea pityocampa, Tortrix viridana, Trichoplusia ni, Zeiraphera canadensis.

Aus der Ordnung der Käfer (Coleoptera) beispielsweise Agrilus sinuatus, Agriotes lineatus, Agriotes obscurus, Amphimallus solstitialis, Anisandrus dispar, Anthonomus grandis, Anthonomus pomorum, Atomaria linearis, Blastophagus piniperda, Blitophaga undata, Bruchus rufimanus, Bruchus pisorum, Bruchus lentis, Byctiscus betulae, Cassida nebulosa, Cerotoma trifurcata, Ceuthorrhynchus assimilis, Ceuthorrhynchus napi, Chaetocnema tibialis, Conoderus vespertinus, Crioceris asparagi, Diabrotica longicornis, Diabrotica 12-punctata, Diabrotica virgifera, Epilachna varivestis, Epitrix hirtipennis, Eutinobothrus brasiliensis, Hylobius abietis, Hypera brunneipennis, Hypera postica, Ips typographus, Lema bilineata, Lema melanopus, Leptinotarsa decemlineata, Limonius californicus, Lissorhoptrus oryzophilus, Melanotus communis, Meligethes aeneus, Melolontha hippocastani, Melolontha melolontha, Oulema oryzae, Ortiorrhynchus sulcatus, Otiorrhynchus ovatus, Phaedon cochleariae, Phyllotreta chrysocephala, Phyllophaga sp., Phyllopertha horticola, Phyllotreta nemorum, Phyllotreta striolata, Popillia japonica, Sitona lineatus, Sitophilus granaria.

Aus der Ordnung der Zweiflügler (Diptera) beispielsweise Aedes aegypti, Aedes vexans, Anastrepha ludens, Anopheles maculipennis, Ceratitis capitata, Chrysomya bezziana, Chrysomya hominivorax, Chrysomya macellaria, Contarinia sorghicola, Cordylobia anthropophaga, Culex pipiens, Dacus cucurbitae, Dacus oleae, Dasineura brassicae, Fannia canicularis, Gasterophilus intestinalis, Glossina morsitans, Haematobia irritans, Haplodiplosis equestris, Hylemyia platura, Hypoderma lineata, Liriomyza sativae, Liriomyza trifolii, Lucilia caprina, Lucilia cuprina, Lucilia sericata, Lycoria pectoralis, Mayetiola destructor, Musca domestica, Muscina stabulans, Oestrus ovis, Oscinella frit, Pegomya hysocyami, Phorbia antiqua, Phorbia brassicae, Phorbia coarctata, Rhagoletis cerasi, Rhagoletis pomonella, Tabanus bovinus, Tipula oleracea, Tipula paludosa.

Aus der Ordnung der Thripse (Thysanoptera) beispielsweise Frankliniella fusca, Frankliniella occidentalis, Frankliniella tritici, Scirtothrips citri, Thrips oryzae, Thrips palmi, Thrips tabaci.

Aus der Ordnung der Hautflügler (Hymenoptera) beispielsweise Athalia rosae, Atta cephalotes, Atta sexdens, Atta texana, Hoplocampa minuta, Hoplocampa testudinea, Monomorium pharaonis, Solenopsis geminata, Solenopsis invicta.

Aus der Ordnung der Wanzen (Heteroptera) beispielsweise Acrosternum hilare, Blissus leucopterus, Cyrtopeltis notatus, Dysdercus cingulatus, Dysdercus intermedius, Eurygaster integriceps, Euschistus impictiventris, Leptoglossus phyllopus, Lygus lineolaris, Lygus pratensis, Nezara viridula, Piesma quadrata, Solubea insularis, Thyanta perditor.

Aus der Ordnung der Pflanzensauger (Homoptera) beispielsweise Acyrthosiphon onobrychis, Adelges laricis, Aphidula nasturtii, Aphis fabae, Aphis pomi, Aphis sambuci, Brachycaudus cardui, Brevicoryne brassicae, Cerosipha gossypii, Dreyfusia nordmannianae, Dreyfusia piceae, Dysaphis radicola, Dysaulacorthum pseudosolani, Empoasca fabae, Macrosiphum avenae, Macrosiphum euphorbiae, Macrosiphon rosae, Megoura viciae, Metopolophium dirhodum, Myzodes persicae, Myzus cerasi, Nilaparvata lugens, Pemphigus bursarius, Perkinsiella saccharicida, Phorodon humuli, Psylla mali, Psylla piri, Rhopalomyzus ascalonicus, Rhopalosiphum maidis, Sappaphis mala, Sappaphis mali, Schizaphis graminum, Schizoneura lanuginosa, Trialeurodes vaporariorum, Viteus vitifolii.

Aus der Ordnung der Termiten (Isoptera) beispielsweise Calotermes flavicollis, Leucotermes flavipes, Reticulitermes lucifugus, Termes natalensis.

Aus der Ordnung der Geradflügler (Orthoptera) beispielsweise Acheta domestica, Blatta orientalis, Blattella germanica, Forficula auricularia, Gryllotalpa gryllotalpa, Locusta migratoria, Melanoplus bivittatus, Melanoplus femur-rubrum, Melanoplus mexicanus, Melanoplus sanguinipes, Melanoplus spretus, Nomadacris septemfasciata, Periplaneta americana, Schistocerca americana, Schistocerca peregrina, Stauronotus maroccanus, Tachycines asynamorus.

Aus der Klasse der Arachnoidea beispielsweise Spinnentiere (Acarina) wie Amblyomma americanum, Amblyomma variegatum, Argas persicus, Boophilus annulatus, Boophilus decoloratus, Boophilus microplus, Brevipalpus phoenicis, Bryobia praetiosa, Dermacentor silvarum, Eotetranychus carpini, Eriophyes sheldoni, Hyalomma truncatum, Ixodes ricinus, Ixodes rubicundus, Ornithodorus moubatä, Otobius megnini, Paratetranychus pilosus, Dermanyssus gallinae, Phyllocoptruta oleivora, Polyphagotarsonemus latus, Psoroptes ovis, Rhipicephalus appendiculatus, Rhipicephalus evertsi, Sarcoptes scabiei, Tetranychus cinnabarinus, Tetranychus kanzawai, Tetranychus pacificus, Tetranychus telarius, Tetranychus urticae.

Aus der Klasse der Nematoden beispielsweise Wurzelgallennematoden, z.B. Meloidogyne hapla, Meloidogyne incognita, Meloidogyne javanica, Zysten bildende Nematoden, z.B. Globodera rostochiensis, Heterodera avenae, Heterodera glycines, Heterodera schachtii, Heterodera trifolii, Stock- und Blattälchen, z.B. Belonolaimus longicaudatus, Ditylenchus destructor, Ditylenchus dipsaci, Heliocotylenchus multicinctus, Longidorus elongatus, Radopholus similis, Rotylenchus robustus, Trichodorus primitivus, Tylenchorhynchus claytoni, Tylenchorhynchus dubius, Pratylenchus neglectus, Pratylenchus penetrans, Pratylenchus curvitatus, Pratylenchus goodeyi.

Die Wirkstoffe können als solche, in Form ihrer Formulierungen oder den daraus bereiteten Anwendungsformen, z.B. in Form von direkt versprühbaren Lösungen, Pulvern, Suspensionen oder Dispersionen, Emulsionen, Öldispersionen, Pasten, Stäubemitteln, Streumitteln, Granulaten durch Versprühen, Vernebeln, Verstäuben, Verstreuen oder Gießen angewendet werden. Die Anwendungsformen richten sich ganz nach den Verwendungszwecken; sie sollten in jedem Fall möglichst die feinste Verteilung der erfindungsgemäßen Wirkstoffe gewährleisten.

Die Wirkstoffkonzentrationen in den anwendungsfertigen Zubereitungen können in größeren Bereichen variiert werden.

Im allgemeinen liegen sie zwischen 0,0001 und 10%, vorzugsweise zwischen 0,01 und 1%.

Die Wirkstoffe können auch mit gutem Erfolg im Ültra-Low-Volume-Verfahren (ULV) verwendet werden, wobei es möglich ist, Formulierungen mit mehr als 95 Gew.-% Wirkstoff oder sogar den Wirkstoff ohne Zusätze auszubringen.

Die Aufwandmenge an Wirkstoff zur Bekämpfung von Schädlingen beträgt unter Freilandbedingungen 0,1 bis 2,0, vorzugsweise 0,2 bis 1,0 kg/ha.

Zur Herstellung von direkt versprühbaren Lösungen, Emulsionen, Pasten oder Öldispersionen kommen Mineralölfraktionen von mittlerem bis hohem Siedepunkt, wie Kerosin oder Dieselöl, ferner Kohlenteeröle sowie Öle pflanzlichen oder tierischen Ursprungs, aliphatische, cyclische und aromatische Kohlenwasserstoffe, z.B. Benzol, Toluol, Xylol, Paraffin, Tetrahydronaphthalin, alkylierte Naphthaline oder deren Derivate, Methanol, Ethanol, Propanol, Butanol, Chloroform, Tetrachlorkohlenstoff, Cyclohexanol, Cyclohexanon, Chlorbenzol, Isophoron, stark polare Lösungsmittel, z.B. Dimethylformamid, Dimethylsulfoxid, N-Methylpyrrolidon, Wasser, in Betracht.

Wäßrige Anwendungsformen können aus Emulsionskonzentraten, Pasten oder netzbaren Pulvern (Spritzpulver, Öldispersionen) durch Zusatz von Wasser bereitet werden. Zur Herstellung von Emulsionen, Pasten oder Öldispersionen können die Substanzen als solche oder in einem Öl oder Lösungsmittel gelöst, mittels Netz-, Haft-, Dispergier- oder Emulgiermitttel in Wasser homogenisiert werden. Es können aber auch aus wirksamer Substanz Netz-, Haft-, Dispergier- oder Emulgiermittel und eventuell Lösungsmittel oder Öl bestehende Konzentrate hergestellt werden, die zur Verdünnung mit Wasser geeignet sind.

Als oberflächenaktive Stoffe kommen Alkali-, Erdalkali-, Ammoniumsalze von Ligninsulfonsäure, Naphthalinsulfonsäure, Phenolsulfonsäure, Dibutylnaphthalinsulfonsäure, Alkylarylsulfonate, Alkylsulfate, Alkylsulfonate, Fettalkoholsulfate und Fettsäuren sowie deren Alkali- und Erdalkalisalze, Salze von sulfatiertem Fettalkoholglykolether, Kondensationsprodukte von sulfoniertem Naphthalin und Naphthalinderivaten mit Formaldehyd, Kondensationsprodukte des Naphthalins bzw. der Naphtalinsulfonsäure mit Phenol und Formaldehyd, Polyoxyethylenoctylphenolether, ethoxy-liertes Isooctylphenol, Octylphenol, Nonylphenol, Alkylphenolpolyglykolether, Tributylphenylpolyglykolether, Alkylarylpolyetheralkohole, Isotridecylalkohol, Fettalkoholethylenoxid-Kondensate, ethoxyliertes Rizinusöl, Polyoxyethylenalkylether, ethoxyliertes Polyoxypropylen, Laurylalkoholpolyglykoletheracetal, Sorbitester, Ligninsulfitablaugen und Methylcellulose in Betracht.

Pulver-, Streu- und Stäubemittel können durch Mischen oder gemeinsames Vermahlen der wirksamen Substanzen mit einem festen Trägerstoff hergestellt werden.

Die Formulierungen enthalten im allgemeinen zwischen 0,01 und 95Gew.-%, vorzugsweise zwischen 0,1 und 90 Gew.-% des Wirkstoffs. Die Wirkstoffe werden dabei in einer Reinheit von 90% bis 100%, vorzugsweise 95% bis 100% (nach NMR-Spektrum) eingesetzt.

Beispiele für Formulierungen sind:
I. 5 Gew.-Teile einer erfindungsgemäßen Verbindung werden mit 95 Gew.-Teilen feinteiligem Kaolin innig vermischt. Man erhält auf diese Weise ein Stäubemittel, das 5 Gew.-% des Wirkstoffs enthält.
II. 30 Gew.-Teile einer erfindungsgemäßen Verbindung werden mit einer Mischung aus 92 Gew.-Teilen pulverförmigem Kieselsäuregel und8 Gew.-Teilen Paraffinöl, das auf die Oberfläche dieses Kieselsäuregels gesprüht wurde, innig vermischt. Man erhält auf diese Weise eine Aufbereitung des Wirkstoffs mit guter Haftfähigkeit (Wirkstoffgehalt 23 Gew.-%).
III. 10 Gew.-Teile einer erfindungsgemäßen Verbindung werden in einer Mischung gelöst, die aus 90 Gew.-Teilen Xylol, 6 Gew.-Teilen des Anlagerungsproduktes von 8 bis 10 Mol Ethylenoxid an 1Mol Ölsäure-N-monoethanolamid, 2 Gew.-Teilen Calciumsalz der Dodecylbenzolsulfonsäure und 2 Gew.-Teilen des Anlagerungsproduktes von 40 Mol Ethylenoxid an 1 Mol Ricinusöl besteht (Wirkstoffgehalt 9 Gew.-%).
IV. 20 Gew.-Teile einer erfindungsgemäßen Verbindung werden in einer Mischung gelöst, die aus 60 Gew.-Teilen Cyclohexanon, 30 Gew.-Teilen Isobutanol, 5 Gew.-Teilen des Anlagerungsproduktes von 7 Mol Ethylenoxid an 1 Mol Isooctylphenol und 5Gew.-Teilen des Anlagerungsproduktes von 40 Mol Ethylenoxid an 1 Mol Ricinusöl besteht (Wirkstoffgehalt 16 Gew.-%).
V. 80 Gew.-Teile einer erfindungsgemäßen Verbindung werden mit 3 Gew.-Teilen des Natriumsalzes der Diisobutylnaphthalin-alpha-sulfonsäure, 10 Gew.-Teilen des Natriumsalzes einer Ligninsulfonsäure aus einer Sulfit-Ablauge und 7 Gew.-Teilen pulverförmigem Kieselsäuregel gut vermischt und in einer Hammermühle vermahlen (Wirkstoffgehalt 80 Gew.-%).
VI. Man vermischt 90 Gew.-Teile einer erfindungsgemäßen Verbindung mit 10 Gew.-Teilen N-Methyl-α-pyrrolidon und erhält eine Lösung, die zur Anwendung in Form kleinster Tropfen geeignet ist (Wirkstoffgehalt 90 Gew.-%).
VII. 20 Gew.-Teile einer erfindungsgemäßen Verbindung werden in einer Mischung gelöst, die aus 40 Gew.-Teilen Cyclohexanon, 30Gew.-Teilen Isobutanol, 20 Gew.-Teilen des Anlagerungsproduktes von 7 Mol Ethylenoxid an 1 Mol Isooctylphenol und 10 Gew.-Teilen des Anlagerungsproduktes von 40 Mol Ethylenoxid an 1 Mol Ricinusöl besteht. Durch Eingießen und feines Verteilen der Lösung in 100 000 Gew.-Teilen Wasser erhält maneine wäßrige Dispersion, die 0,02 Gew.-% des Wirkstoffs enthält.
VIII. 20 Gew.-Teile einer erfindungsgemäßen Verbindung werden mit 3 Gew.-Teilen des Natriumsalzes der Diisobutylnaphthalin-αsulfonsäure, 17 Gew.-Teilen des Natriumsalzes einer Ligninsulfonsäure aus einer Sulfit-Ablauge und 60 Gew.-Teilen pulverförmigem Kieselsäuregel gut vermischt und in einer Hammermühle vermahlen. Durch feines Verteilen der Mischung in 20000 Gew.-Teilen Wasser erhält man eine Spritzbrühe, die 0,1 Gew.-% des Wirkstoffs enthält.

Granulate, z.B. Umhüllungs-, Imprägnierungs- und Homogengranulate, können durch Bindung der Wirkstoffe an feste Trägerstoffe hergestellt werden. Feste Trägerstoffe sind z.B. Mineralerden, wie Silicagel, Kieselsäuren, Kieselgele, Silikate, Talkum, Kaolin, Attaclay, Kalkstein, Kalk, Kreide, Bolus, Löß, Ton, Dolomit, Diatomeenerde, Calcium- und Magnesiumsulfat, Magnesiumoxid, gemahlene Kunststoffe, Düngemittel, wie z.B. Ammoniumsulfat, Ammoniumphosphat, Ammoniumnitrat, Harnstoffe und pflanzliche Produkte, wie Getreidemehl, Baumrinden-, Holz- und Nußschalenmehl, Cellulosepulver und andere feste Trägerstoffe.

Zu den Wirkstoffen können Öle verschiedenen Typs, Herbizide, Fungizide, andere Schädlingsbekämpfungsmittel, Bakterizide, gegebenenfalls auch erst unmittelbar vor der Anwendung (Tankmix), zugesetzt werden. Diese Mittel können zu den erfindungsgemäßen Mit-teln im Gewichtsverhältnis 1:10 bis 10:1 zugemischt werden.

### Synthesebeispiele

Die in den nachstehenden Synthesebeispielen wiedergegebenen Vorschriften wurden unter entsprechender Abwandlung der Ausgangsverbindungen zur Gewinnung weiterer Verbindungen I benutzt. Die so erhaltenen Verbindungen sind in den anschließenden Tabellen mit physikalischen Angaben aufgeführt.

### Beispiel 1 E-2-Methoxyimino-2-[(2-[6-(methoximinoeth-1'-yl)-pyridin-2-yl)]oxy-methyl)phenyl]essigsäuremethylester (Verbindung Nr. 2)

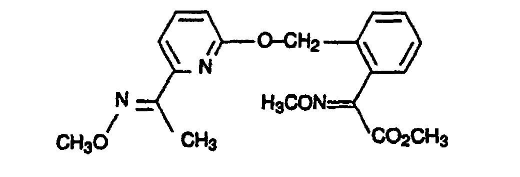

### Stufe 1: 2-Ethoxy-6-brompyridin:

20 g 2,6-Dibrompyridin wurden in 130 ml Dimethylformamid vorgelegt. 6,4 g Natriumethanolat wurden zugegeben. Das Reaktiongemisch wurde für zwei Stunden bei 55°C gerührt. Nach dem Abkühlen auf Raumtemperatur wurde das Reaktionsgemisch in ca. 900 ml halbkonzentrierte wäßrige Ammoniumchlorid-Lösung gegossen. Es wurde 4 mal mit Methyl-tert.-butylether extrahiert. Die vereinigten organischen Phasen wurden 3 mal mit halbkonzentrierter wäßriger Natriumhydrogencarbonat-Lösung gewaschen, anschließend über Natriumsulfat getrocknet. Nach dem Abziehen des Lösungsmittels verblieben 16,8 g 2-Ethoxy-6-brompyridin, das ohne weitere Reinigung für die Folgestufe eingesetzt wurde.

### Stufe 2: 2-Ethoxy-6-acetylpyridin:

16,8 g 2-Ethoxy-6-brompyridin wurden bei -75°C in 220 ml Tetrahydrofuran vorgelegt. 54,4 ml n-Butyllithium (1,6 molare Lösung in n-Hexan) wurden bei dieser Temperatur zugetropft. Es wurde zwei Stunden bei -75°C nachgerührt, dann wurden bei dieser Temperatur 7,6 g N,N-Dimethylacetamid zugetropft. Man rührte eine weitere Stunde bei -75°C, dann wurde auf -10°C erwärmt und bei dieser Temperatur zunächst ca. 170 ml 20 prozentige wäßrige Ammoniumchlorid-Lösung dann 200 ml Wasser zugetropft. Es wurde 3 mal mit Methyl-tert.-butylether extrahiert. Die vereinigten organischen Phasen wurden 3 mal mit Wasser gewaschen, anschließend über Natriumsulfat getrocknet. Nach Abziehen des Lösungsmittels wurde der verbleibende Rückstand an Kieselgel mit Cyclohexan/Methyltert.-butylether 8:1 chromatographiert. Man erhielt 5,5 g Ethoxy-6-acetylpyridin als farblosen Feststoff (Fp.: 36-37°C).

### Stufe 3: 2-Hydroxy-6-acetylpyridin:

48,5 g 2-Ethoxy-6-acetylpyridin wurden in 145 ml Bromwasserstoff-Lösung (30 prozentige Lösung in Eisessig) vorgelegt und auf 95°C erwärmt. Bei dieser Temperatur wurden weitere 176 ml Bromwasserstoff-Lösung (30%-ige Lösung in Eisessig) zugetropft. Es wurde 3 Stunden bei 95°C gerührt, dann auf Raumtemperatur abgekühlt. Es wurde auf Eiswasser gegossen und mit 50 prozentiger wäßriger Natronlauge auf pH 7 neutralisiert. Anschließend wurde 3 mal mit Methyl-tert.-butylether extrahiert. Die wäßrige Phase wurde eingeengt. Der verbleibende Rückstand wurde 4 mal mit Aceton verrührt. Nach dem Einengen der vereinigten Aceton-Phasen verblieben 38,5 g 2-Hydroxy-6-acetylpyridin als farbloser Feststoff (Fp.: 115-117°C).

### Stufe 4: 2-Hydroxy-6-(methoximinoeth-1'-yl)-pyridin:

4,1 g 2-Hydroxy-6-acetylpyridin und 3,8g Methoxyaminhydrochlorid wurden in 60 ml Methanol vorgelegt und mit 5%-iger wäßriger Natronlauge auf pH 5 eingestellt. Es wurde drei Stunden bei 60°C dann 14 Stunden bei Raumtemperatur gerührt. Das Reaktionsgemisch wurde eingeengt. Das verbleibende Rohprodukt wurde an Kieselgel mit Essigester chromatographiert. Man erhielt 4 g 2-Hydroxy-6-(methoximinoeth-1'-yl)-pyridin als farblosen Feststoff (Fp.: 102-103°C).

### Stufe 5: Titelverbindung:

4 g 2-Hydroxy-6-(methoximinoeth-1'-yl)-pyridin, 6,9 g E-2-Methoxyimino-2-[2-(brommethyl)phenyl]essigsäuremethylester und 5 g Kaliumcarbonat wurden in 80 ml Dimethylformamid für 2 Stunden bei 60°C dann 14 Stunden bei Raumtemperatur gerührt. Das Reaktionsgemisch wurde eingeengt. Der verbleibende Rückstand wurde in Methyl-tert.-butylether aufgenommen. Es wurde mit Wasser gewaschen, über Natriumsulfat getrocknet und erneut eingeengt. Das Rohprodukt wurde in 20 ml Isopropanol heiß gelöst. Beim Abkühlen auf Raumtemperatur setzte Kristallisation ein. Es wurden 40 ml n-Hexan zugesetzt. Das ausgefallene Produkt wurde abfiltriert und getrocknet. Man erhielt 6,4 g der Titelverbindung als farblosen Feststoff (Fp.: 100-101°C).

### Beispiel 2 E-2-Methoxyimino-2-[(2-[6-(methoximinoeth-1'-yl)-pyri-din-2-yl)]oxymethyl)phenyl]essigsäuremethylamid (Verbindung Nr. 1)

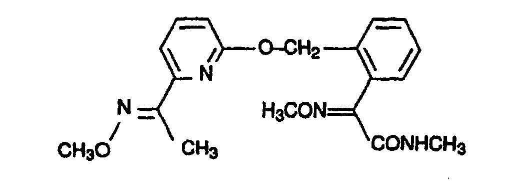

### Weg A:

5,2 g des Methylesters (Titelverbindung aus Beispiel 1) wurden in 50 ml Tetrahydrofuran gelöst und mit 10,9 g 40%-iger wäßriger Methylaminlösung versetzt. Man rührte 5 Stunden bei 60°C. Nach dem Abkühlen wurde der Ansatz eingeengt. Der verbleibende Rückstand wurde in 100 ml Methyl-tert.-butylether aufgenommen. Die organische Phase wurde mit Wasser gewaschen, über Natriumsulfat getrocknet und abschließend bis zur Trockne eingedampft. Es verblieben 4,9 g der Titelverbindung als farbloser Feststoff (Fp.: 63-64°C).

### Weg B:

### Stufe 1: 2-Brom-6-acetylpyridin:

29,7g 2,6-Dibrompyridin wurden nach Literatur [J. Organomet. Chem. 56 (1973) 53 - 66; Chem. Ber. 125 (1992) 1169-1190] in 300 ml Diethylether mit 86,1 ml n-Butyllithium (1,6 molare Lösung in n-Hexan) und 12 g N,N-Dimethylacetamid zu 2-Brom-6-acetylpyridin umgesetzt.

### Stufe 2: 2-Brom-6-(methoximinoeth-1'-yl)-pyridin:

2g 2-Brom-6-acetylpyridin und 1,25g Methoxyaminhydrochlorid wurden in 20 ml Pyridin 14 Stunden bei Raumtemperatur (ca. 25°C) gerührt. Das Reaktionsgemisch wurde in Methyl-tert.-butylether aufgenommen. Die organische Phase wurde zunächst mit 10 prozentiger Salzsäure, dann mit Wasser gewaschen, über Natriumsulfat getrocknet und abschließend bis zur Trockne eingedampft. Es verblieben 2,3 g 2-Brom-6-(methoximinoeth-1'-yl)-pyridin, das ohne weitere Reinigung in Stufe 3 eingesetzt wurde.

### Stufe 3: Titelverbindung:

Zu 0,14g Natriumhydrid in 5 ml Dimethylformamid wurde bei Raumtemperatur eine Lösung aus 1,1 g E-2-Methoxyimino-2-[2-(hydroxy-methyl)phenyl]essigsäuremethylamid in 10 ml Dimethylformamid getropft. Es wurde für 10 Minuten im Ultraschallbad nachgerührt. Anschließend wurde eine Lösung aus 1,15 g 2-Brom-6-(methoximinoeth-1'-yl)-pyridin in 12 ml Dimethylformamid zugetropft. Es wurde vier Stunden bei 120°C gerührt. Nach dem Abkühlen wurde der Ansatz eingeengt. Der verbleibende Rückstand wurde in Methyltert.-butylether aufgenommen. Die organische Phase wurde mit Wasser gewaschen, über Natriumsulfat getrocknet und abschließend bis zur Trockne eingedampft. Der verbleibende Rückstand wurde an Kieselgel mit Cyclohexan/Methyl-tert.-butylether 1:2 chromatographiert. Man erhielt 0,35 g der Titelverbindung als farblosen Feststoff (Fp.: 63 -64°C).

### Beispiel 3 E-2-Methoxyimino-2-[(2-[3-methyl-5-(ethoximinoeth-1'-yl)-pyridin-2-yl)]oxy-methyl)phenyl]essigsäuremethylamid (Verbindung Nr. 17)

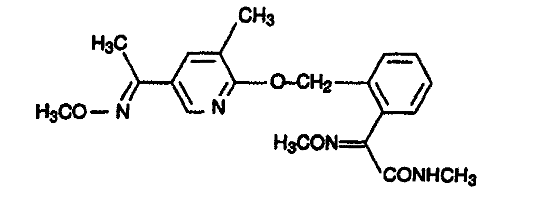

### Stufe 1: 2-Brom-3-methyl-5-acetylpyridin:

12,2 g 2-Brom-3-methyl-5-iodpyridin [hergestellt nach J. Org. Chem. 60 (1995) 5358] wurden in 120 ml Diethylether vorgelegt. Bei -75°C wurden 28,1 ml n-Butyllithium (1,6 molare Lösung in n-Hexan) zugetropft. Es wurde eine Stunde bei -75°C, dann 30 Minuten bei -40°C gerührt. Bei -75°C wurden 3,9 g Dimethylacetamid zugetropft. Es wurde 15 Minuten bei -75°C, dann 2 Stunden bei -40°C gerührt. Dann wurde auf -10°C erwärmt und die Reaktionsmischung wurde mit ca. 100 ml 20%-iger wäßriger Ammoniumchlorid-Lösung versetzt. Die organische Phase wurde abgetrennt. Die wäßrige Phase wurde 3 mal mit Methyl-tert.-butylether extrahiert. Die vereinigten organischen Phasen wurden 3 mal mit Wasser gewaschen, anschließend über Natriumsulfat getrocknet und eingeengt. Der verbleibende Rückstand wurde an Kieselgel mit Cyclohexan/Methyltert.-butylether 3:1 chromatographiert. Man erhielt 5,3 g 2-Brom-3-methyl-5-acetylpyridin als farblosen Feststoff (Fp.: 114-115°C).

### Stufe 2: E-2-Methoxyimino-2-[(2-[3-methyl-5-acetyl-pyridin-2-yl)]oxy-methyl)phenyl]essigsäuremethylamid:

Zu 0,39g Natriumhydrid in 5 ml Dimethylformamid wurde bei Raum-temperatur eine Lösung aus 3,1 g E-2-Methoxyimino-2-[2-(hydroxymethyl)phenyl]essigsäuremethylamid in 25 ml Dimethylformamid getropft. Es wurde für 15 Minuten im Ultraschallbad nachgerührt. Anschließend wurde eine Lösung aus 3g 2-Brom-3-methyl-5-acetylpyridin in 40 ml Dimethylformamid zugetropft. Es wurde drei Stunden bei 60°C gerührt. Nach dem Abkühlen wurde der Ansatz eingeengt. Der verbleibende Rückstand wurde in Essigester aufgenommen. Die organische Phase wurde mit Wasser gewaschen, über Natriumsulfat getrocknet und abschließend bis zur Trockne eingedampft. Der verbleibende Rückstand wurde an Kieselgel mit Cyclohexan/Essigester 1:1 chromatographiert. Man erhielt 2,6 g E-2-Methoxyimino-2-[(2-[3-methyl-5-acetyl-pyridin-2-yl)]oxy-methyl)phenyl]essigsäuremethylamid als farblosen Feststoff (Fp.: 97-99°C).

### Stufe 3: Titelverbindung:

1g E-2-Methoxyimino-2-[(2-[3-methyl-5-acetyl-pyridin-2-yl)]oxymethyl)phenyl]essigsäuremethylamid aus Stufe 2 und 0,57 g Ethoxyaminhydrochlorid wurden in 10 ml Methanol vorgelegt. Das Reaktionsgemisch wurde mit 10%-iger Salzsäure auf pH 5 eingestellt. Es wurde zwei Stunden bei 60°C und weitere 12 Stunden bei Raumtemperatur gerührt. Das Reaktionsgemisch wurde eingeengt. Der verbleibende Rückstand wurde in Methyl-tert.-butylether aufgenommen, mit Wasser gewaschen und erneut eingeengt. Das Rohprodukt wurde in wenig Methyl-tert.-butylether gelöst. Durch Zugabe von n-Pentan wurde das Produkt kristallin ausgefällt. Man erhielt 0,8 g der Titelverbindung als farblosen Feststoff (Fp.: 97-99°C).

### Beispiel 4 E-2-Methoxyimino-2-[(2-[3-chlor-5-(prop-1"-en-3"oximinoeth-1'-yl)-pyridin-2-yl)]oxymethyl)phenyl]essigsäuremethylamid (Verbindung Nr. 14)

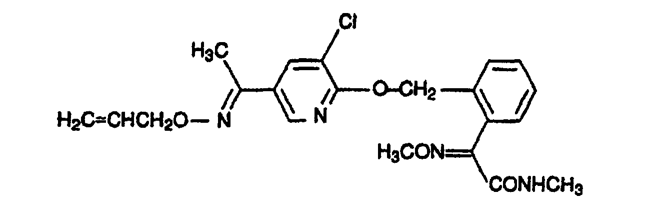

### Stufe 1: 2,3-Dichlor-5-acetylpyridin:

2,3-Dichlor-5-acetylpyridin wurde nach Literatur hergestellt (vgl. DE-A 38 38 243, EP-A 446 872; Tetrahedron 48, 22 (1992) 9233ff.).

### Stufe 2: E-2-Methoxyimino-2- [(2-[3-chlor-5-acetyl-pyridin-2-yl)]oxymethyl)phenyl]essigsäuremethylamid

Zu 2,64g Natriumhydrid in 50 ml Dimethylformamid wurde bei Raumtemperatur eine Lösung aus 22,2 g E-2-Methoxyimino-2-[2-(hydroxymethyl)phenyl]essigsäuremethylamid in 200 ml Dimethylformamid getropft. Es wurde für 20 Minuten im Ultraschallbad nachgerührt. Anschließend wurde eine Lösung aus 19g 2,3-Dichlor-5-acetylpyridin in 150 ml Dimethylformamid zugetropft. Es wurde 14 Stunden bei Raumtemperatur dann fünf Stunden bei 50°C gerührt. Nach dem Abkühlen wurde das Reaktionsgemisch in 1,5 l Wasser aufgenommen und 3 mal mit Methyl-tert.-butylether extrahiert. Die vereinigten organischen Phasen wurden mit Wasser gewaschen, über Natriumsulfat getrocknet und eingeengt. Der verbleibende Rückstand wurde aus Isopropanol umkristallisiert. Man erhielt 15,5 g E-2-Methoxyimino-2-[(2-[3-chlor-5-acetyl-pyridin-2-yl)]oxy-methyl)phenyl]essigsäuremethylamid als farblosen Feststoff (Fp.: 133-134°C).

### Stufe 3: Titelverbindung:

1,5 g E-2-Methoxyimino-2-[(2-[3-chlor-5-acetyl-pyridin-2-yl)]-oxy-methyl)phenyl]essigsäuremethylamid aus Stufe 2 und 0,66 g Prop-1-en-3-oxyaminhydrochlorid wurden in 0,48 g Pyridin für 12 Stunden gerührt. Das Reaktionsgemisch wurde eingeengt. Der verbleibende Rückstand wurde in Methyl-tert.-butylether aufgenommen, mit Wasser gewaschen, über Natriumsulfat getrocknet und erneut eingeengt. Das Rohprodukt wurde aus n-Hexan/Essigester umkristallisiert. Man erhielt 1,25 g der Titelverbindung als farblosen Feststoff (Fp.: 77-79°C).

### Beispiel 5 E-2-Methoxyimino-2- [(2-[5-(prop-1''-in-3''-oximinoeth-1'-yl)-pyridin-2-yl)]oxy-methyl)phenyl]essigsäuremethylamid (Verbindung Nr. 28)

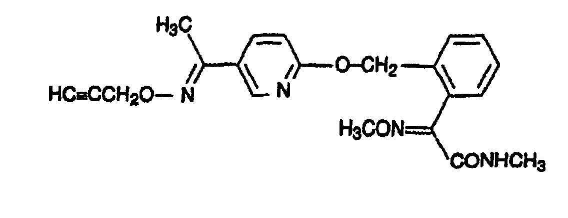

### Stufe 1: 2-Chlor-5-acetylpyridin bzw. 2-Brom-5-acetylpyridin:

2-Chlor-5-acetylpyridin bzw. 2-Brom-5-acetylpyridin wurden nach Literatur hergestellt [siehe Chem. Ber. 125 (1992) 1169 - 1190; Tetrahedron 48, 22 (1992) 9233ff.] und konnten jeweils für Stufe 2 eingesetzt werden. Im folgenden ist exemplarisch die Umsetzung mit 2-Chlor-5-acetylpyridin beschrieben.

### Stufe 2: E-2-Methoxyimino-2-[(2-[5-acetyl-pyridin-2-yl)]oxymethyl)phenyl]essigsäuremethylamid:

Zu 2,22g Natriumhydrid in 20 ml Dimethylformamid wurde bei Raumtemperatur eine Lösung aus 17,8 g E-2-Methoxyimino-2-[2-(hydroxymethyl)phenyl]essigsäuremethylamid in 120 ml Dimethylformamid getropft. Es wurde für 20 Minuten bei Raumtemperatur und für 90 Minuten bei 40°C im Ultraschallbad nachgerührt. Anschließend wurde eine Lösung aus 12,4g 2-Chlor-5-acetylpyridin in 100 ml Dimethylformamid zugetropft. Es wurde 3 Stunden bei 45°C und weitere 12 Stunden bei Raumtemperatur gerührt. Das Reaktionsgemisch wurde in 1 l Wasser aufgenommen und 3 mal mit Methyl-tert.-butylether extrahiert. Die vereinigten organischen Phasen wurden mit Wasser gewaschen, über Natriumsulfat getrocknet und eingeengt. Der verbleibende Rückstand wurde aus Isopropanol umkristallisiert. Man erhielt 16,7 g E-2-Methoxyimino-2-[(2-[5-acetyl-pyridin-2-yl)]oxy-methyl)phenyl]essigsäuremethylamid als farblosen Feststoff (Fp.: 132-134°C).

### Stufe 3: Titelverbindung:

1,4 g E-2-Methoxyimino-2-[(2-[5-acetyl-pyridin-2-yl)]oxy-methyl)phenyl]essigsäuremethylamid aus Stufe 2 in 25 ml Methanol und 0,66 g Prop-1-in-3-oxyaminhydrochlorid wurden in 0,49 g Pyridin für 3 Stunden bei 45°C und 12 Stunden bei Raumtemperatur gerührt. Das Reaktionsgemisch wurde eingeengt. Der verbleibende Rückstand wurde in Methyl-tert.-butylether aufgenommen, mit Wasser gewaschen, über Natriumsulfat getrocknet und erneut eingeengt. Man erhielt 1,54 g der Titelverbindung als farbloses Harz.
IR [cm⁻¹] : 1672, 1602, 1526, 1490, 1316, 1288, 1256, 1039, 1007, 979.

### Beispiel 6 E-2-Methoxyimino-2-[(2-[5-(benzyloximinoeth-1'-yl)-pyridin-2-yl)]oxy-)phenyl]essigsäuremethylamid (Verbindung Nr. 22)

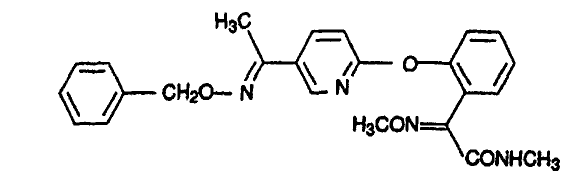

### Stufe 1: E-2-Methoxyimino-2-[(2-[5-acetyl-pyridin-2-yl) ] oxy-)phenyl] essigsäuremethylamid:

8,9g 2-Chor-5-acetylpyridin, 9,9 g E-2-Methoxyimino-2-[2-(hydroxy)phenyl]essigsäuremethylamid und 9,9g Kaliumcarbonat wurden in 120 ml Dimethylformamid 12 Stunden bei 70°C gerührt. Das Reaktionsgemisch wurde in 0,5 l Wasser aufgenommen und 3 mal mit Methyl-tert.-butylether extrahiert. Die vereinigten organischen Phasen wurden mit Wasser gewaschen, über Natriumsulfat getrocknet und eingeengt. Der verbleibende Rückstand wurde an Kieselgel mit Cyclohexan/Essigester 1:2 chromatographiert. Man erhielt 3,5 g E-2-Methoxyimino-2-[(2-[5-acetyl-pyridin-2-yl)]oxy-)phenyl]essigsäuremethylamid als farblosen Feststoff (Fp.: 116-117°C).

### Stufe 3: Titelverbindung:

Ein Reaktionsgemisch aus 0,7 g E-2-Methoxyimino-2-[(2-[5-acetylpyridin-2-yl)]oxy-)phenyl]essigsäuremethylamid (aus Stufe 1) in 15 ml Methanol und 0,34 g Benzyloxyamin wurde mit 10%-iger Salzsäure auf pH 5 eingestellt. Es wurde 3 Stunden bei 45°C und 12 Stunden bei Raumtemperatur gerührt. Das Reaktionsgemisch wurde eingeengt. Der verbleibende Rückstand wurde in Methyl-tert.-butylether aufgenommen, mit Wasser gewaschen, über Natriumsulfat getrocknet und erneut eingeengt. Man erhielt 0,8 g der Titelverbindung als farblosen Feststoff (Fp.: 88-89°C).

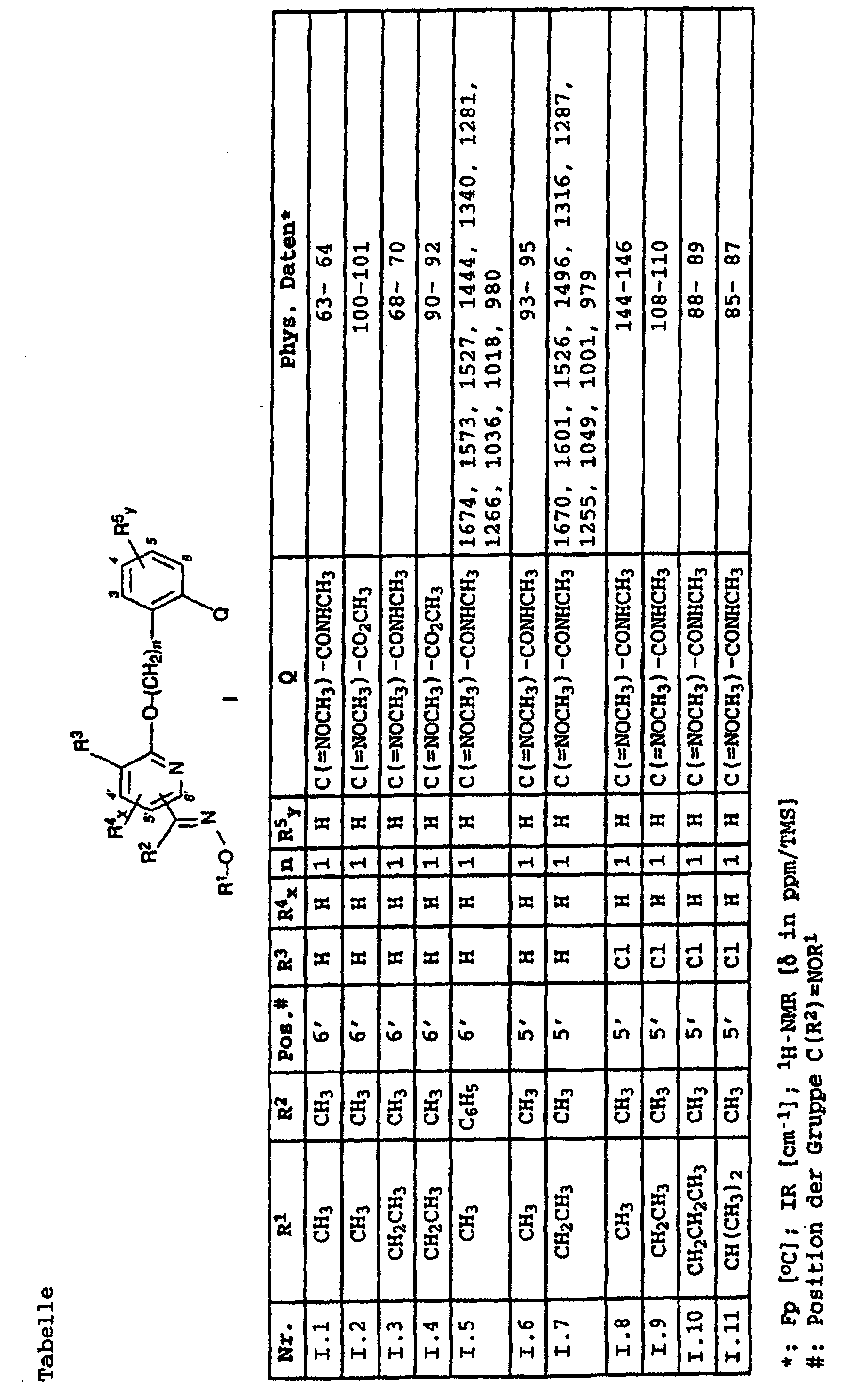

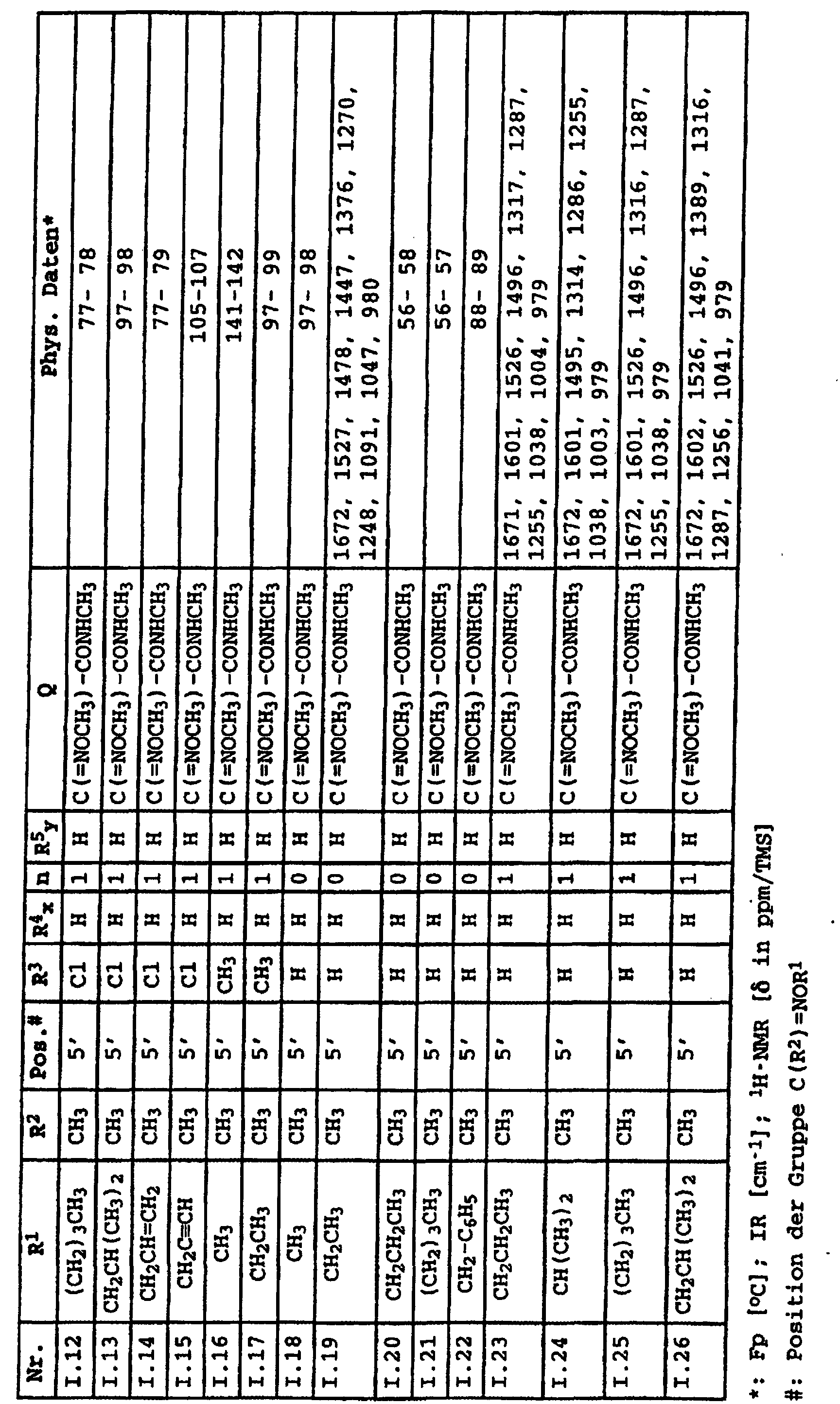

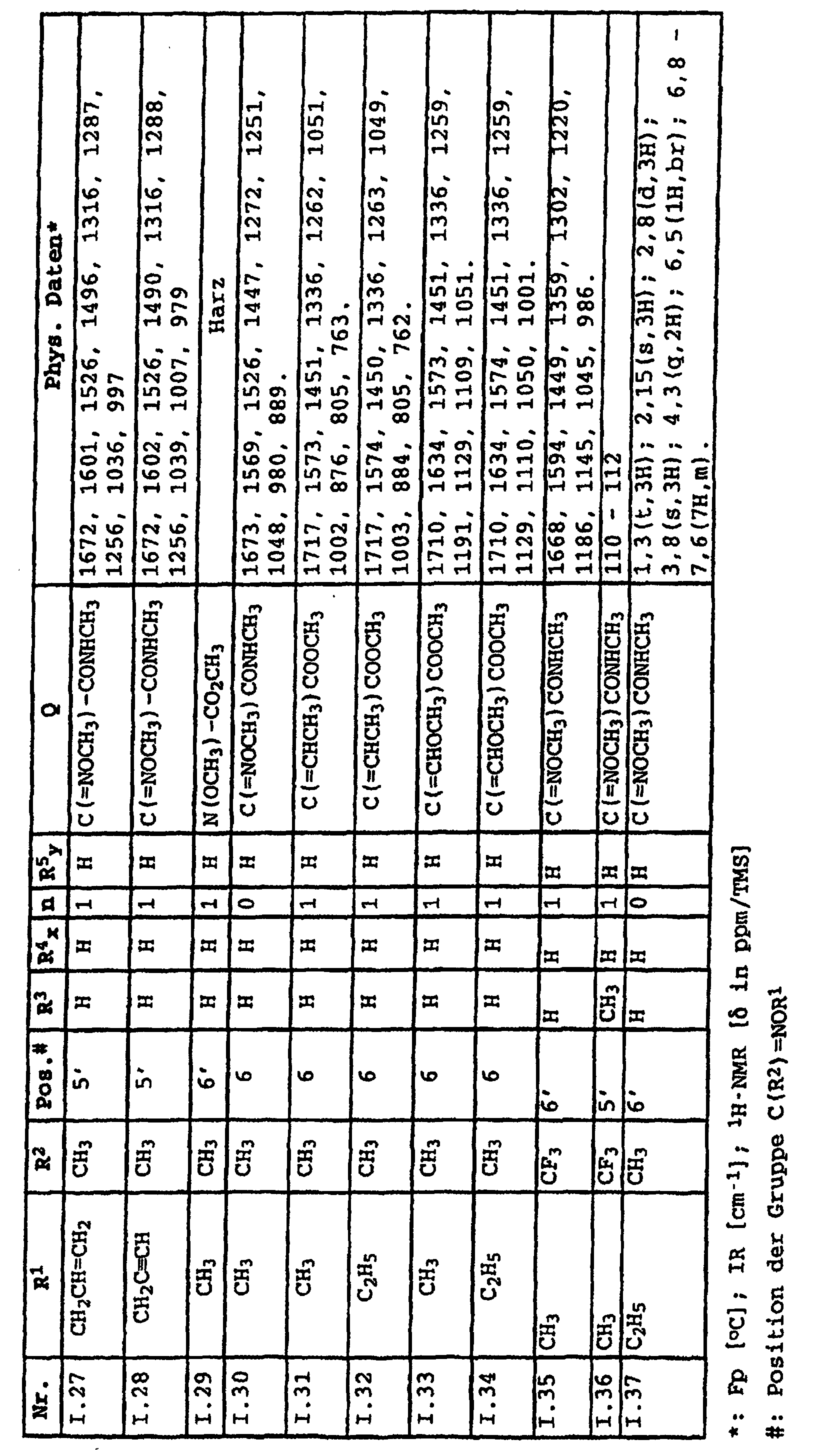

### Beispiele für die Wirkung gegen Schadpilze

Die fungizide Wirkung der Verbindungen der allgemeinen Formel I ließ sich durch die folgenden Versuche zeigen:

Die Wirkstoffe wurden als 20%-ige Emulsion in einem Gemisch aus 70 Gew.-% Cyclohexanon, 20 Gew.-% Nekanil® LN (Lutensol® AP6, Netzmittel mit Emulgier- und Dispergierwirkung auf der Basis ethoxylierter Alkylphenole) und 10 Gew.-% Emulphor® EL (Emulan® EL, Emulgator auf der Basis ethoxylierter Fettalkohole) aufbereitet und entsprechend der gewünschten Konzentration mit Wasser verdünnt.

### Wirkung gegen Plasmopara viticola (Rebenperonospora)

Topfreben (Sorte: "Müller Thurgau") wurden mit der Wirkstoffaufbereitung tropfnaß gespritzt (Aufwandmenge: 16 ppm). Nach 8 Tagen wurden die Pflanzen mit einer Zoosporenaufschwemmung des Pilzes *Plasmopara viticola* besprüht und 5 Tage bei 20-30°C bei hoher Luftfeuchtigkeit aufbewahrt. Vor der Beurteilung wurden die Pflanzen danach für 16h bei hoher Luftfeuchtigkeit aufbewahrt. Die Auswertung erfolgte visuell.

In diesem Test zeigten die mit den erfindungsgemäßen Verbindungen I.1, I.2, I.3, I.4, I.5, I.6, I.7, I.8, I.9, I.10, I.11, I.12, I.13, I.14, I.15, I.16, I.17, I.20, I.23, I.24, I.25, I.26, I.27, I.28, I.29, I.32, I.33 und I.34 behandelten Pflanzen einen Befall von 15% und weniger, während die unbehandelten (Kontroll-) Pflanzen zu 80% befallen waren.

### Wirkung gegen Puccinia recondita (Weizenbraunrost)

Blätter von Weizensämlingen (Sorte "Kanzler") wurden mit Sporen des Braunrosts (*Puccinia recondita*) bestäubt. Die so behandelten Pflanzen wurden 24h bei 20-22°C und einer relativen Luftfeuchtigkeit von 90-95% inkubiert und anschließend mit der wäßrigen Wirkstoffaufbereitung behandelt (Aufwandmenge: 63 ppm). Nach weiteren 8 Tagen bei 20-22°C und 65-70% relativer Luftfeuchtigkeit wurde das Ausmaß der Pilzentwicklung ermittlelt. Die Auswertung erfolgte visuell.

In diesem Test zeigten die mit den erfindungsgemäßen Verbindungen I.1, I.3, I.6, I.7, I.8, I.10, I.11, I.12, I.13, I.14, I.15, I.16, I.17, I.22, I.23, I.24, I.25, I.26, I.28, I.29, I.31, I.32, I.33 und I.34 behandelten Pflanzen einen Befall von 15% und weniger, während die unbehandelten (Kontroll-) Pflanzen zu 60% befallen waren.

### Beispiele für die Wirkung gegen tierische Schädlinge

Die Wirkung der Verbindungen der allgemeinen Formel I gegen tierische Schädlinge ließ sich durch folgende Versuche zeigen:

Die Wirkstoffe wurden
a. als 0,1%-ige Lösung in Aceton oder
b. als 10%-ige Emulsion in einem Gemisch aus 70 Gew.-% Cyclohexanon, 20 Gew.-% Nekanil® LN (Lutensol® AP6, Netzmittel mit Emulgier- und Dispergierwirkung auf der Basis ethoxylierter Alkylphenole) und 10 Gew.-% Emulphor® EL (Emulan® EL, Emulgator auf der Basis ethoxylierter Fettalkohole)
aufbereitet und entsprechend der gewünschten Konzentration mit Aceton im Fall von a. bzw. mit Wasser im Fall von b. verdünnt.

Nach Abschluß der Versuche wurde die jeweils niedrigste Konzentration ermittelt, bei der die Verbindungen im Vergleich zu unbehandelten Kontrollversuchen noch eine 80 bis 100%-ige Hemmung bzw. Mortalität hervorriefen (Wirkschwelle bzw. Minimalkonzentration).

### Nephotettix cincticeps (Grüne Reiszikade), Kontaktwirkung

Rundfilter wurden mit der wäßrigen Wirkstoffaufbereitung behandelt und anschließend mit 5 adulten Zikaden belegt. Nach 24h wurde die Mortalität beurteilt.

In diesem Test zeigten die Verbindungen I.23, I.24, I.25, I.26, I.27 und I.28 Wirkschwellen von 0,2 mg.

### Aphis fabae (Schwarze Laus), Kontaktwirkung

Stark befallene Buschbohnen (Vicia faba) wurden mit der wässrigen Wirkstoffaufbereitung behandelt. Nach 24h wurde die Mortalitätsrate bestimmt.

In diesem Test zeigten die Verbindungen I.7 und I.11 Wirkschwellen von 200 ppm.

### Tetranychus urticae (Spiasmilbe), Kontaktwirkung

Getopfte Buschbohnen, die das zweite Folgeblattpaar zeigten, wurden mit wässrigen Wirkstoffaufbereitung behandelt. Nach 24h wurden die Pflanzen mit Hilfe von stark befallenen Blattstücken infiziert. Nach 12 Tagen im Gewächshaus wurde der Befall bestimmt.

In diesem Test zeigten die Verbindungen I.10, I.11, I.12, I.13, I.14 und I.22 Wirkschwellen von 200 ppm und weniger.

## Patentansprüche

1. Pyridyl-phenyl- und -benzylether der Formel I, 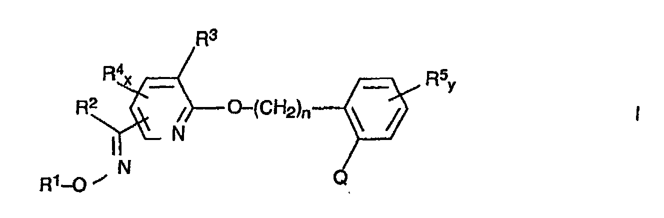 sowie deren Salze und N-Oxide, in denen die Substituenten und Indices die folgende Bedeutung haben:
Q C(CO₂CH₃)=CHCH₃, C(CO₂CH₃)=CHOCH₃, C(CONH₂)=NOCH₃, C(CO₂CH₃)=NOCH₃, C(CONHCH₃)=NOCH₃ oder N(OCH₃)-CO₂CH₃;
n 0 oder 1;
R¹ Wasserstoff oder
C₁-C₆-Alkyl, C₃-C₆-Alkenyl, C₃-C₆-Alkinyl, C₁-C₆-Halogenalkyl, C₃-C₆-Halogenalkenyl, Aryl-C₁-C₂-alkyl, wobei der Arylrest partiell oder vollständig halogeniert sein kann und/oder eine bis drei der folgenden Gruppen tragen kann: C₁-C₄-Alkyl, C₁-C₄-Halogenalkyl und C₁-C₄-Alkoxy.
R² Wasserstoff oder
C₁-C₆-Alkyl, C₂-C₆-Alkenyl, C₂-C₆-Alkinyl, C₃-C₆-Cycloalkyl oder Phenyl.
R³ Wasserstoff, Halogen, C₁-C₄-Alkyl oder C₁-C₂-Halogenalkyl;
x 0, 1 oder 2, wobei die Reste R⁴ verschieden sein können, wenn x für 2 steht;
R⁴ Cyano, Nitro, Halogen, C₁-C₂-Alkyl oder C₁-C₂-Alkoxy;
y 0, 1, 2 oder 3, wobei die Reste R⁵ verschieden sein können, wenn y für 2 oder 3 steht;
R⁵ Cyano, Halogen, C₁-C₄-Alkyl, C₁-C₄-Halogenalkyl oder C₁-C₄-Alkoxy.

2. Verfahren zur Herstellung der Verbindungen I gemäß Anspruch 1, **dadurch gekennzeichnet, daß** man ein Pyridinderivat der Formel IIa 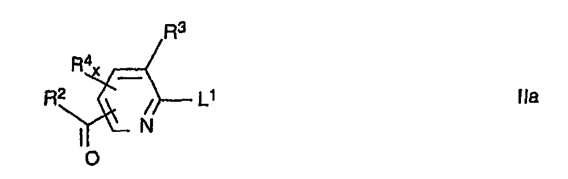 in der L¹ für eine nucleophil austauschbare Abgangsgruppe steht, in einem inerten Lösungsmittel mit einem Phenol bzw. Benzylalkohol der Formel IIIa 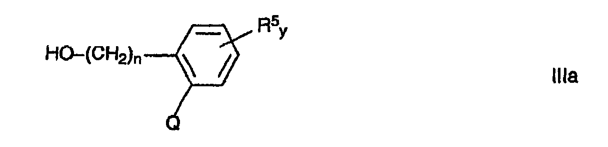 in den entsprechenden Ether der Formel IVa 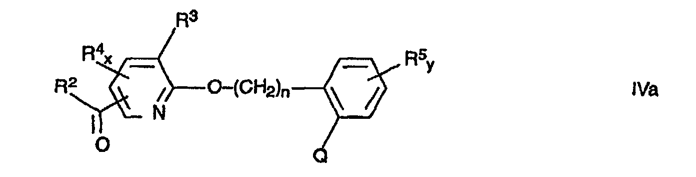 überführt und IVa anschließend mit einem O-substituierten Hydroxylamin (R¹-O-NH₂) oder dessen Salz zu I umsetzt.

3. Verfahren zur Herstellung der Verbindungen I gemäß Anspruch 1, **dadurch gekennzeichnet, daß** man ein Pyridinderivat der Formel IIa gemäß Anspruch 2 mit einem O-substituierten Hydroxylamin (R¹-O-NH₂) oder dessen Salz in die entsprechende Verbindung der Formel Va 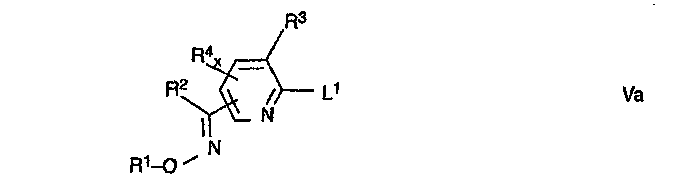 überführt und Va anschließend in einem inerten Lösungsmittel mit einem Phenol bzw. Benzylalkohol der Formel IIIa gemäß Anspruch 2 zu I umsetzt.

4. Verfahren zur Herstellung der Verbindungen I gemäß Anspruch 1, in denen n für 1 steht, **dadurch gekennzeichnet, daß** man einen Pyridinalkohol der Formel IIb 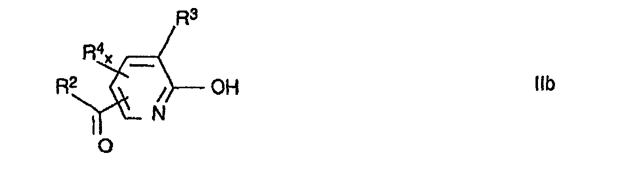 in einem inerten Lösungsmittel mit einer Benzylverbindung der Formel IIIb 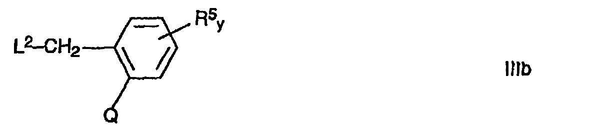 in der L² für eine nucleophil austauschbare Abgangsgruppe steht, in den entsprechenden Benzylether der Formel IVb 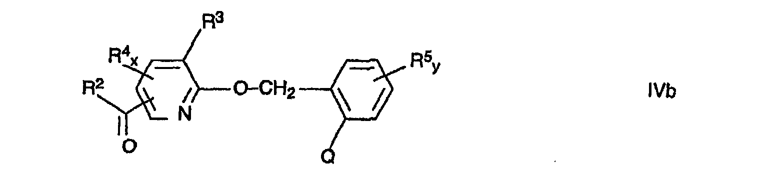 überführt und IVb anschließend mit einem O-substituierten Hydroxylamin (R¹-O-NH₂) oder dessen Salz zu I umsetzt.

5. Verfahren zur Herstellung der Verbindungen I gemäß Anspruch 1, in denen n für 1 steht, **dadurch gekennzeichnet, daß** man einen Pyridinalkohol der Formel IIb gemäß Anspruch 4 mit einem O-substituierten Hydroxylamin (R¹-O-NH₂) oder dessen Salz in die entsprechende Verbindung der Formel Vb überführt 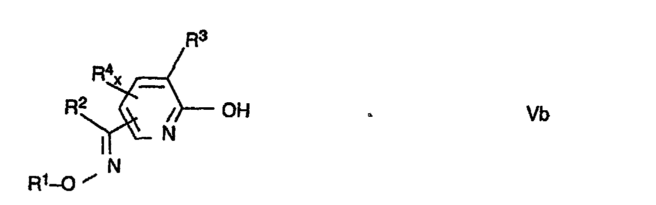 und Vb anschließend in einem inerten Lösungsmittel mit einer Benzylverbindung der Formel IIIb gemäß Anspruch 4 zu I umsetzt.

6. Verfahren zur Herstellung der Verbindungen IVa gemäß Anspruch 2, **dadurch gekennzeichnet, daß** man einen Ether der Formel Xa 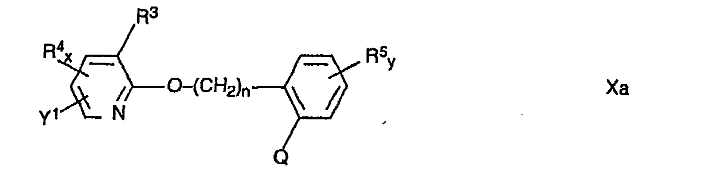 in der Y¹ für ein Halogenatom steht, entweder
a) in Gegenwart einer metallorganischen Base in einem inerten Lösungsmittel mit einer aktivierten Carbonsäure der Formel VIIb 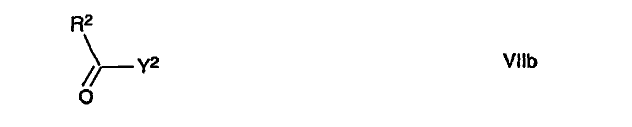 in der Y² für ein Halogenatom, einen Amid- oder einen Esterrest steht, oder einem entsprechenden Cyanid R²-C≡N, oder
b) in einem inerten Lösungsmittel mit einer Zinn-organischen Verbindung der Formel XI 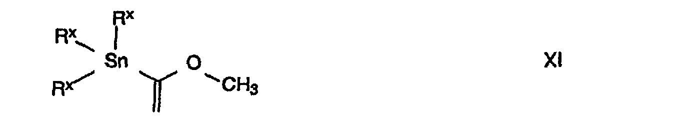 in der die Reste R^{x} voneinander unabhängig für Alkyl stehen,
umsetzt.

7. Verbindungen der allgemeinen Formel II 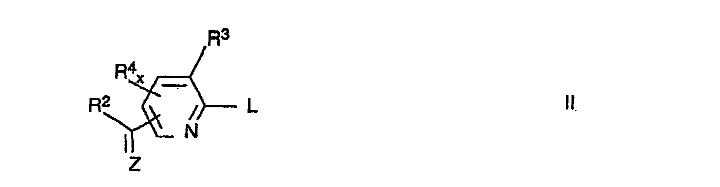 in der die Substituenten R², R³ und R⁴ und der Index x die in Anspruch 1 gegebene Beutung haben und L und Z für die folgenden Gruppen stehen:
L Hydroxy, Halogen, Methyl-, Trifluormethyl-, Phenyl- oder Methylphenyl-Sulfonat;
Z eine Gruppe NOR¹, wobei R¹ die in Anspruch 1 gegebene Bedeutung hat.

8. Geeignetes Mittel, enthaltend einen festen oder flüssigen Trägerstoff und eine Verbindung der allgemeinen Formel I gemäß Anspruch 1 zur Bekämpfung von Schädlingen oder Schadpilzen.

9. Verfahren zur Bekämpfung von Schadpilzen, **dadurch gekennzeichnet, daß** man die Pilze oder die vor Pilzbefall zu schützenden Materialien, Pflanzen, den Boden oder Saatgüter mit einer wirksamen Menge einer Verbindung der allgemeinen Formel I gemäß Anspruch 1 behandelt.

10. Verfahren zur Bekämpfung von Schädlingen, **dadurch gekennzeichnet, daß** man die Schädlinge oder die vor ihnen zu schützenden Materialien, Pflanzen, den Boden oder Saatgüter mit einer wirksamen Menge einer Verbindung der allgemeinen Formel I gemäß Anspruch 1 behandelt.

## Claims

1. A pyridyl phenyl or pyridyl benzyl ether of the formula I 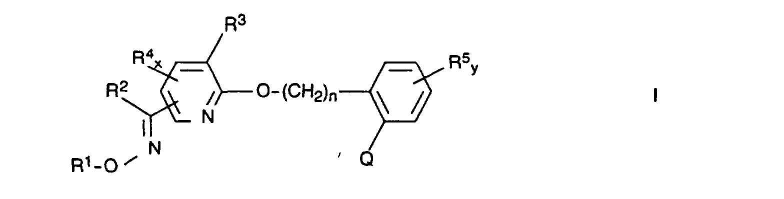 or a salt or N-oxide thereof where the substituents and indices have the following meanings:
Q is C(CO₂CH₃)=CHCH₃, C(CO₂CH₃)=CHOCH₃, C(CONH₂)=NOCH₃, C(CO₂CH₃)=NOCH₃, C(CONHCH₃)=NOCH₃ or N(OCH₃)-CO₂CH₃;
n is 0 or 1;
R¹ is hydrogen or
C₁-C₆-alkyl, C₃-C₆-alkenyl, C₃-C₆-alkynyl, C₁-C₆-haloalkyl, C₃-C₆-haloalkenyl, aryl-C₁-C₂-alkyl, it being possible for the aryl radical to be partially or fully halogenated and/or to have attached to it one to three of the following groups: C₁-C₄-alkyl, C₁-C₄-haloalkyl and C₁-C₄-alkoxy;
R² is hydrogen or
C₁-C₆-alkyl, C₂-C₆-alkenyl, C₂-C₆-alkynyl, C₃-C₆-cycloalkyl or phenyl;
R³ is hydrogen, halogen, C₁-C₄-alkyl or C₁-C₂-haloalkyl;
x is 0, 1 or 2, it being possible for the radicals R⁴ to be different if x is 2;
R⁴ is cyano, nitro, halogen, C₁-C₂-alkyl or C₁-C₂-alkoxy;
y is 0, 1, 2 or 3, it being possible for the radicals R⁵ to be different if y is 2 or 3;
R⁵ is cyano, halogen, C₁-C₄-alkyl, C₁-C₄-haloalkyl or C₁-C₄-alkoxy.

2. A process for the preparation of a compound I as claimed in claim 1, which comprises converting a pyridine derivative of the formula IIa 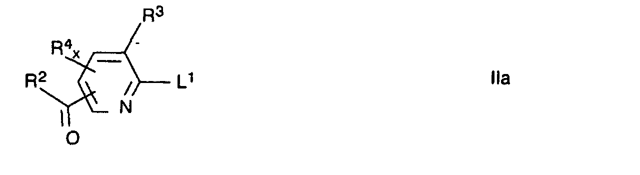 where L¹ is a nucleophilically exchangeable leaving group with a phenol or a benzyl alcohol of the formula IIIa 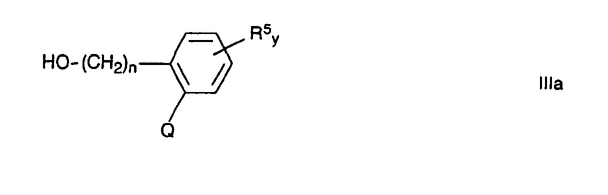 in an inert solvent to give the corresponding ether of the formula IVa 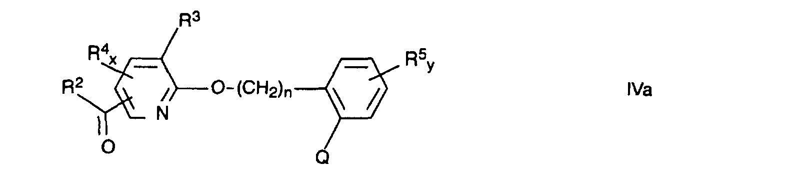 and subsequently reacting IVa with an O-substituted hydroxylamine (R¹-O-NH₂) or a salt thereof to give I.

3. A process for the preparation of a compound I as claimed in claim 1, which comprises converting a pyridine derivative of the formula IIa as set forth in claim 2 with an O-substituted hydroxylamine (R¹-O-NH₂) or a salt thereof to give the corresponding compound of the formula Va 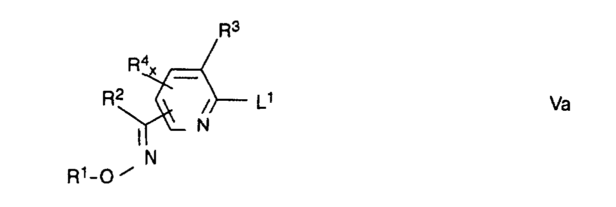 and subsequently reacting Va with a phenol or a benzyl alcohol of the formula IIIa as set forth in claim 2 in an inert solvent to give I.

4. A process for the preparation of a compound I as claimed in claim 1 where n is 1, which comprises converting a pyridine alcohol of the formula IIb 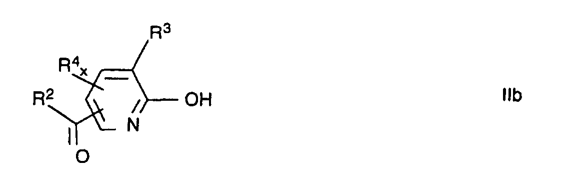 with a benzyl compound of the formula IIIb 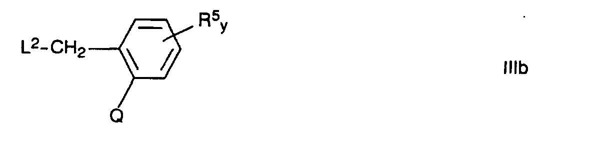 where L² is a nucleophilically exchangeable leaving group in an inert solvent to give the corresponding benzyl ether of the formula IVb 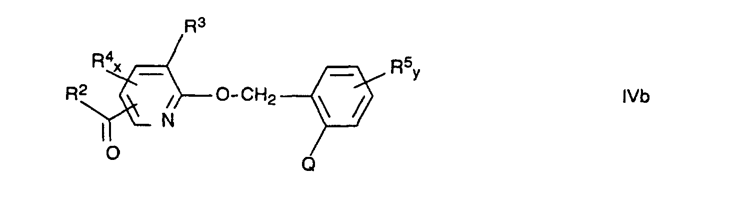 and subsequently reacting IVb with an O-substituted hydroxylamine (R¹-O-NH₂) or a salt thereof to give I.

5. A process for the preparation of a compound I as claimed in claim 1 where n is 1, which comprises converting a pyridine alcohol of the formula IIb as set forth in claim 4 with an O-substituted hydroxylamine (R¹-O-NH₂) or a salt thereof to give the corresponding compound of the formula Vb 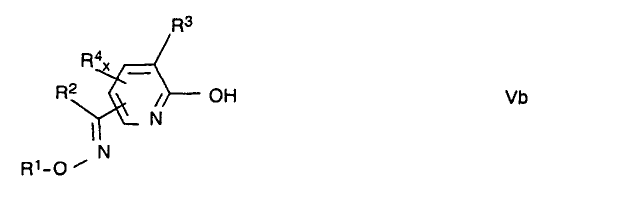 and subsequently reacting Vb with a benzyl compound of the formula IIIb as set forth in claim 4 in an inert solvent to give I.

6. A process for the preparation of a compound IVa as set forth in claim 2, which comprises reacting an ether of the formula Xa 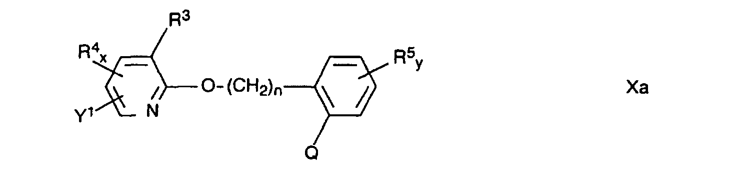 where Y¹ is a halogen atom
either
a) with an activated carboxylic acid of the formula VIIb 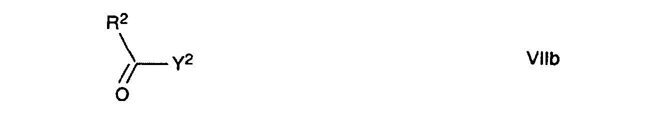 where Y² is a halogen atom, an amide radical or an ester radical, or with a corresponding cyanide R²-C≡N, in an inert solvent in the presence of an organometallic base, or
b) with an organotin compound of the formula XI 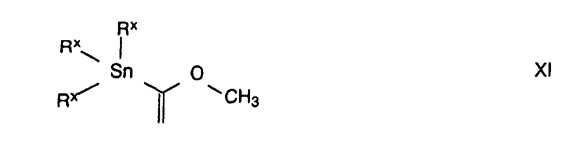
where the radicals R^{x} independently of one another are alkyl, in an inert solvent.

7. A compound of the general formula II 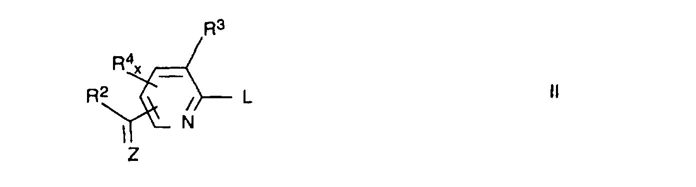 where the substituents R², R³ and R⁴ and the index x have the meanings given in claim 1 and L and Z are the following groups:
L is hydroxyl, halogen, methylsulfonate, trifluoromethylsulfonate, phenylsulfonate or methylphenylsulfonate;
Z is a group NOR¹, R¹ having the meaning given in claim 1.

8. A suitable composition comprising a solid or liquid carrier and a compound of the general formula I as claimed in claim 1 for controlling pests or harmful fungi.

9. A method of controlling harmful fungi, which comprises treating the fungi, or the materials, plants, the soil or seeds to be protected against fungal infection, with an effective amount of a compound of the general formula I as claimed in claim 1.

10. A method of controlling pests, which comprises treating the pests, or the materials, plants, the soil or seeds to be protected against them, with an effective amount of a compound of the general formula I as claimed in claim 1.

## Revendications

1. Ethers de pyridyle, de phényle et de benzyle de formule I, 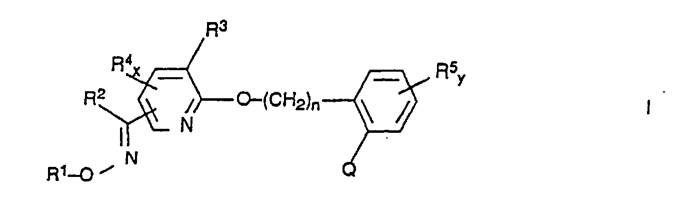 ainsi que leurs sels et N-oxydes, dans lesquels les substituants et indices ont la signification suivante:
Q C(CO₂CH₃)=CHCH₃, C(CO₂CH₃)=CHOCH₃, C(CONH₂)=NOCH₃, C(CO₂CH₃)=NOCH₃, C(CONHCH₃)=NOCH₃ ou N(OCH₃)-CO₂CH₃;
n 0 ou 1;
R¹ hydrogène ou
alkyle en C₁-C₆, alcényle C₃-C₆, alcynyle en C₃-C₆, halogénoalkyle en C₁-C₆, halogéno-alcényle en C₃-C₆, aryl-alkyle(C₁-C₂), tandis que le reste aryle peut être partiellement ou totalement halogéné et/ou peut porter un à trois des groupes suivants: alkyle en C₁-C₄, halogénoalkyle en C₁-C4 et alcoxy en C₁-C₄;
R² hydrogène ou
alkyle en C₁-C₆, alcényle en C₂-C₆, alcynyle en C₂-C₆, cycloalkyle en C₃-C₆ ou phényle;
R³ hydrogène, halogène, alkyle en C₁-C₄ ou halogénoalkyle en C₁-C₂;
x 0, 1 ou 2, tandis que les restes R⁴ peuvent être différents quand x vaut 2;
R⁴ cyano, nitro, halogène, alkyle en C₁-C₂ ou alcoxy en C₁-C₂;
y 0, 1, 2 ou 3, tandis que les restes R⁵ peuvent être différents quand y vaut 2 ou 3;
R⁵ cyano, halogène, alkyle en C₁-C₄, halogénoalkyle en C₁-C₄ o alcoxy en C₁-C₄.

2. Procédé pour la préparation des composés I selon la revendication 1, **caractérisé par le fait qu'**on convertit un dérivé de pyridine de formule IIa 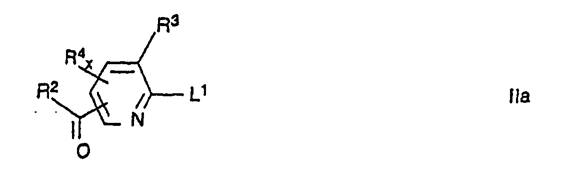 où L¹ désigne un groupe séparable, échangeable par voie nucléophile, dans un solvant inerte avec un phénol ou un alcool benzylique de formule IIIa 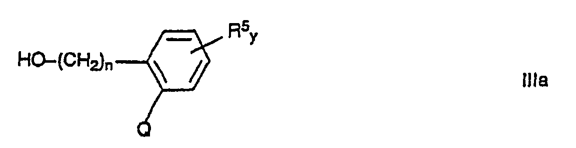 en l'éther correspondant de formule IVa 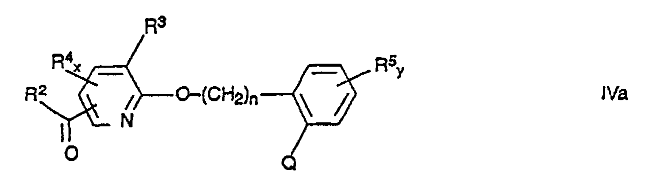 et on fait ensuite réagir IVa avec une hydroxylamine O-substituée (R¹-O-NH₂) ou son sel pour obtenir I.

3. Procédé pour la préparation des composés I selon la revendication 1 **caractérisé par le fait que** l'on convertit un dérivé de pyridine de formule IIa selon la revendication 2 avec une hydroxylamine O-substituée (R¹-O-NH₂) ou son sel en le composé de formule Va 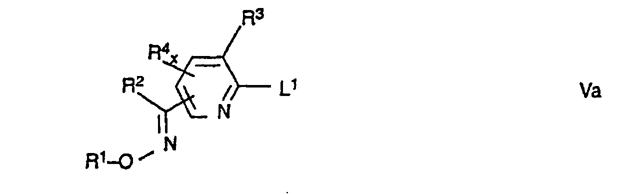 Correspondant et on fait ensuite réagir Va dans un solvant inerte avec un composé d'alcool benzylique respectivement phénolique de formule IIIa selon la revendication 2 pour obtenir I.

4. Procédé pour la préparation des composés I selon la revendication 1 dans lesquels n vaut 1, **caractérisé par le fait qu'**on convertit un alcool pyridinique de formule IIb 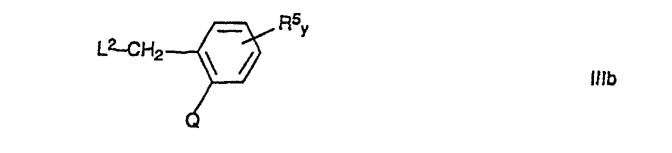 dans un solvant inerte avec un composé benzylique de formule IIIb 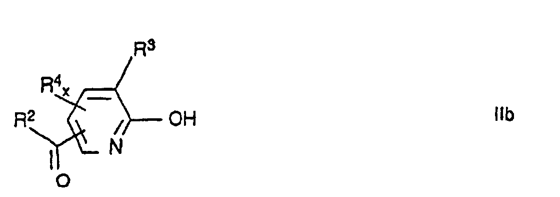 où L² désigne un groupe séparable, échangeable par voie nucléophile, en l'éther benzylique correspondant de formule IVb 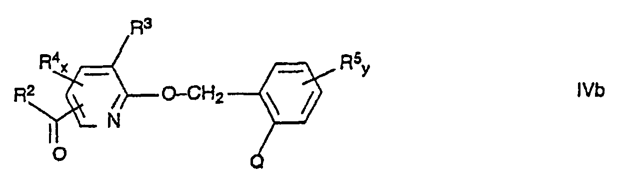 et on fait ensuite réagir IVb avec une hydroxylamine O-substituée (R¹-O-NH₂) ou son sel pour obtenir I.

5. Procédé pour la préparation des composés I selon la revendication 1, dans lesquels n vaut 1, **caractérisé par le fait qu'**on convertit un alcool pyridinique de formule IIb selon la revendication 4 avec une hydroxylamine O-substituée (R¹-O-NH₂) ou son sel en le composé de formule Vb correspondant 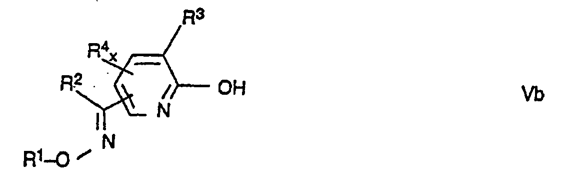 et on fait ensuite réagir Vb dans un solvant inerte avec un composé benzylique de formule IIIb selon la revendication 4 pour obtenir I.

6. Procédé pour la préparation des composés IVa selon la revendication 2, **caractérisé par le fait qu'**on convertit un éther de formule Xa 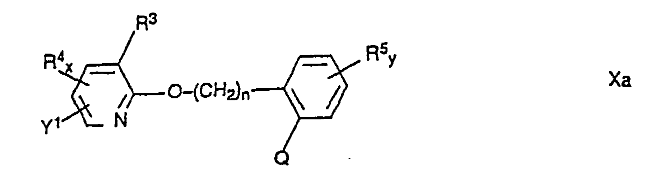 où Y¹ désigne un atome d'halogène, soit
a) en présence d'une base organo-métallique dans un solvant inerte avec un acide carboxylique activé de formule VIIb 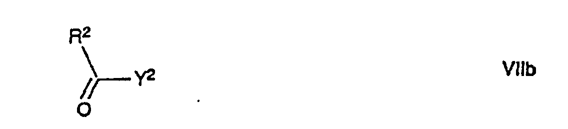 dans laquelle Y² désigne un atome d'halogène, un reste amide ou un reste ester, soit
b) dans un solvant inerte avec un composé organique d'étain de formule XI 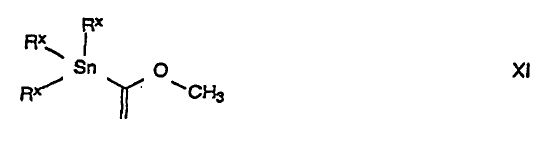
où les restes R^{x} désignent, indépendamment l'un de l'autre, un alkyle.

7. Composés de formule générale II 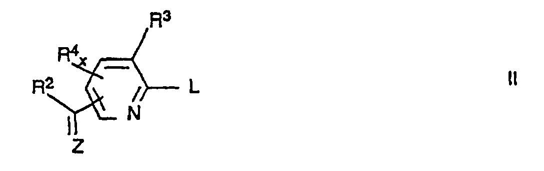 où les substituants R², R³ et R⁴ et l'indice x ont la signification indiquée dans la revendication 1, et L et Z désignent les groupes suivants:
L hydroxy, halogène, méthyl-, trifluorométhyl-, phényl- ou méthylphényl-sulfonate;
Z un groupe NOR¹, où R¹ a la signification indiquée dans la revendication 1.

8. Produit approprié, contenant un support solide ou liquide et un composé de formule générale I selon la revendication 1 pour la lutte contre les parasites ou les champignons nuisibles.

9. Procédé pour la lutte contre les champignons nuisibles, **caractérisé par le fait qu'**on traite les champignons ou les matériaux, plantes, sols ou semences à à protéger contre une attaque par les champignons, avec une quantité efficace d'un composé de formule générale I selon la revendication 1.

10. Procédé pour la lutte contre les parasites, **caractérisé par le fait qu'**on traite les parasites ou les matériaux, plantes, sol ou semences à protéger contre eux avec une quantité efficace d'un composé de formule générale I selon la revendication 1.
